# EUROPEAN PATENT SPECIFICATION

(11) **EP 1 618 094 B1**
(45) Date of publication and mention of the grant of the patent: **05.09.2007**
(21) Application number: 04760673.6
(22) Date of filing: 30.04.2004
(51) Int. Cl.: C07D 239/30, C07D 407/14, C07D 403/04, C07D 403/14, C07D 239/38, C07D 409/12, C07D 417/04, A61K 31/517

(54) **SUBSTITUTED HETEROARYLS AS INHIBITORS OF PROTEIN TYROSINE PHOSPHATASES**
SUBSTITUIERTE HETEROARYLVERBINDUNGEN ALS INHIBITOREN VON PROTEINTYROSINPHOSPHATASEN
HETEROARYLES SUBSTITUES UTILISES EN TANT QU'INHIBITEURS DES PROTEINE-TYROSINE PHOSPHATASES

(30) Priority: 30.04.2003 US 466869 P
(43) Date of publication of application: 25.01.2006
(73) Proprietor: The Institutes for Pharmaceutical Discovery, LLC, Branford, CT 06405 (US)
(72) Inventor: SAVOY, Jennifer, Clinton, CT 06413 (US); GERACI, Leo, Clinton, CT 06413 (US); PARKER, Garrett, Branford, CT 06405 (US); VAN ZANDT, Michael, C., Guilford, CT 06437 (US); WHITEHOUSE, Darren, Westbrook, CT 06498 (US); HU, Shaojing, Hamden, CT 06514 (US)
(74) Representative: Adam, Holger
(86) International application number: PCT/US2004/013579
(87) International publication number: WO 2004/099159

(56) References cited:
- WO-A-01/19830
- US-A- 3 884 919
- DATABASE CROSSFIRE BEILSTEIN BEILSTEIN INSTITUTE FOR ORGANIC CHEMISTRY, FRANKFURT-MAIN, DE; XP002295081 Database accession no. BRN: 588183 & COLLECT. CZECH. CHEM. COMMUN., vol. 44, 1979, pages 2243-2248,
- DATABASE CROSSFIRE BEILSTEIN BEILSTEIN INSTITUTE FOR ORGANIC CHEMISTRY, FRANKFURT-MAIN, DE; XP002295082 Database accession no. BRN: 5606672 & J. PRAKT. CHEM., vol. 32, no. 5, 1982, pages 841-846,
- SARMIENTO M ET AL: "Structure-based discovery of small molecule inhibitors targeted to protein tyrosine phosphatase 1B" JOURNAL OF MEDICINAL CHEMISTRY, AMERICAN CHEMICAL SOCIETY. WASHINGTON, US, vol. 43, no. 2, 27 January 2000 (2000-01-27), pages 146-155, XP002216617 ISSN: 0022-2623
- DATABASE CROSSFIRE BEILSTEIN BEILSTEIN INSTITUTE FOR ORGANIC CHEMISTRY, FRANKFURT-MAIN, DE; XP002295083 Database accession no. BRN: 209253 & TETRAHEDRON LETTERS, 1968, pages 325-330,
- DATABASE CROSSFIRE BEILSTEIN BEILSTEIN INSTITUTE FOR ORGANIC CHEMISTRY, FRANKFURT-MAIN, DE; XP002295084 Database accession no. BRN:677854 & SYNTHESIS, 1977, pages 345-346,

## Description

### BACKGROUND OF THE INVENTION

### Cross Reference to Related Applications

This application claims priority from U.S. Provisional Application Serial Number 60/466,869, filed April 30, 2003.

### Field of the Invention

The invention relates to substituted heteroaryls and more specifically to such compounds that are useful in the treatment of syndrome X (consisting of such abnormalities as obesity, dyslipidemia, hypercoagulation, hypertension, insulin resistance and leading to heart disease and diabetes), obesity, diabetes, immunological disease, bleeding disorders and/or cancer. More specifically, it relates to such compounds that are capable of inhibiting Protein tyrosine phosphatases (PTPs), in particular Protein tyrosine phosphatase-1B (PTP-1B) which is a negative regulator of the insulin and leptin signaling pathway and improves insulin-sensitivity.

### Description of the Related Art

This invention relates to a class of heterocycle substituted carboxylic acids that are inhibitors of various PTPs, in particular PTP-1B.

Protein tyrosine phosphatases are a large family of transmembrane or intracellular enzymes that dephosphorylate substrates involved in a variety of regulatory processes (Fischer et al., 1991, Science 253:401-406). Protein tyrosine phosphatase-1B (PTP-1B) is an approximately 50 kd intracellular protein, which is present in abundant amounts in various human tissues (Charbonneau et al., 1989, Proc. Natl. Acad. Sci. USA 86:5252-5256; Goldstein, 1993, Receptor 3:1-15).

Determining which proteins are substrates of PTP-1B has been of considerable interest. One substrate which has aroused especial interest is the insulin receptor. The binding of insulin to its receptor results in autophosphorylation of the domain. This causes activation of the insulin receptor tyrosine kinase, which phosphorylates the various insulin receptor substrate (IRS) proteins that propagate the insulin signaling event further downstream to mediate insulin's various biological effects.

Seely et al., 1996, Diabetes 45:1379-1385 ("Seely") studied the relationship of PTP-1B and the insulin receptor in vitro. Seely constructed a glutathione S-transferase (GST) fusion protein of PTP-1B that had a point mutation in the PTP-1B catalytic domain. Although catalytically inactive, this fusion protein was able to bind to the insulin receptor, as demonstrated by its ability to precipitate the insulin receptor from purified receptor preparations and from whole cell lysates derived from cells expressing the insulin receptor.

Ahmad et al., 1995, J. Biol. Chem. 270:20503-20508 used osmotic loading to introduce PTP-1B neutralizing antibodies into rat KRC-7 hepatoma cells. The presence of the antibody in the cells resulted in an increase of 42% and 38%, respectively, in insulin stimulated DNA synthesis and phosphatidyinositol 3' kinase activity. Insulin receptor autophosphorylation and insulin receptor substrate-1 tyrosine phosphorylation were increased 2.2 and 2.0-fold, respectively, in the antibody-loaded cells. The antibody-loaded cells also showed a 57% increase in insulin stimulated insulin receptor kinase activity toward exogenous peptide substrates.

Kennedy et al., 1999, Science 283: 1544-1548 showed that protein tyrosine phosphatase PTP-1B is a negative regulator of the insulin signaling pathway, indicating that inhibitors of this enzyme are beneficial in the treatment of Type 2 diabetes, which appears to involve a defect in an early process in insulin signal transduction rather than a structural defect in the insulin receptor itself. (J. M. Olefsky, W. T. Garvey, R. R. Henry, D. Brillon, S. Matthai and G. R. Freidenberg, G. R. (1988).) Cellular mechanisms of insulin resistance in non-insulin-dependent (Type II) diabetes. (Am. J. Med. 85: Suppl. 5A, 86-105.) A drug that improved insulin sensitivity would have several advantages over traditional therapy of NIDDM using sulfonylureas, which do not alleviate insulin resistance but instead compensate by increasing insulin secretion.

Ragab et al (2003, J. Biol. Chem 278(42), 40923-32) showed that PTP 1B is involved in regulating platelet aggregation. Hence, inhibition of PTP 1B can be predicted to have an effect on bleeding disorder, and cardiovascular disease.

Romsicki et al., (2003, Arch Biochem. Biophys 414(1), 40-50) showed that TC PTP is structurally and functionally very similar. A PTP 1B inhibitor is very likely to also inhibit TC PTP. A knockout of the TC PTP gene produces a phenotype with impaired immune function. (You-Ten et al., 1997, J. Exp. Med. 186(5), 683-93). Hence, inhibitors of PTP 1B can be predict to inhibit TC PTP and modulate immune response.

It has also been demonstrated that PT-P1B is a negative regulator of leptin signaling (Kaszua et al. MolCell..Endocrinology, 195:109-118, 2002). PTP-1B deficient mice show enhanced potency for exogenous leptin to suppress food intake (Cheng, et al. Developmental Cell 2:497-503, 2002). Thus, inhibitors of PTP-1B augment the beneficial effects of leptin on food intake, body weight regulation and metabolism, in normal individuals and leptin resistant individuals.

Therefore, inhibitors of PTPs, and inhibitors of PTP-1B in particular, are useful in controlling or treating obesity, syndrome X, Type 2 diabetes, in improving glucose tolerance, and in improving insulin sensitivity in patients in need thereof. Such compounds are also useful in treating or controlling other PTP mediated diseases, such as the treatment of cancer, neurodegenerative diseases, immunological disorders, bleeding and cardiovascular disorders, and the like.

Further prior art documents disclose the following:

US 3,884,919 discloses 1-(Heterocyclic)-indol-3-ylacetic acid derivatives, processes for their preparation, and pharmaceutical compositions comprising them. An illustrative compound is 1-(7-chloroquinazolin-4-yl)-5-methoxy-2-methylindol-3-ylacetic acid. The compounds have anti-inflammatory, analgesic and antipyretic activity.

Ji 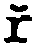 i Gaspari 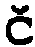 et al. disclose products of the acid hydrolysis of 7-chloro-1,3-dihydro-3-hydroxy-5-phenyl-2*H*-1,4-benzodiazepin-2-one (oxazepam) (see Collect. Czech. Chem. Commun., vol. 44. 1979, pages 2243-2248).

G. Kempter et al. in Journal f. prakt. Chemie, vol. 324, no. 5, 1982, pages 841-846 disclose N-alkylation and dehydrogenation of 1,2,3,4-tetrahydroquinazolines.

WO 01/19830 discloses compounds, compositions, methods of their use, and methods for their manufacture, where such compounds of Formula (1) are pharmacologically useful inhibitors of Protein Tyrosine Phosphatases. The compounds are said to be useful in the treatment of type I diabetes, type II diabetes, impaired glucose tolerance, insulin resistance, obesity, and other diseases.

The article by Mauro Sarmiento et al., J. Med. Chem. 2000, 43, 146-155 relates to structure-based discovery of small molecule inhibitors targeted to protein tyrosine phosphatase 1B.

J. Sauer & K.K. Mayer describe in Tetrahedron Letters, no. 3, 1968, pages 325-330 thermolysis and photolysis of 3,4-diphenyl-Δ²-1,2,4-oxdiazolinone-(5) and 2, 4-diphenyl- Δ²-1,3,4-oxdiazolinone-(5).

V. Gomez Parra et al. describe in Synthesis, 1977, pages 345-346 the synthesis of heterocycles via nitrilium salts (2-aminoquinazolines).

### SUMMARY OF THE INVENTION

In a broad aspect, the invention encompasses the compounds of formula (II) shown below, pharmaceutical compositions containing the compounds and the use of such compounds in the manufacture of a medicament for the treatment of diabetes.

The invention provides compounds of formula II: 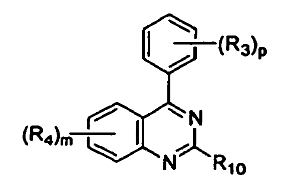 or a pharmaceutically acceptable salt thereof, wherein
- m: is 0, 1, 2, 3, or 4;
- p: is 0, 1, 2, 3, 4, or 5;
- R₁₀: is indolyl, substituted with 1, 2, 3, or 4 groups that are independently selected from the group consisting of oxo,-C(O)-(C₁-C₆ alkyl)-C(O)-OR₁, -C(O)-NH-(C₁-C₆ alkyl)-C(O)-OR₁, -C(O)-(C₁-C₆ haloalkyl)-C(O)-OR₁, C₂-C₆ haloalkenyl-C(O)-OR₁, -C(O)-OR₁, -SO₂-(C₁-C₆ alkyl), -SO₂-phenyl, halogen, C₁-C₄ alkyl, C₁-C₄ alkoxy, C₁-C₄ alkoxycarbonyl, C₂-C₆ alkanoyl optionally substituted with CO₂H or CO₂-(C₁-C₄ alkyl), CO₂H, and phenyl substituted with 1, 2, 3, 4, or 5 groups that are independently halogen, C₁-C₄ alkyl, C₁-C₄ alkoxy, NO₂, CF₃, or OCF₃ ;
- R₁: is H, C₁-C₆ alkyl, - (C₁-C₅ alkyl)-CO₂H, or -(C₁-C₅ alkyl)-CO₂, C₁-C₆ alkyl;
- each R₃: is independently C₁-C₆ alkyl, halogen, C₁-C₄ alkoxy, -CN, -OH, -C(O)-OR₁, -C(O)-(C₁-C₆ alkyl)-C(O)-OR₁ or -(C₁-C₄ alkoxy)-phenyl, wherein the alkyl portions are optionally substituted with one or two groups that are independently oxo, -NH₂, -OH, -SO₂-C₁-C₆ alkyl, C₁-C₆ alkyl, C₁-C₆ alkoxy,-C(O)-OR₁, or C₁-C₄-aryl; and
each R₄ is independently halogen, heterocycloalkyl, heteroaryl, C₁-C₆ haloalkyl, C₁-C₆ alkoxy, or -NO₂, wherein the heterocycloalkyl is optionally substituted with one or two groups that are independently C₁-C₆ alkyl, C₁-C₆ alkoxy, -OH,-NH₂, -NO₂, oxo, or -CN.

The compounds of formula II bind to PTPs, and in particular to PTP-1B. The interaction with the enzyme, specifically PTP-1B, preferably results in inhibition of the enzyme.

The invention also includes intermediates that are useful in making the compounds of the invention.

The invention also provides pharmaceutical compositions comprising a compound or salt of formula II and at least one pharmaceutically acceptable carrier, solvent, adjuvant or diluent.

The invention also provides the use of a compound or salt according to formula II for the manufacture of a medicament for use in treating diabetes.

The invention also discloses methods of preparing the compounds of the invention and the intermediates used in those methods.

The invention also discloses the use and compositions for combination therapy of Type I and Type II diabetes. In these embodiments, the invention discloses formulations and pharmaceutical compositions, as well as the use for treating Type I and Type II diabetes with the compounds of formula I plus additional compounds and medicaments as disclosed in more detail below. In these embodiments, the use of the invention can comprise treatment methods for Type I and Type II diabetes where the compounds of formula I are formulated with a therapeutically-effective amount of said additional compounds and medicaments. In alternative embodiments, treatment methods of the invention for Type I and Type II diabetes comprise administration of the inventive compounds of formula I as disclosed herein concomitantly, simultaneously or together with a therapeutically-effective amount of said additional compounds and medicaments.

### DETAILED DESCRIPTION OF THE INVENTION

Preferred compounds include those of formula II: 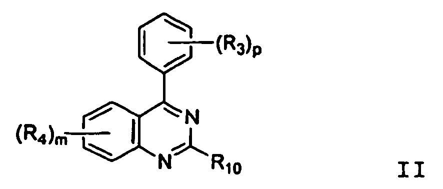 or a pharmaceutically acceptable salt thereof, wherein
- m: is 0, 1, 2, 3, or 4,
- p: is 0, 1, 2, 3, 4, or 5;
- R₁₀: is indolyl substituted with 1, 2, 3, or 4 groups that are independently selected from the group consisting of oxo,-C(O)-(C₁-C₆ alkyl)-C(O)-OR₁, -C(O)-NH-(C₁-C₆ alkyl)-C(O)-OR₁, -C(O)-(C₁-C₆ haloalkyl)-C(O)-OR₁, C₁-C₆ haloalkenyl-C(O)-OR₁, -C(O)-OR₁, -SO₂-(C₁-C₆ alkyl), -SO₂-phenyl, halogen, C₁-C₄ alkyl, C₁-C₄ alkoxy, C₁-C₄ alkoxycarbonyl, C₂-C₆ alkanoyl optionally substituted with CO₂H or CO₂-(C₁-C₄ alkyl), CO₂H, and phenyl optionally substituted with 1, 2, 3, 4, or 5 groups that are independently halogen, C₁-C₄ alkyl, C₁-C₄ alkoxy, NO₂, CF₃ and OCF₃;
- R₁: is H, C₁-C₆ alkyl, -(C₁-C₅ alkyl)-CO₂H, or -(C₁-C₅ alkyl)-CO₂-C₁-C₆ alkyl;
- each R₃: is independently C₁-C₆ alkyl, halogen, C₁-C₄ alkoxy, -CN, -OH, -C(O)-OR₁, -C(O)-(C₁-C₆ alkyl)-C(O)-OR₁ or -(C₁-C₄ alkoxy)-phenyl, wherein the alkyl portions are optionally substituted with one or two groups that are independently oxo, -NH₂, -OH, -SO₂-C₁-C₆ alkyl, C₁-C₆ alkyl, C₁-C₆ alkoxy, -C(O)-OR₁, or C₁-C₄-aryl; and
- each R₄: is independently halogen, heterocycloalkyl, heteroaryl, C₁-C₆ haloalkyl, C₁-C₆ alkoxy, or -NO₂, wherein the heterocycloalkyl is optionally substituted with one or two groups that are independently C₁-C₆ alkyl, C₁-C₆ alkoxy,-OH, -NH₂, -NO₂, oxo, or -CN.

Preferred compound according to formula II are compounds wherein R₁₀ is indolyl, wherein each of the above is substituted with 1, 2, or 3 groups that are independently selected from the group consisting of oxo, -C(O)-(C₁-C₆ alkyl)-C(O)-OR₁, -C(O)-NH-(C₁-C₆ alkyl)-C(O)-OR₁, -C(O)-(C₁-C₆ haloalkyl)-C(O)-OR₁, C₁-C₆ haloalkenyl-C(O)-OR₁, -SO₂-(C₁-C₆ alkyl), -SO₂-phenyl, halogen, C₁-C₄ alkyl, C₁-C₄ alkoxy, C₁-C₄ alkoxycarbonyl, C₂-C₆ alkanoyl optionally substituted with CO₂H or CO₂-(C₁-C₄ alkyl), CO₂H, and phenyl substituted with 1, 2, 3, 4, or 5 groups that are independently halogen, C₁-C₄ alkyl, C₁-C₄ alkoxy, NO₂, CF₃ or OCF₃; and

Also preferred are compounds wherein R₁₀ is indolyl substituted with one or two groups that are independently halogen, -C(O)-(C₁-C₆ alkyl)-C(O)-OR₁, -C(O)-NH-(C₁-C₆ alkyl)-C(O)-OR₁, -C(O)-(C₁-C₆ haloalkyl)-C(O)-OR₁, or C₁-C₆ haloalkenyl-C(O)-OR₁. Preferably, the halogen is bromo or chloro. Preferably, R₁ is -H and the C₁-C₆ alkyl is -C₂H₄- or -CH₂-, C₁-C₆ haloalkyl is -CF₂-, and C₁-C₆ haloalkenyl is -CH=CF-.

Also preferred are compounds according to formula II, wherein each R₃ is independently C₁-C₆ alkyl, halogen, -CN, C₁-C₆-alkoxy, -(C₁-C₄ alkoxy)-phenyl; and each R₄ is independently halogen, or furanyl.

Also preferred are compound according to formula II, wherein R₁₀ is indol-1-yl, substituted with 1, 2, or 3 groups that are independently selected from the group consisting of -C(O)-C₂H₄-C(O)-OH, -C(O)-NH-C₂H₄-C(O)-OH, -SO₂-(C₁-C₆ alkyl), -SO₂-phenyl, halogen, C₁-C₄ alkyl, C₁-C₄ alkoxy, C₄ alkoxycarbonyl, C₂-C₆ alkanoyl substituted with CO₂H or CO₂-(C₁-C₄ alkyl), CO₂H, and phenyl substituted with 1, 2, 3, 4, or 5 groups that are independently halogen, C₁-C₄ alkyl, C₁-C₄ alkoxy, NO₂, CF₃ or OCF₃;
each R₃ is independently C₁-C₆ alkyl, halogen, or benzyloxy;
each R₄ is independently halogen.

Preferably, in Formula II R₁₀ is indol-1-yl or indol-2-yl, each of which is substituted with 1 or 2 groups that are independently -SO₂-(C₁-C₆ alkyl), -SO₂-phenyl, halogen, C₁-C₄ alkyl, C₁-C₄ alkoxy, C₄ alkoxycarbonyl, C₂-C₆ alkanoyl optionally substituted with CO₂H or CO₂-(C₁-C₄ alkyl), CO₂H, or phenyl substituted with 1, 2, 3, 4, or 5 groups that are independently halogen, C₁-C₄ alkyl, C₁-C₄ alkoxy, NO₂, CF₃ or OCF₃, and each R₃ is independently C₁-C₆ alkyl, halogen, or benzyloxy and each R₄ is independently halogen.

Preferably, R₁₀ In formula II is indol-1-yl substituted with 1 or 2 groups that are independently halogen, C₁-C₄ alkyl, C₁-C₄ alkoxy, C₄ alkoxycarbonyl, or C₂-C₆ alkanoyl optionally substituted with CO₂H or CO₂-(C₁-C₄ alkyl), and each R₃ is independently C₁-C₆ alkyl, halogen, or benzyloxy and each R₄ is independently halogen.

Preferably, R₁₀ in Formula II is indol-1-yl substituted with 1 or 2 groups that are independently halogen, C₁-C₄ alkyl, C₁-C₄ alkoxy, or C₂-C₄ alkanoyl optionally substituted with CO₂H or CO₂-(C₁-C₄ alkyl).
Preferably, in Formula II R₁₀ is indol-1-yl substituted with 1 or 2 groups that are independently halogen, C₁-C₄ alkyl, C₁-C₄ alkoxy, or C₂-C₄ alkanoyl optionally substituted with CO₂H or CO₂-(C₁-C₄ alkyl), where at least one group is an optionally substituted C₂-C₄ alkanoyl in the 3-position of the indole ring.

In another aspect, the invention encompasses the use of a compound or salt of formula II, or a pharmaceutical composition comprising a compound or salt of formula II for the manufacture of a medicament for treating diabetes.

In another aspect, the invention encompasses a pharmaceutical composition of a compound or salt of claim 1 and at least one pharmaceutically acceptable solvent, carrier, adjuvant or excipient.

The term "heterocycloalkyl," refers to a ring or ring system containing at least one heteroatom selected from nitrogen, oxygen, and sulfur, wherein said heteroatom is in a non-aromatic ring. The heterocycloalkyl ring is optionally fused to or otherwise attached to other heterocycloalkyl rings and/or non-aromatic hydrocarbon rings and/or phenyl rings. Preferred heterocycloalkyl groups have from 3 to 7 members. Examples of heterocycloalkyl groups include, for example, 1,2,3,4-tetrahydroisoquinolinyl, piperazinyl, morpholinyl, piperidinyl, tetrahydrofuranyl, pyrrolidinyl, pyridinonyl, and pyrazolidinyl. Preferred heterocycloalkyl groups include piperidinyl, piperazinyl, morpholinyl, pyrrolidinyl, pyridinonyl, dihydropyrrolidinyl, and pyrrolidinonyl.

The compounds of general Formula II may be administered orally, topically, parenterally, by inhalation or spray or rectally in dosage unit formulations containing conventional non-toxic pharmaceutically acceptable carriers, adjuvants and vehicles. The term parenteral as used herein includes percutaneous, subcutaneous, intravascular (e.g., intravenous), intramuscular, or intrathecal injection or infusion techniques and the like. In addition, there is provided a pharmaceutical formulation comprising a compound of general Formula II and a pharmaceutically acceptable carrier. One or more compounds of general Formula II may be present in association with one or more non-toxic pharmaceutically acceptable carriers and/or diluents and/or adjuvants, and if desired other active ingredients. The pharmaceutical compositions containing compounds of general Formula II may be in a form suitable for oral use, for example, as tablets, troches, lozenges, aqueous or oily suspensions, dispersible powders or granules, emulsion, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preservative agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques. In some cases such coatings may be prepared by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatin capsules, wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Formulations for oral use may also be presented as lozenges.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the active ingredients in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, may also be present.

Pharmaceutical compositions of the invention may also be in the form of oil-in-water emulsions. The oily phase may be a vegetable oil or a mineral oil or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol, glucose or sucrose. Such formulations may also contain a demulcent, a preservative and flavoring and coloring agents. The pharmaceutical compositions may be in the form of a sterile injectable aqueous or oleaginous suspension. This suspension may be formulated according to the known art using those suitable dispersing or wetting agents and suspending agents that have been mentioned above. The sterile injectable preparation may also be a sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The compounds of general Formula II may also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Compounds of general Formula II may be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

For disorders of the eye or other external tissues, e.g., mouth and skin, the formulations are preferably applied as a topical gel, spray, ointment or cream, or as a suppository, containing the active ingredients in a total amount of, for example, 0.075 to 30% w/w, preferably 0.2 to 20% w/w and most preferably 0.4 to 15% w/w. When formulated in an ointment, the active ingredients may be employed with either paraffinic or a water-miscible ointment base.

Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas. Examples of such dermal penetration enhancers include dimethylsulfoxide and related analogs. The compounds of this invention can also be administered by a transdermal device. Preferably topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane. The transdermal patch may include the compound in a suitable solvent system with an adhesive system, such as an acrylic emulsion, and a polyester patch. The oily phase of the emulsions of this invention may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. Emulsifiers and emulsion stabilizers suitable for use in the formulation of the present invention include Tween 60, Span 80, cetostearyl alcohol, myristyl alcohol, glyceryl monostearate, and sodium lauryl sulfate, among others. The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for the active ingredients. The antiinflammatory active ingredients are preferably present in such formulations in a concentration of 0.5 to 20%, advantageously 0.5 to 10% and particularly about 1.5% w/w. For therapeutic purposes, the active compounds of this combination invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or nonaqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Dosage levels of the order of from about 0.1 mg to about 140 mg per kilogram of body weight per day are useful in the treatment of the above-indicated conditions (about 0.5 mg to about 7 g per patient per day). The amount of active ingredient that may be combined with the carrier materials to produce a single dosage form will vary depending upon the host treated and the particular mode of administration. Dosage unit forms will generally contain between from about 1 mg to about 500 mg of an active ingredient. The daily dose can be administered in one to four doses per day. In the case of skin conditions, it may be preferable to apply a topical preparation of compounds of this invention to the affected area two to four times a day.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition may also be added to the animal feed or drinking water. It may be convenient to formulate the animal feed and drinking water compositions so that the animal takes in a therapeutically appropriate quantity of the composition along with its diet. It may also be convenient to present the composition as a premix for addition to the feed or drinking water. Preferred non-human animals include domesticated animals.

### Methods of Preparation

The compounds of the present invention and reference compounds not of the invention may be prepared by use of known chemical reactions and procedures. General methods for synthesizing the compounds are presented below. It is understood that the nature of the substituents required for the desired target compound often determines the preferred method of synthesis. All variable groups of these methods are as described in the generic description if they are not specifically defined below.

Certain compounds of the invention can be conveniently prepared from the corresponding substituted 2-aminobenzophenone as illustrated in Scheme A. In this method, the desired 2-aminobenzophenone, A-1, is conveniently treated with urea and an acid, preferably acetic acid, to form the cyclized condensation product, A-2. Subsequent activation of the urea moiety by conversion to vinyl chloride, A-3, with POCl₃ or PCl₅ followed by coupling to the desired amine, NHR₁R₂ (where NHR₁R₂ may be a cyclic amine such as, for example, piperidine, pyrrolidine, pyrrolidinone, indole, indoline or imidazolidine), gives the 2-aminoquinazoline, A-4. The specific coupling reaction conditions may depend on the substituents R₁₋₄ required for the desired target molecule. Often, the chloroquinazoline can simply be heated with the desired amine in a solvent like diphenylether, THF or DMF, or in the absence of solvent altogether.

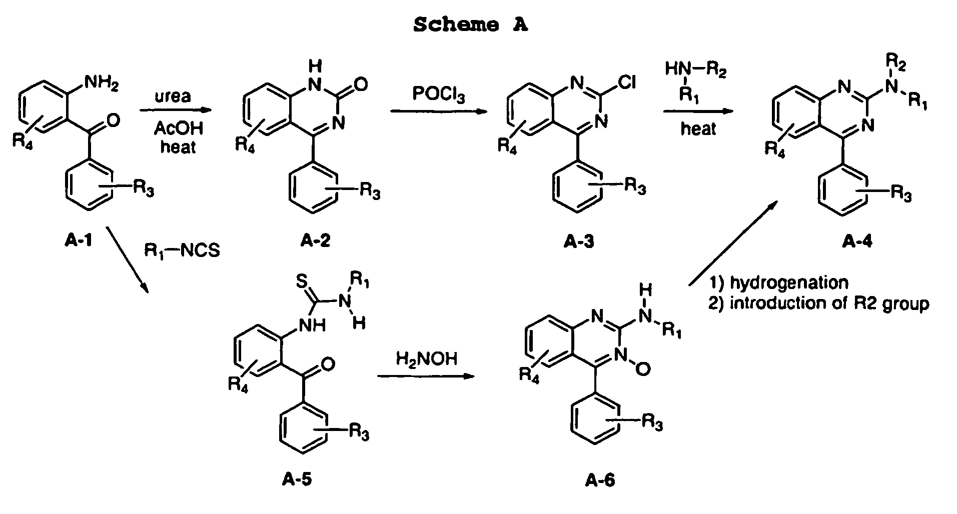

For some molecules, a preferred method of synthesis may involve coupling the 2-aminobenzophenone with an isothiocyanate substituted with the desired R¹ substituent to form intermediate A-5. These reactions can often be performed simply by heating the two reagents is a suitable solvent. For some examples, the presence of a base like triethylamine, diisopropylethylamine, or pyridine will facilitate the reaction. The thiourea intermediate A-5, once formed, is treated with hydroxylamine to give the 2-aminoquinazoline oxide, A-6. Reduction, preferably with hydrogen gas using a palladium or nickel catalyst provides the desired 2-aminoquinazoline.

If the desired 2-aminobenzophenone is not commercially available, it can be prepared by a variety of known methods. One convenient method uses commercially available anthranilic acids as described in scheme B. In this method the anthranilic acid is treated with a reagent like acetic anhydride to form the activated intermediate B-2. Subsequent addition of an aryl or heteroaryl nucleophile, such as Grignard reagent B-3 provides the corresponding 2-aminobenzophenone derivative B-4.

Using an alternative method, the anthranilic acid can be treated with a reagent like carbonyldiimidazole (CDI), phosgene or triphosgene to form the corresponding anhydride B-5. Subsequent addition of methoxymethylamine forms the methoxymethyl amide commonly know as a Weinreb amide. Addition of an aryl or heteroaryl lithium reagent (2 equivalents) provides the desired 2-aminobenzophenone.

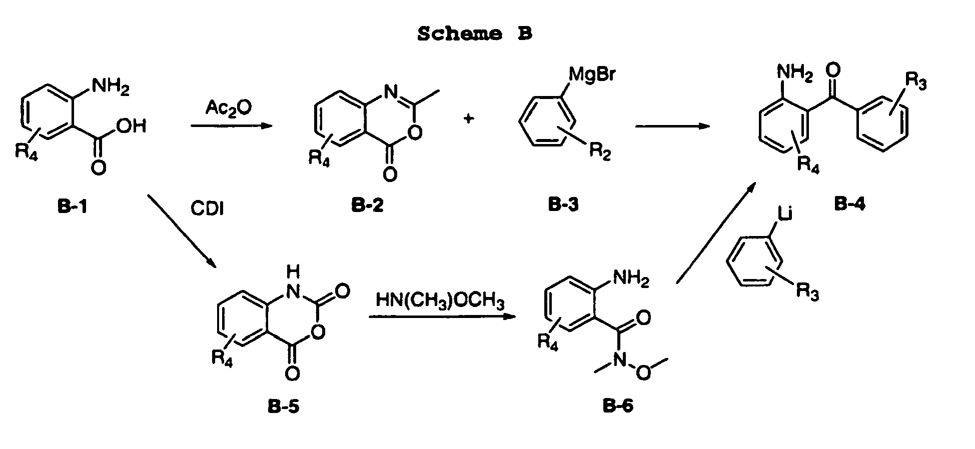

If the desired anthranilic acid is not commercially available it can be prepared by a variety of methods well known to those skilled in the art.

An alternative approach to make substituted 2-aminobenzophenones utilizes a benzyl nitrile and nitrobenzene derivative. As outlined in scheme C, treatment of nitrobenzene derivative C-1 and benzyl nitrile C-2 with a base like potassium hydroxide in ethanol gives the cyclized product C-3. Reduction of the benzoxisole with hydrogen in the presence of a palladium or nickel catalyst provides the desired 2-aminobenzophenone B-4. If a specific substituent is not stable to these reaction conditions, a variety of other reduction methods can be used. Some of them include iron and acetic acid, tin chloride and hydride reagents.

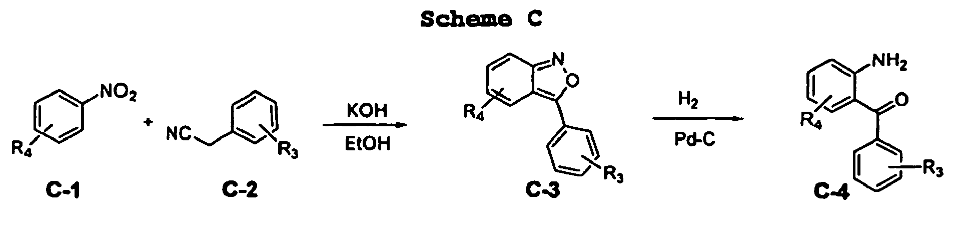

Another method for preparing compounds of the invention and reference compounds not of the invention utilizes the chemistry outlined in scheme D below. Here a substituted anthranilic acid ester, D-1, is treated with potassium isocyanate followed by aqueous sodium hydroxide to give the corresponding quinazolinedione, D-2. Subsequent treatment with POCl₃ provides dichlorointermediate D-3. Selective coupling to the 4'-position gives monochloride D-4. This displacement can be carried out using a variety of methods depending on the particular product of interest. For examples of D-4 where X is a bond, the coupling reaction can be conveniently carried out using a transition metal catalyzed coupling reaction. Some examples include coupling an aryl or heteroarylboronic acid, tin or zinc reagent with a palladium catalyst.

For examples of D-4 where X is nitrogen, the substituted amine derivative can typically be introduced directly using a simple displacement reaction. When X is NR₁R₂, NR₁R₂ may be an aromatic amine, such as indole.

Once the R₂ substituent is in place, the 2-amino group can be introduced as previously described.

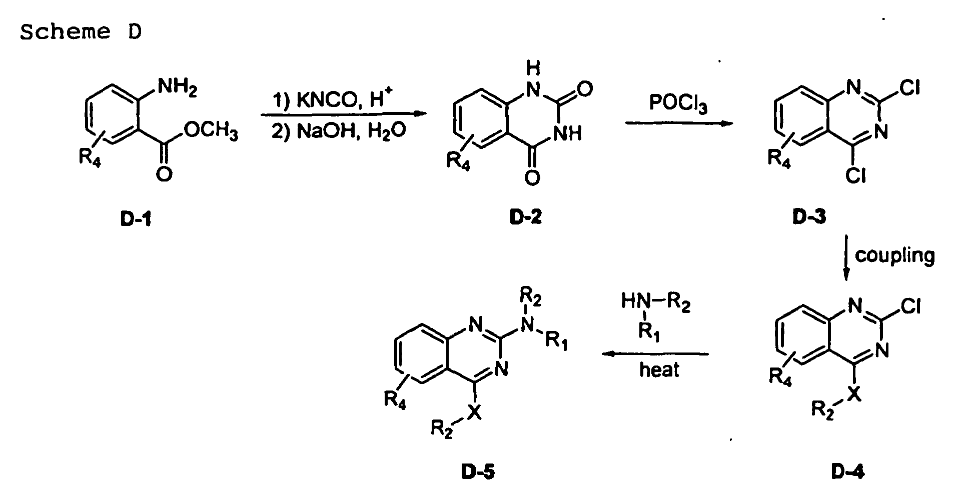

Another method for preparing compounds of the invention and reference compounds not of the invention utilizes the chemistry outlined in scheme E below. Here the amino nitrile is reacted with carbon dioxide to form the quinazoline dione, which is then coupled to an R₄ group (in scheme E, R₄ is furanyl) via a transition metal catalyzed reaction. The resulting coupled product is treated with a chloride source to form the dichloro quinazoline, which is then selectively coupled via a transition metal catalyzed reaction to form the mono-chloro quinazoline product (wherein R₃ is CN). The mono-chloro quinazoline product is then treated with a variety of amines, including cyclic, acyclic, and aromatic amines, to form the desired final product.

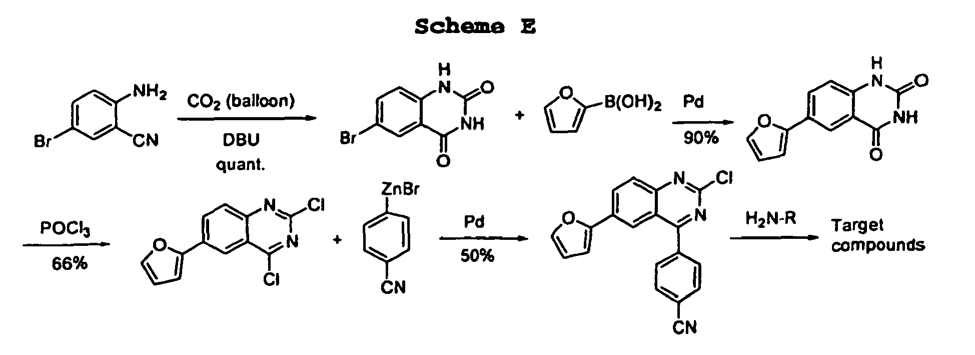

Another method for preparing compounds of the invention and reference compounds not of the invention utilizes the chemistry outlined in scheme F below. Here, the benzophenone is reacted with urea for form the quinazolinone prodct, which is then reacted with a chloride source to form the mono chloro quinazoline. The mono chloro quinazoline is then treated with an amine (here, 4-aminobenzenesulfonamide) to form the coupled product. The final product is then obtained by treating the coupled product with a transition metal catalyst and an appropriate R₄ group (here R₄ is furanyl.)

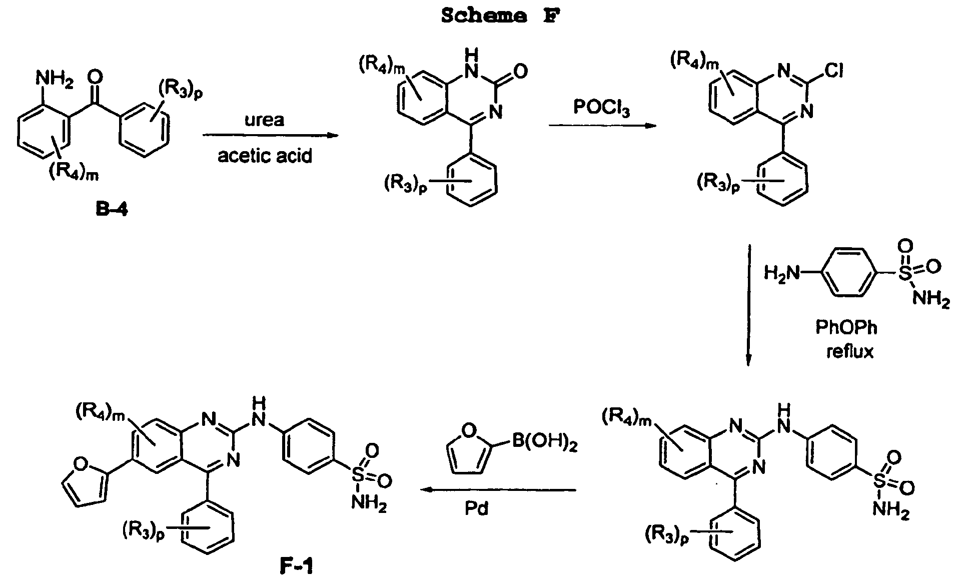

### Examples

### Reference Example 1

### Preparation of 2-Chloro-4,6-diphenylpyrimidine.

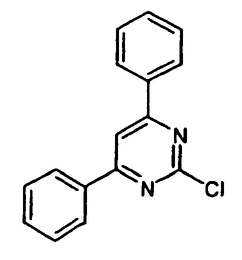

A mixture of 2,4,6-Trichloropyrimidine, 2.76g (15.0 mmol), phenylboronic acid, 3.66g (30.0 mmol), Pd(OAc)₂, 86mg (0.38 mmol), triphenylphosphine, 200mg (0.76 mmol) in 150mL of ethylene glycol dimethyl ether was heated to obtain a clear solution. To the solution was added 25mL of 4.0M aq. Na₂CO₃. The reaction mixture was refluxed for 24h at 70 ºC. The mixture was cooled to room temperature and diluted with 100mL ethyl acetate. The organic layer was washed with water (2x50mL), sat. aq. NaCl (1x50mL), and dried (MgSO₄). After the solution was concentrated, the residue was recrystallized with Et₂O-Heptane (1:3) to afford the desired product in 1.64g (41%) as a pale yellow solid. ¹H NMR (CDCl₃): δ 8.15-8.12 (m, 4H), 8.02 (s, 1H), 7.57-7.51 (m, 6H).

### Example 2 (Compound No. 50)

### Preparation of 4-{5-Bromo-1-[4-(4-butyl-phenyl)-6-furan-2-yl-quinazolin-2-yl]-1H-indol-3-yl}-4-oxo-butyric acid

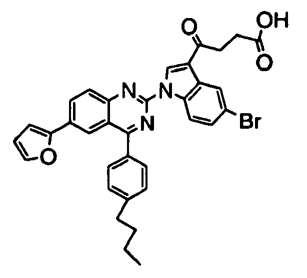

This compound was prepared in a manner analogous to that set forth in Example 53, except 4-n-butylphenylmagnesium bromide and ZnCl₂ (1 mole equivalent) was used instead of 2-pyridylzinc bromide in step 5. In step 7 the reaction was acidified (2N HCl), diluted with H₂O (10mL) and the organics were extracted with EtOAc (3 x 25 mL). The concentrated oil was redissolved in a minimal amount THF and heptane was added until a yellow solid precipitated out. The solid was filtered and dried to give 4-{5-Bromo-1-[4-(4-butyl-phenyl)-6-furan-2-yl-quinazolin-2-yl]-1*H-*indol-3-yl}-4-oxo-butyric acid: R_{f} 0.22 (Heptane/EtOAc, 3:2, v/v). ¹H NMR (DMSO-d₆, 300 MHz) δ 12.09 (br s, 1H) 9.27 (s, 1H), v/v). ¹H NMR (DMSO-d₆, 300 MHz) δ 12.09 (br s, 1H) 9.27 (s, 1H), 8.93 (d, *J* = 9 Hz, 1H), 8.40-8.44 (m, 2H), 8.28 (d, *J =* 2 Hz, 1H), 8.18 (d, *J* = 9 Hz, 1H), 7. 92 (d, *J* = 8 Hz, 2H), 7.83 (d, *J* = 2 Hz, 1H), 7.59 (dd, *J* =9, 2 Hz, 1H), 7.54 (d, *J =* 8 Hz, 2H), 7.19 (d, *J* = 3 Hz, 1H), 6.66 (dd, *J* = 3, 2 Hz, 1H), 3.31 (t, *J* = 6 Hz, 2H), 2.77 (t, *J* = 7.5Hz, 2H), 2.61 (t, *J* = 6Hz, 2H), 1.74-1.64 (m, 2H), 1.47-1.34 (m, 2H), .968 (t, *J* = 7.5 Hz, 3H). ESI-LCMS *m*/*z* calcd for C₃₄H₂₈BrN₃O₄ : 622.5 ; found 624.0 (M + 1)⁺.

### Reference Example 3 (Reference Compound No. 71)

### Preparation of 4-{5-Bromo-1-[4-(1-carboxy-2-phenyl-ethylamino)-6-furan-2-yl-quinazolin-2-yl]-1H-indole-3-yl}-4-oxo-butyric acid.

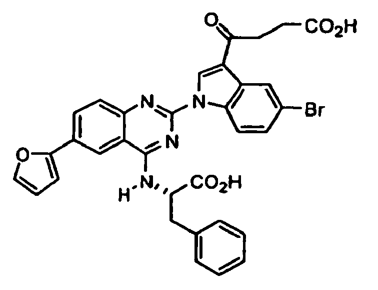

This compound was prepared in a manner analogous to that set forth in Example 64, except *L*-phenylalanine methyl ester was used instead of p-anisidine in step 5 to provide 4-{5-bromo-1-[4-(1-carboxy-2-phenyl-ethylamino)-6-furan-2-yl-quinazolin-2-yl]-1*H*-indole-3-yl}-4-oxo-butyric acid as a brown solid: R_{f} 0.64 (CH₂Cl₂/MeOH, 12:1, v/v) ¹H NMR (DMSO-*d*₆, 300 MHz) δ 9.19 (d, *J* = 7.2 Hz, 1 H), 9.09 (s, 1 H), 8.90 (d, *J* = 8.8 Hz, 1 H), 8.70 (d, *J* = 1.8 Hz, 1 H), 8.38 (d, *J* = 2.0 Hz, 1 H), 8.15 (dd, *J* = 8.7, 2.0 Hz, 1 H), 7.86 (d, *J* = 1.2 Hz, 1 H), 7.82 (d, *J* = 8.7 Hz, 1 H), 7.53 (dd, *J* = 8.7, 2.0 Hz, 1 H), 7.41 (d, *J* = 7.3 Hz, 1 H), 7.24 (t, *J* = 7.3 Hz, 2 H), 7.13 (t, *J* = 7.3 Hz, 2 H), 7.08 (d, *J* = 3.4 Hz, 1 H), 6.68 (dd, *J* = 3.4, 1.2 Hz, 1 H), 5.00 (q, *J* = 7.2 Hz, 1 H), 3.38 (d, *J* = 7.2 Hz, 1 H), 3.27 (t, *J* = 6.4 Hz, 2 H), 2.63 (t, *J* = 6.4 Hz, 2 H).
ESI-LCMS *m*/*z* calcd for C₃₃H₂₅BrN₄O₆: 652.1; found 653.0 (M + 1)⁺.

### Reference Example 4 (Reference Compound No. 72)

### Preparation of 4-{2-[5-Bromo-3-(3-carboxy-propionyl)-indol-1-yl]-6-furan-2-yl-quinazolin-4-yl}-piperazine-1-carboxylic acid tert-butyl ester

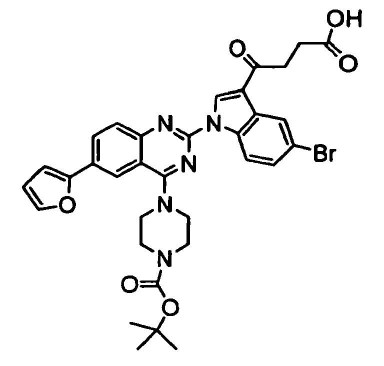

This compound was prepared in a manner analogous to that set forth in Example 64, except piperazine-1-carboxylic acid *tert*-butyl ester was used instead of p-anisidine in step 5 to provide 4-{2-[5-Bromo-3-(3-carboxy-propionyl)-indol-1-yl]-6-furan-2-yl-quinazolin-4-yl}-piperazine-1-carboxylic acid *tert-*butyl ester (0.40g, 41%) : R_{f} 0.34 (CH₂Cl₂/MeOH, 19:1, v/v). ¹H NMR (DMSO-*d*₆, 300 MHz) δ 9.14 (s, 1H), 8.88 (d, *J* = 9 Hz, 1H), 8.34 (d, *J* = 2 Hz, 1H), 8.20-8.17 (m, 2H), 7.92 (d, *J* = 9 Hz, 1H), 7.82 (d, *J* = 2 Hz, 1H), 7.56 (dd, *J* = 9, 2 Hz, 1H), 7.16 (d, *J* = 3 Hz, 1H), 6.65 (dd, *J* = 3, 2 Hz, 1H), 4.01 (m, 4H), 3.67 (m, 4H), 3.29 (t, *J* = 6 Hz, 2H), 2.61 (t, *J* = 6 Hz, 2H), 1.45 (s, 9H). ESI-LCMS *m*/*z* calcd for C₃₃H₃₂BrN₅O₆ : 674.5 ; found 676.0 (M + 1)⁺.

### Reference Example 5 (Reference Compound No. 120)

### 4-[5-Chloro-1-(4,6-diphenyl-pyrimidin-2-yl)-1H-indol-3-yl]-4-oxo-butyric acid 2-trimethylsilanylethyl ester.

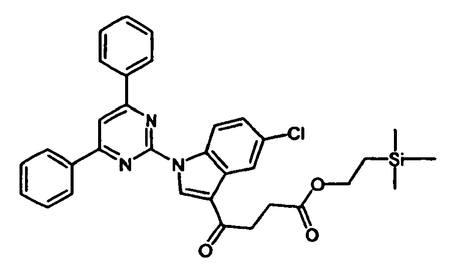

A mixture of 2-chloro-4,6-diphenylpyrimidine, 250mg (0.94 mmol), 4-(5-chloro-1H-indol-3-yl)-4-oxo-butyric acid 2-trimethylsilanyl-ethyl ester, 380mg (1.03 mmol), K₂CO₃ 260mg (1.87 mmol), and N,N-dimethylaminopyridine, 11mg (0.09 mmol) in 20mL DMSO was heated to 80°C for 6h. The mixture was cooled to room temperature and diluted with 100mL of EtOAc. The mixture was washed with sat. aq. LiCl (3x100mL), water (3x100mL), sat. aq. NaCl (1x100mL), and dried (MgSO₄). After the solution was concentrated, the residue was purified via column chromatography (eluted with 10% EtOAc-heptane) to afford the desired product in 0.47g (88%) as a pale yellow solid. ¹H NMR (DMSO-d₆): δ 9.39 (s, 1H), 8.87 (d, 1H, J = 9.7Hz), 8.56 (s, 1H), 8.51-8.48 (m, 3H), 8.26 (d, 1H, J = 2.7Hz), 7.64-7.62 (m, 5H), 7.52 (dd, 1H, 9.7, 2.7Hz), 4.10 (dd, 2H, J = 9.0, 9.0Hz), 3.40 (t, 2H, J = 7.2Hz), 2.67 (t, 2H, J = 7.2Hz), 0.94 (dd, 2H, J = 9.0, 9.0Hz), 0.00 (s, 9H).

### Reference Example 6 (Reference Compound 95)

### Preparation of 4-{5-Bromo-1-[4-(3,4-dihydro-1H-isoquinolin-2-yl)-6-pyrrolidin-1-yl-[1,3,5]-triazin-2-yl)-1H-indol-3-yl]-4-oxo-butyric acid.

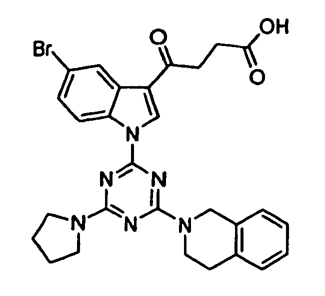

A solution of A-(5-bromo-1*H*-indol-3-yl)-4-oxobutyric acid (148 mg, 0.5 mmol) in anhydrous dimethylformamide (5 mL) was added dropwise to a stirred suspension of sodium hydride (95%, 50 mg, 2.0 mmol) in dimethylformamide (5 mL). After 30 mins, a solution of 1-(4-chloro-6-tetrahydro-1H-pyroll-1-yl-[1,3,5]-triazin-2-yl)-1,2,3,4-tetrahydroquinoline (158 mg, 0.5 mmol) in dimethylformamide (5 mL) was added dropwise. The reaction mixture was stirred at 70°C for 16 hours, cooled to room temperature and then poured carefully into water (20 mL), acidified to pH 4 with 0.5N hydrochloric acid and extracted with ethyl acetate (3 x 25 mL). The combined extract was washed with water, brine, dried over anhydrous MgSO₄, filtered and concentrated *in vacuo.* Purification by flash column chromatography (5% methanol in dichloromethane) afforded the title compound as a white solid (221 mg, 77 %), Rf: 0.30 (10% methanol in dichloromethane); 1H NMR (THF-d8, 300 MHz) δ 8.78 (1H, s, ArH), 8.43 (1H, d, J = 9 Hz, ArH), 8.36 (1H, d, J = 2 Hz, ArH), 7.77 (1H, d, J = 9 Hz, ArH), 7.20 (1H, dd, J = 9, 2 Hz, ArH), 7.08 (1H, t, J = 7 Hz, ArH), 7.02 (1H, d, J = 7 Hz), 6.92 (1H, t, J = 7 Hz, Ar-H), 3.98 (2H, t, J = 6 Hz, CH₂N), 3.45 (4H, br s, 2 x CH₂N), 3.06 (2H, t, J = 6 Hz, CH₂CO), 2.78 (1H, s, CHHN), 2.72 (2H, t, J = 7 Hz, CH₂CO), 2.64 (1H, s, CHHN), 2.56 (2H, t, J = 7 Hz, CH₂), 1.92 (4H, m, CH₂CH₂); ESI-LCMS e/z calculated for C₂₈H₂₇BrN₆O₃ 575.464, found 575 [M+H (⁷⁹Br)]⁺, 577 [M+H (⁸¹Br)]⁺, 597 [M+Na (⁷⁹Br)]⁺, 599 [M+Na (⁸¹Br)]⁺.

### Example 7

The following compounds are prepared essentially according to the procedures described in the schemes, charts, examples and preparations set forth herein.

These compounds were named using ChemDraw v. 6.02, which is sold by Cambridgesoft.com in Cambridge, MA, or using Name Pro IUPAC Naming Software, version 5.09, available from Advanced Chemical Development, Inc., 90 Adelaide Street West, Toronto, Ontario, M5H 3V9, Canada.

| Compound No. | Structure | Name |
|---|---|---|
| 1* | 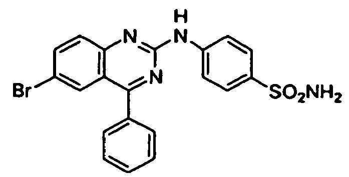 | 4-(6-Bromo-4-phenyl-quinazolin-2-ylamino)-benzenesulfonamide |
| 2* | 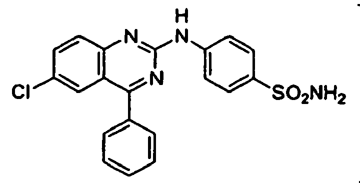 | 4-(6-Chloro-4-phenyl-quinazolin-2-ylamino)-benzenesulfonamide |
| 3* | 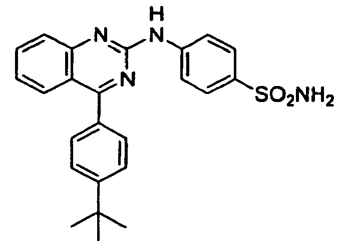 | 4-[4-(4-tert-Butyl-phenyl)-quinazolin-2-ylamino]-benzenesulfonamide |
| 4* | | 2-{[4-(6-Bromo-4-phenyl-quinazolin-2-ylamino)-benzenesulfonyl]-methyl-amino}-3-phenyl-propionic acid |
| 5* | 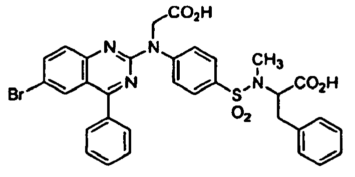 | 2-({4-[(6-Bromo-4-phenyl-quinazolin-2-yl)-carboxymethyl-amino]-benzenesulfonyl}-methyl-amino)-3-phenyl-propionic acid |
| 6* | | [(6-Bromo-4-phenyl-quinazolin-2-yl)-(4-sulfamoyl-phenyl)-amino]-acetic acid |
| 7* | | 2-(6-Bromo-4-phenyl-quinazolin-2-ylamino)-benzenesulfonamide |
| 8* | | 4-[(6-Bromo-4-phenyl-quinazolin-2-yl)-(4-sulfamoyl-phenyl)-amino]-butyric acid |
| 9* | 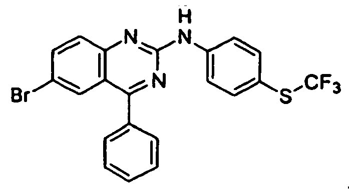 | (6-Bromo-4-phenyl-quinazolin-2-yl)-(4-trifluoromethylsulfanyl-phenyl)-amine |
| 10* | | 3-(6-Bromo-4-phenyl-quinazolin-2-ylamino)-benzenesulfonamide |
| 11* | 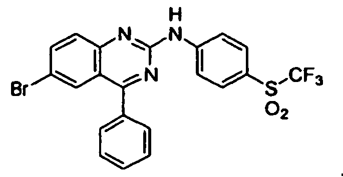 | (6-Bromo-4-phenyl-quinazolin-2-yl)-(4-trifluoromethanesulfonyl-phenyl)-amine |
| 12* | 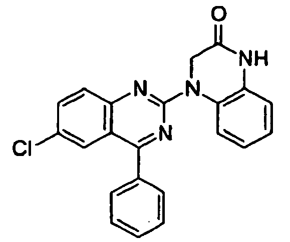 | 4-(6-Chloro-4-phenyl-quinazolin-2-yl)-3,4-dihydro-1H-quinoxalin-2-one |
| 13* | | N-[4-(6-Bromo-4-phenyl-quinazolin-2-ylamino)-phenyl]-acetamide |
| 14* | | 1-[4-(6-Bromo-4-phenyl-quinazolin-2-ylamino)-phenyl]-ethanone |
| 15* | | [3-(6-Bromo-4-phenyl-quinazolin-2-ylamino)-phenyl]-piperidin-1-yl-methanone |
| 16* | | [3-(6-Bromo-4-phenyl-quinazolin-2-ylamino)-phenyl]-morpholin-4-yl-methanone |
| 17* | | (6-Chloro-4-phenyl-quinazolin-2-yl)-(4-nitro-phenyl)-amine |
| 18* | 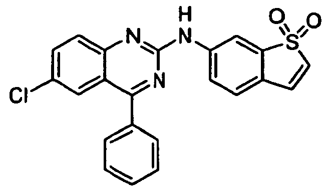 | 6-chloro-*N*-(1,1-dioxido-1-benzothien-6-yl)-4-phenylquinazolin-2-amine |
| 19* | | 2-(6-Bromo-4-phenyl-quinazolin-2-ylamino)-3-phenyl-propionic acid |
| 20* | | 2-[6-Bromo-4-(4-butyl-phenyl)-quinazolin-2-ylamino]-benzenesulfonamide |
| 21* | 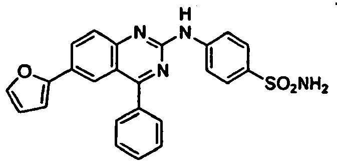 | 4-(6-Furan-2-yl-4-phenyl-quinazolin-2-ylamino)-benzenesulfonamide |
| 22* | | 4-(6-Chloro-4-phenyl-quinazolin-2-ylamino)-benzoic acid methyl ester |
| 23* | | N-(2,4-Dimethyl-phenyl)-4-(6-methyl-4-phenyl-quinazolin-2-ylamino)-benzamide |
| 24* | 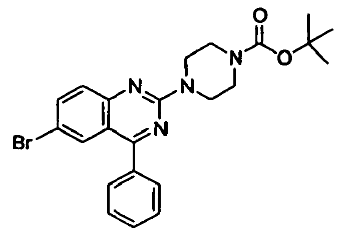 | 4-(6-Bromo-4-phenyl-quinazolin-2-yl)-piperazine-1-carboxylic acid tert-butyl ester |
| 25* | | [4-(6-Bromo-4-phenyl-quinazolin-2-ylamino)-phenyl]-piperidin-1-yl-methanone |
| 26* | 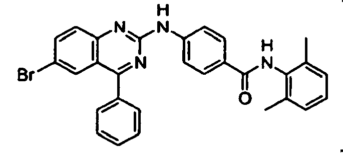 | 4-(6-Bromo-4-phenyl-quinazolin-2-ylamino)-N-(2,6-dimethyl-phenyl)-benzamide |
| 27* | | (6-Bromo-4-phenyl-quinazolin-2-yl)-(4-nitro-phenyl)-amine |
| 28* | | (6-Bromo-4-phenyl-quinazolin-2-yl)-(2-methyl-5-nitro-phenyl)-amine |
| 29* | 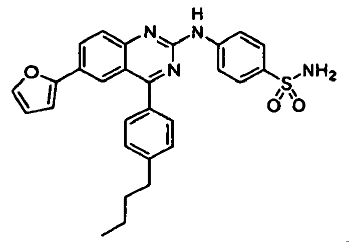 | 4-[4-(4-Butyl-phenyl)-6-furan-2-yl-quinazolin-2-ylamino]-benzenesulfonamide |
| 30* | | [6-Chloro-4-(4-ethyl-phenyl)-quinazolin-2-yl]-(4-nitro-phenyl)-amine |
| 31* | 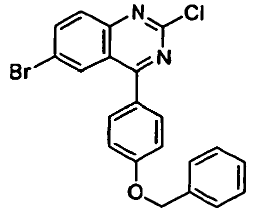 | 4-(4-Benzyloxy-phenyl)-6-bromo-2-chloro-quinazoline |
| 32* | 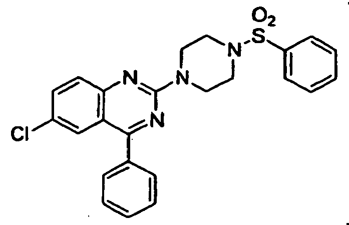 | 2-(4-Benzenesulfonyl-piperazin-1-yl)-6-chloro-4-phenyl-quinazoline |
| 33* | 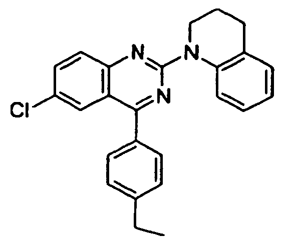 | 6-Chloro-2-(3,4-dihydro-2H-quinolin-1-yl)-4-(4-ethyl-phenyl)-quinazoline |
| 34* | | (6-Chloro-4-phenyl-quinazolin-2-yl)-(2-nitro-phenyl)-amine |
| 35* | | 2-(6-Furan-2-yl-4-phenyl-quinazolin-2-ylamino)-benzenesulfonamide |
| 36* | | [(6-Chloro-4-phenyl-quinazolin-2-yl)-(2-nitro-phenyl)-amino]-acetic acid ethyl ester |
| 37 | | 1-(6-Chloro-4-phenyl-quinazolin-2-yl)-1H-indole-5-carboxylic acid methyl ester |
| 38* | 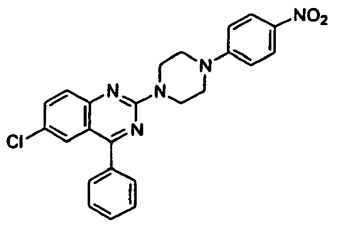 | 6-Chloro-2-[4-(4-nitro-phenyl)-piperazin-1-yl]-4-phenyl-quinazoline |
| 39 | 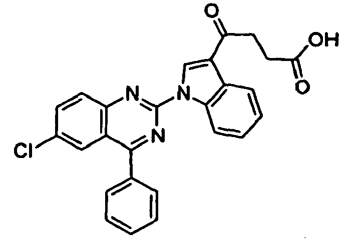 | 4-[1-(6-Chloro-4-phenyl-quinazolin-2-yl)-1H-indol-3-yl]-4-oxo-butyric acid |
| 40 | | 4-[5-Bromo-1-(6-chloro-4-phenyl-quinazolin-2-yl)-1H-indol-3-yl]-4-oxo-butyric acid |
| 41 | | 4-[5-Bromo-1-(6-fluoro-4-phenyl-quinazolin-2-yl)-1H-indol-3-yl]-4-oxo-butyric acid |
| 42 | | 4-{1-[6-Bromo-4-(2-fluoro-phenyl)-quinazolin-2-yl]-1H-indol-3-yl}-4-oxo-butyric acid |
| 43 | | 4-{5-Bromo-1-[6-furan-2-yl-4-(4-methoxy-phenyl)-quinazolin-2-yl]-1H-indol-3-yl}-4-oxo-butyric acid |
| 44 | | 4-{5-Bromo-1-[4-(4-cyano-phenyl)-6-furan-2-yl-quinazolin-2-yl]-1H-indol-3-yl}-4-oxo-butyric acid |
| 45 | | 4-{5-Bromo-1-[4-(4-fluoro-phenyl)-6-furan-2-yl-quinazolin-2-yl]-1H-indol-3-yl}-4-oxo-butyric acid |
| 46 | | 4-[1-(6-Chloro-4-phenyl-quinazolin-2-yl)-6-fluoro-1H-indol-3-yl]-4-oxo-butyric acid |
| 47 | | 4-[1-(6-Furan-2-yl-4-phenyl-quinazolin-2-yl)-1H-indol-3-yl]-4-oxo-butyric acid |
| 48 | | 4-[5-Bromo-1-(6-furan-2-yl-4-phenyl-quinazolin-2-yl)-1H-indol-3-yl]-4-oxo-butyric acid |
| 49 | | 4-{1-[4-(4-Butyl-phenyl)-6-furan-2-yl-quinazolin-2-yl]-1H-indol-3-yl}-4-oxo-butyric acid |
| 50 | 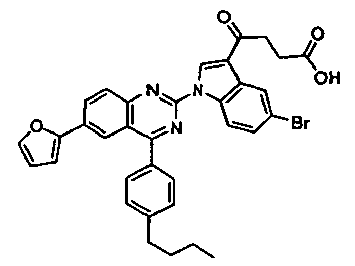 | 4-{5-Bromo-1-[4-(4-butyl-phenyl)-6-furan-2-yl-quinazolin-2-yl]-1H-indol-3-yl}-4-oxo-butyric acid |
| 51 | | 4-[5-Chloro-1-(6-chloro-4-phenyl-quinazolin-2-yl)-1H-indol-3-yl]-4-oxo-butyric acid |
| 52* | | 4-[5-Bromo-1-(6-chloro-4-phenyl-quinolin-2-yl)-1H-indol-3-yl]-4-oxo-butyric acid |
| 53* | | 4-[5-Bromo-1-(6-furan-2-yl-4-pyridin-2-yl-quinazolin-2-yl)-1H-indol-3-yl]-4-oxo-butyric acid |
| 54* | | 4-[5-Chloro-1-(6-chloro-4-phenyl-quinazolin-2-yl)-1H-indol-3-yl]-4-hydroxy-butyric acid |
| 55 | | {[5-Chloro-1-(6-chloro-4-phenyl-quinazolin-2-yl)-1H-indole-3-carbonyl]-amino}-acetic acid |
| 56 | | 3-(3-Carboxy-propionyl)-1-(6-chloro-4-phenyl-quinazolin-2-yl)-1H-indole-5-carboxylic acid methyl ester |
| 57* | | 4-[5-Chloro-1-(6-chloro-4-phenyl-quinazolin-2-yl)-1H-indol-3-yl]-N-hydroxy-4-oxo-butyramide |
| 58 | | 3-{[5-Chloro-1-(6-chloro-4-phenyl-quinazolin-2-yl)-1H-indole-3-carbonyl]-amino}-propionic acid |
| 59* | | 3-[5-Chloro-1-(6-chloro-4-phenyl-quinazolin-2-yl)-1H-indol-3-yl]-3-hydroxy-propionic acid |
| 60 | 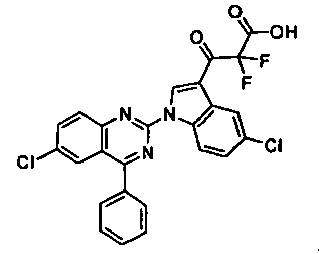 | 3-[5-Chloro-1-(6-chloro-4-phenyl-quinazolin-2-yl)-1H-indol-3-yl]-2,2-difluoro-3-oxo-propionic acid |
| 61 | 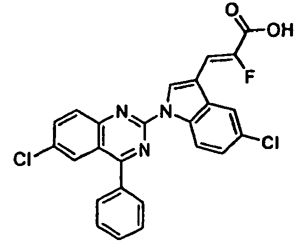 | 3-[5-Chloro-1-(6-chloro-4-phenyl-quinazolin-2-yl)-1H-indol-3-yl]-2-fluoro-acrylic acid |
| 62 | | 3-[5-Chloro-1-(6-chloro-4-phenyl-quinazolin-2-yl)-1H-indol-3-yl]-3-oxo-propionic acid |
| 63 | | 4-[6-Chloro-1-(6-chloro-4-phenyl-quinazolin-2-yl)-1H-indol-3-yl]-4-oxo-butyric acid |
| 64* | | 4-{5-Bromo-1-[6-furan-2-yl-4-(4-methoxy-phenylamino)-quinazolin-2-yl]-1H-indol-3-yl)-4-oxo-butyric acid |
| 65* | | 4-{5-Bromo-1-[6-furan-2-yl-4-(4-methoxy-benzylamino)-quinazolin-2-yl]-1H-indol-3-yl)-4-oxo-butyric acid |
| 66* | | 4-(5-Bromo-1-{6-furan-2-yl-4-[(furan-2-ylmethyl)-amino]-quinazolin-2-yl}-1H-indol-3-yl)-4-oxo-butyric acid |
| 67* | | 4-(5-Chloro-1-[4-(4-fluoro-benzylamino)-6-furan-2-yl-quinazolin-2-yl]-1H-indol-3-yl)-4-oxo-butyric acid |
| 68* | | 4-[5-Chloro-1-(6-furan-2-yl-4-pyridin-2-yl-quinazolin-2-yl)-1H-indol-3-yl]-4-oxo-butyric acid |
| 69* | | 4-(5-Chloro-1-{6-furan-2-yl-4-[(pyridin-4-ylmethyl)-amino]-quinazolin-2-yl}-1H-indol-3-yl)-4-oxo-butyric acid |
| 70* | | 4-{5-Chloro-1-[6,7-dimethoxy-4-(4-methoxy-benzylamino)-quinazolin-2-yl]-1H-indol-3-yl}-4-oxo-butyric acid |
| 71* | 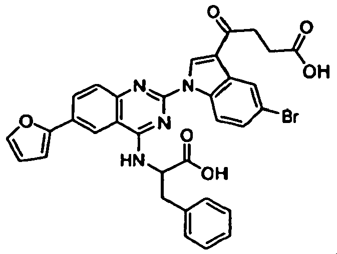 | 4-{5-Bromo-1-[4-(1-carboxy-2-phenyl-ethylamino)-6-furan-2-yl-quinazolin-2-yl]-1H-indol-3-yl}-4-oxo-butyric acid |
| 72* | 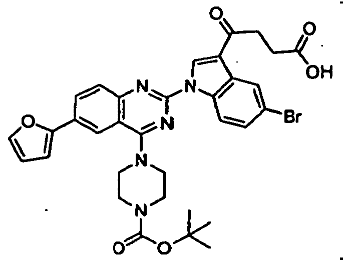 | 4-{2-[5-Bromo-3-(3-carboxy-propionyl)-indol-1-yl]-6-furan-2-yl-quinazolin-4-yl}-piperazine-1-carboxylic acid tert-butyl ester |
| 73* | | 4-[5-Bromo-1-(6-furan-2-yl-4-morpholin-4-yl-quinazolin-2-yl)-1H-indol-3-yl]-4-oxo-butyric acid |
| 74* | | 1-{2-[5-Bromo-3-(3-carboxy-propionyl)-indol-1-yl]-6-furan-2-yl-quinazolin-4-yl}-piperidine-4-carboxylic acid |
| 75* | | 1-{2-[3-(3-Carboxy-propionyl)-5-chloro-indol-1-yl]-6-furan-2-yl-quinazolin-4-yl}-piperidine-4-carboxylic acid ethyl ester |
| 76* | | 1-{2-[3-(3-Carboxy-propionyl)-5-chloro-indol-1-yl]-6-furan-2-yl-quinazolin-4-yl}-piperidine-4-carboxylic acid |
| 77* | | 1-{2-[5-Bromo-3-(3-carboxy-propionyl)-indol-1-yl]-6-furan-2-yl-quinazolin-4-yl}-piperidine-4-carboxylic acid ethyl ester |
| 78* | 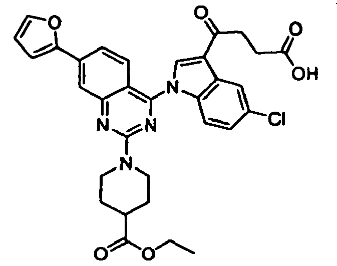 | 1-(4-[3-(3-Carboxy-propionyl)-5-chloro-indol-1-yl]-7-furan-2-yl-quinazolin-2-yl}-piperidine-4-carboxylic acid ethyl ester |
| 79* | | 4-[5-Chloro-1-(6-furan-2-yl-4-morpholin-4-yl-quinazolin-2-yl)-1H-indol-3-yl]-4-oxo-butyric acid |
| 80* | | 1-{2-[3-(3-Carboxy-propionyl)-5-chloro-indol-1-yl]-6-nitro-quinazolin-4-yl}-piperidine-4-carboxylic acid ethyl ester |
| 81* | | 4-{5-Chloro-1-[6-furan-2-yl-4-(4-hydroxy-piperidin-1-yl)-quinazolin-2-yl]-1H-indol-3-yl}-4-oxo-butyric acid |
| 82* | | 1-[2-[3-(3-Carboxy-propionyl)-5-chloro-indol-1-yl]-6-(2-oxo-pyrrolidin-1-yl)-quinazolin-4-yl]-piperidine-4-carboxylic acid ethyl ester |
| 83* | | 4-(5-Chloro-1-[4-(4-hydroxy-piperidin-1-yl)-6-trifluoromethyl-quinazolin-2-yl]-1H-indol-3-yl)-4-oxo-butyric acid |
| 84* | | 4-(5-Chloro-1-[4-(4-hydroxymethyl-piperidin-1-yl)-6-trifluoromethyl-quinazolin-2-yl]-1H-indol-3-yl}-4-oxo-butyric acid |
| 85* | 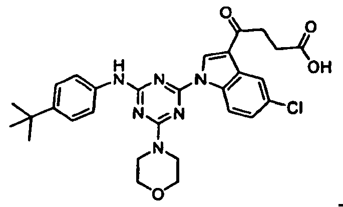 | 4-{1-[4-(4-tert-Butyl-phenylamino)-6-morpholin-4-yl-[1,3,5]triazin-2-yl]-5-chloro-1H-indol-3-yl}-4-oxo-butyric acid |
| 86* | | 2-{2-[5-Bromo-3-(3-carboxy-propionyl)-indol-1-yl]-6-furan-2-yl-quinazolin-4-ylamino}-butyric acid |
| 87* | | 2-{2-[3-(3-Carboxy-propionyl)-5-chloro-indol-1-yl]-6-furan-2-yl-quinazolin-4-ylamino}-butyric acid |
| 88* | | 4-{5-Chloro-1-[6-furan-2-yl-4-(2-methanesulfonyl-ethylamino)-quinazolin-2-yl]-1H-indol-3-yl}-4-oxo-butyric acid |
| 89* | | 4-{1-[4-(Carbamoylmethyl-amino)-6-furan-2-yl-quinazolin-2-yl]-5-chloro-1H-indol-3-yl}-4-oxo-butyric acid |
| 90* | | 4-[5-Cyano-1-(4,6-diphenyl-pyrimidin-2-yl)-1H-indol-3-yl]-4-oxo-butyric acid |
| 91* | 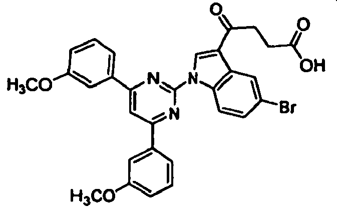 | 4-{1-[4,6-Bis-(3-methoxy-phenyl)-pyrimidin-2-yl]-5-bromo-1H-indol-3-yl}-4-oxo-butyric acid |
| 92* | | 4-[5-Chloro-1-(4,6-diphenyl-pyrimidin-2-yl)-1H-indol-3-yl]-4-oxo-butyric acid |
| 93* | | 4-[6-Chloro-1-(4,6-diphenyl-pyrimidin-2-yl)-1H-indol-3-yl]-4-oxo-butyric acid |
| 94* | | 4-[5-Bromo-1-(4,6-diphenyl-pyrimidin-2-yl)-1H-indol-3-yl]-4-oxo-butyric acid |
| 95* | 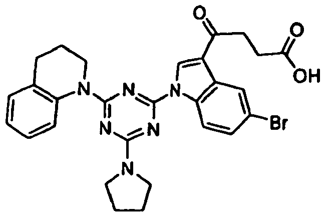 | 4-{5-Bromo-1-[4-(3,4-dihydro-2H-quinolin-1-yl)-6-pyrrolidin-1-yl-[1,3,5]triazin-2-yl]-1H-indol-3-yl}-4-oxo-butyric acid |
| 96* | | 4-{5-Chloro-1-[4-(3-methoxy-phenyl)-6-phenyl-pyrimidin-2-yl]-1H-indol-3-yl}-4-oxo-butyric acid |
| 97* | | 4-[5-Chloro-1-(4-phenyl-6-phenylamino-pyrimidin-2-yl)-1H-indol-3-yl]-4-oxo-butyric acid |
| 98* | | 4-{5-Chloro-1-[6-(4-chloro-phenyl)-2-phenyl-pyrimidin-4-yl]-1H-indol-3-yl}-4-oxo-butyric acid |
| 99* | | 4-{5-Chloro-1-[5-(4-chloro-phenyl)-pyrimidin-2-yl]-1H-indol-3-yl}-4-oxo-butyric acid |
| 100* | | 4-{5-Chloro-1-[4-(dibenzofuran-4-ylamino)-benzyl]-1H-indol-3-yl}-4-oxo-butyric acid |
| 101* | | 4-{5-Chloro-1-[4-(2-methoxy-dibenzofuran-3-ylamino)-benzyl]-1H-indol-3-yl}-4-oxo-butyric acid |
| 102* | | 4-[5-Chloro-1-(4-thianthren-1-yl-benzyl)-1H-indol-3-yl]-4-oxo-butyric acid |
| 103* | | 4-[5-Chloro-1-(4-dibenzofuran-4-yl-benzyl)-1H-indol-3-yl]-4-oxo-butyric acid |
| 104* | | 4-[5-Chloro-1-(4-dibenzothiophen-4-yl-benzyl)-1H-indol-3-yl]-4-oxo-butyric acid |
| 105* | | 4-{5-Chloro-1-[4-(4-chloro-phenyl)-5-(4-methoxy-phenyl)-thiazol-2-yl]-1H-indol-3-yl}-4-oxo-butyric acid |
| 106* | 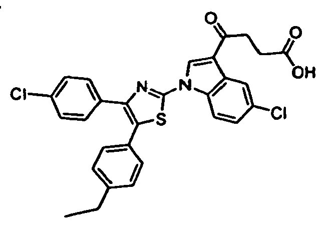 | 4-{5-Chloro-1-[4-(4-chloro-phenyl)-5-(4-ethyl-phenyl)-thiazol-2-yl]-1H-indol-3-yl}-4-oxo-butyric acid |
| 107* | | 4-{5-Chloro-1-[4-(4-fluoro-phenyl)-5-(4-methoxy-phenyl)-thiazol-2-yl]-1H-indol-3-yl}-4-oxo-butyric acid |
| 108* | | 4-{5-Chloro-1-[2-oxo-2-(5,5,8,8-tetramethyl-5,6,7,8-tetrahydro-naphthalen-2-yl)-ethyl]-1H-indol-3-yl}-4-oxo-butyric acid |
| 109* | | 4-{5-Chloro-1-[2-(5-chloro-3-methyl-benzo[b]thiophen-2-yl)-2-oxo-ethyl]-1H-indol-3-yl}-4-oxo-butyric acid |
| 110* | | 4-[5-Bromo-1-(6-furan-2-yl-4-hydroxy-quinazolin-2-yl]-1H-indole-3-yl]-4-oxo-butyric acid |
| 111* | | 4-(5-Bromo-1-{6-furan-2-yl-4-[(furan-2-ylmethyl)-amino]-quinazolin-2-yl}-1H-indole-3-yl)-4-oxo-butyric acid |
| 112* | | 4-{5-Chloro-1-[6-furan-2-yl-4-(4-hydroxymethyl-piperidin-1-yl)-quinazolin-2-yl]-1H-indol-3-yl}-4-oxo-butyric acid |
| 113* | | 4-(5-Chloro-1-(4-[(furan-2-ylmethyl)-amino]-6,7-dimethoxy-quinazolin-2-yl)-1H-indol-3-yl)-4-oxo-butyric acid |
| 114* | | 1-{6-Amino-2-[3-(3-carboxy-propionyl)-5-chloro-indol-1-yl]-quinazolin-4-yl}-piperidine-4-carboxylic acid ethyl ester |
| 115* | | 4-[1-(6-Amino-4-morpholin-4-yl-quinazolin-2-yl)-5-chloro-1H-indol-3-yl]-4-oxo-butyric acid. |
| 116* | | 4-{5-Chloro-1-[4-(4-methoxy-benzylamino)-6-trifluoromethyl-quinazolin-2-yl]-1H-indol-3-yl}-4-oxo-butyric acid |
| 117* | | 4-{5-Chloro-1-[4-(4-methoxy-phenylamino)-6-trifluoromethyl-quinazolin-2-yl]-1H-indol-3-yl}-4-oxo-butyric acid |
| 118* | | 2-Chloro-4,6-diphenylpyrimidine |
| 119* | | 4-(5-Chloro-1H-indol-3-yl)-4-oxo-butyric acid 2-trimethylsilanyl-ethyl ester |
| 120* | 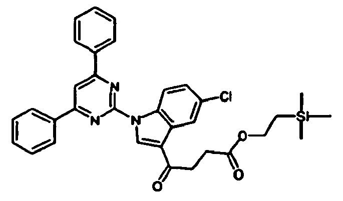 | 4-[5-Chloro-1-(4,6-diphenyl-pyrimidin-2-yl)-1H-indol-3-yl]-4-oxo-butyric acid 2-trimethylsilanylethyl ester |
| 121* | | 4-[5-Chloro-1-(2,6-diphenyl-pyrimidin-4-yl)-1H-indol-3-yl]-4-oxo-butyric acid |
| 122* | | 4-[5-Chloro-1-(3-cyano-4,6-diphenyl-pyridin-2-yl)-1H-indol-3-yl]-4-oxo-butyric acid |
| 123* | | 2-[4-(4-Butyl-phenyl)-6-furan-2-yl-quinazolin-2-ylamino]-benzenesulfonamide |

| | | |
|---|---|---|
| ("*" indicates Reference Compounds) | | |

### Reference Example 7a (Reference compound 123)

### Preparation of 2-[4-(4-Butyl-phenyl)-6-furan-2-yl-quinazolin-2-ylamino]-benzenesulfonamide

### Step 1: 6-Bromo-1H-benzo[d] [1,3]oxazine-2,4-dione

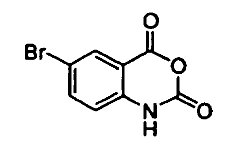

A solution of 2-amino-5-bromo-benzoic acid (15 g, 69 mmol) in acetic anhydride (60 mL) was heated to 130 °C. After 16 h, the solution was concentrated, and subsequently azeotroped with toluene (3 x 100 mL) to remove the remaining acetic anhydride. The resulting brown solid was dried in vacuo to provide 6-bromo-2-methyl-benzo[d] [1,3] oxazin-4-one.

### Step 2: (2-Amino-5-bromo-phenyl)-(4-butyl-phenyl)-methanone

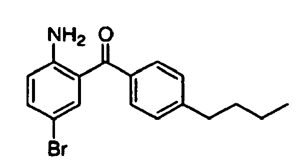

A solution of 6-bromo-2-methyl-benzo[d] [1,3]oxazin-4-one (8.5 g, 35 mmol) in CH₂Cl₂ (100 mL) was cooled to -78 °C and treated with 4-n-butyl phenyl magnesium bromide (71 mL, 35 mmol, 0.5 M in THF). The reaction was slowly warmed to room temperature and stirred overnight and quenched with sat'd ammonium chloride (30 mL). After stirring one hour, the organic layer was extracted with ethyl acetate, dried over sodium sulfate and concentrated to an orange oil. The oil was dissolved in MeOH (50 mL) and treated with 2 N HCl (5 equiv, 177 mmol). After refluxing overnight, the solution was cooled to 0 °C and basified with 1 N NaOH to pH 8. The organic layer was extracted with ethyl acetate, dried over sodium sulfate and concentrated. Purification by flash column chromatography. (5% ethyl acetate in heptane) gave 2-amino-5-bromo-phenyl - 4-butylphenyl-methanone (6.88 g, 60%) as a yellow solid.

### Step 3: 6-Bromo-4-(4-butyl-phenyl)-1H-quinazolin-2-one

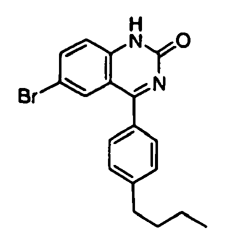

A solution of 2-amino-5-bromo-phenyl-4-butyl-phenyl-methanone (6 g, 18 mmol) in acetic acid (50 mL) was treated with urea (3.25 g, 54 mmol) and heated to 130 °C for 4 h. After cooling to room temperature, the solution was concentrated. The resulting solid was dissolved in a minimal amount of CH₂Cl₂ and precipitated with heptane. The resulting solid was filtered, washed with ethyl acetate and dried in vacuo to give 6-bromo-4-butyl-phenyl-1H-quinazolin-2-one (5.50 g, 85%) as a light yellow solid.

### Step 4: 6-Bromo-4-(4-butyl-phenyl)-2-chloro-quinazoline

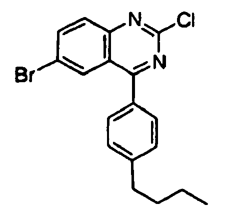

In a glass tube sealed with a teflon cap, a solution of 6-bromo-4-butyl-phenyl-1H-quinazolin-2-one (1 g, 2.8 mmol) in POCl₃ (5 mL) was heated to 130 °C for 2 hours. After cooling to room temperature, the solution was cautiously added to ice water. The resulting precipitate was extracted with CH₂Cl₂, dried over sodium sulfate and concentrated. Purification by flash column chromatography (5% ethyl acetate in heptane) gives 6-bromo-4-(4-butyl-phenyl)-2-chloro-quinazoline (0.59 g, 56%).

### Step 5: 2-[6-Bromo-4-(4-butyl-phenyl)-quinazolin-2-ylamino]-benzenesulfonamide

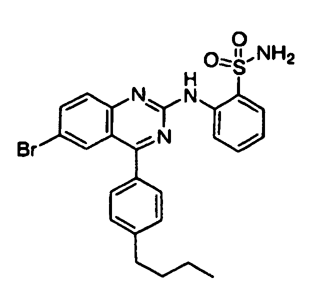

In a glass tube sealed with a teflon cap, a solution of 6-bromo-4-(4-butyl-phenyl)-2-chloro-quinazoline (0.212 g, 0.56 mmol) and 2-aminobenzenesulfanilamide (0.117 g, 0.68 mmol) in diphenyl ether (1 mL) was heated to 175 °C for 48 h. After cooling to room temperature, purification by flash column chromatography (5-30% ethyl acetate / heptane) gives 2-[6-bromo-4-(4-butyl-phenyl)-quinazolin-2-ylamino]-benzenesulfonamide (0.092g, 32%).

### Step 6: 2-[4-(4-Butyl-phenyl)-6-furan-2-yl-quinazolin-2-ylamino]-benzenesulfonamide

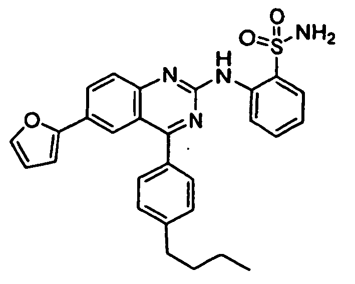

A solution of 2-[6-bromo-4-(4-butyl-phenyl)-quinazolin-2-ylamino]-benzenesulfonamide (0.08g, 0.16 mmol), 2-furanboronic acid (0.023 g, .19 mmol). Na₂CO₃ (0.166 g, 1.6 mmol) and Pd(PPh₃)₄ (0.018g, 0.016 mmol) in ethylene glycol dimethyl ether (3 mL) and water (1 mL) was heated to 80 °C. After stirring for 2 h, the solution was diluted with H₂O (10 mL) and extracted with ethyl acetate. Purification by flash column chromatography (10-40 % ethyl acetate / heptane) gives 2-[4-(4-butyl-phenyl)-6-furan-2-yl-quinazolin-2-ylamino]-benzenesulfonamide (0.036g, 46 %). R_{f} = 20 (5% MeOH in CH₂Cl₂).

### Reference Example 8 (Reference compound 53)

### Preparation of 4-[5-Bromo-1-(6-furan-2-yl-4-pyridin-2-yl-quinazolin-2-yl)-1H-indole-3-yl]-4-oxo-butyric acid

### Step 1: 2-Amino-5-bromo-benzonitrile

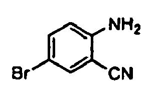

Prepared according to the literature procedure by Roche, D. Prasad, K.; Repic, O. ; Blacklock, T. J. Tetrahedron Lett. 2000, 41, 2083. R_{f} 0.46 (40% ethyl acetate in heptane) ¹H NMR (CDCl₃, 300 MHz) δ 7.47 (d, *J* = 2.3 Hz, 1 H), 7.39 (dd, *J₁* = 8.9 Hz, *J₂* = 2.2 Hz, 1 H), 6.62 (d, *J* = 8.9 Hz, 1 H). ESI-LCMS *m*/*z* calcd for C₇H₅BrN₂ : 196.0 ; found 197.0 (M + 1)⁺.

### Step 2: 6-Bromo-1H-quinazoline-2,4-dione

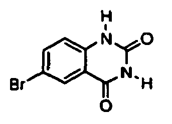

Prepared according to the literature procedure by Mizuno, T.; Okamoto, N, Ito, T.; Miyata, T. Tetrahedron Lett. 2000, 41, 1051 with some modifications.
A mixture of 2-amino-5-bromo-benzonitrile (10 g, 52 mmol) and 1,8-diazobicyclo[5.4.0]-undec-7-ene (DBU) (30 mL, 200 mmol) in dimethylformamide (DMF) (50 mL) was warmed (100 °C bath) with stirring under an atmosphere of carbon dioxide from an attached latex balloon for 48 hours. The solution was removed from the heat, added to cooled (ice bath) 1 N HCl (500 mL) and the solids were collected, washed with H₂O and dried to provide 6-bromo-1*H*-quinazoline-2,4-dione as a yellow solid (12 g, quantitative): R_{f} 0.27 (5% methanol in dichloromethane) ¹H NMR (DMSO-d₆, 300 MHz) δ 11.41 (s, 1 H), 11.27 (s, 1 H), 7.91 (d, *J* = 2.3 Hz, 1 H), 7.77 (dd, *J₁* = 8.8 Hz, *J₂* = 2.3 Hz, 1 H), 7.11 (d, *J* = 8.8 Hz, 1 H). ESI-LCMS *m*/*z* calcd for C₈H₅BrN₂O₂: 240.0 ; found 241.0 (M + 1)⁺.

### Step 3: 6-Furan-2-yl-1H-quinazoline-2,4-dione

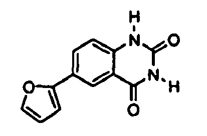

A mixture of 6-bromo-1*H*-quinazoline-2,4-dione (8.0 g, 33 mmol), 2-furanboronic acid (4.5 g, 40 mmol), potassium phosphate (K₃PO₄) (17.5 g, 82 mmol) and tetrakistriphenyphosphine palladium (Pd(PPh₃)₄) (2.0 g, 1.7 mmol) in DMF (80 mL, N₂ degassed) in a Teflon screw cap glass pressure vessel was warmed (100 °C bath) with stirring. After 16 h, the contents were added to H₂O (250 mL), the solids were filtered and washed with H₂O (50 mL). After drying, the solids were stirred in methylene chloride/heptane (4:1, 100 mL), filtered and washed with CH₂Cl₂/heptane ((4:1, 50 mL) to provide 6-furan-2-yl-1*H*-quinazoline-2,4-dione as a tan solid (7 g, 90%): R_{f} 0.27 (5% methanol in dichloromethane) ¹H NMR (DMSO-d₆, 300 MHz) δ 8.10 (d, *J* = 2.0 Hz, 1 H), 7.94 (dd, *J₁*= 8.5 Hz, *J₂* = 2.0 Hz, 1 H), 7.73 (d, *J* = 1.6 Hz, 1 H), 7.18 (d, *J* = 8.5 Hz, 1 H), 6.95 (d, *J* = 3.1 Hz, 1 H), 6.58 (dd, *J* = 3.1, 1.6 Hz, 1 H). ESI-LCMS *m*/*z* calcd for C₁₂H₈N₂O₃: 228.0 ; found 229.0(M + 1)⁺.

### Step 4: 2,4-Dichloro-6-furan-2-yl-quinazoline

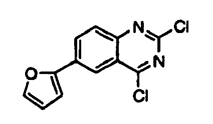

Prepared according to the literature procedure by Fujino, K.; Takami, H.; Atsumi, T.; Ogasa, T.; Mohri, S-I; Kasai, M. Org. Process Res. Dev. 2001, 5, 426 with some modifications. A mixture of 6-furan-2-yl-1*H*-quinazoline-2,4-dione (1.0 g, 4.4 mmol), phosphorous oxychloride (POCl₃) (2 mL, 21 mmol) and diisopropylethylamine (1.7 mL, 9.7 mmol) in toluene (5 mL) was warmed (85 °C bath) with stirring (3 h). The liquids were removed via distillation, the solids dissolved in toluene/ethyl acetate (EtOAc) (3:1, 20 mL) and the solution was added slowly to cooled (ice bath) 2 M K₂HPO₄ (30 mL) and EtOAc (10 mL). The mixture was filtered, the organic layer separated, dried (Na₂SO₄) and the solvent removed. The solid was dissolved in CH₂Cl₂ and pulled through a 1.5" plug of silica eluting with EtOAc/heptane (1:1) to provide 2,4-dichloro-6-furan-2-yl-quinazoline as a yellow solid (0.8 g, 66%). R_{f} 0.63 (n-heptane/EtOAc, 4:1, v/v) ¹H NMR (CDCl₃, 300 MHz) δ 8.44 (d, *J* = 1.9 Hz, 1 H), 8.24 (dd, *J* = 8.9, 1.9 Hz, 1 H), 7.98 (d, *J* = 8.9 Hz, 1 H), 7. 59 (d, *J* = 1.6 Hz, 1 H), 6.91 (d, *J* = 3.1 Hz, 1 H), 6.57 (dd, *J* = 3.1, 1.6 Hz, 1 H). ESI-LCMS *m*/*z* calcd for C₁₂H₆Cl₂N₂O: 264.0 ; found 265.0 (M + 1)⁺.

### Step 5: 2-Chloro-6-furan-2-yl-4-pyridin-2-yl-quinazoline

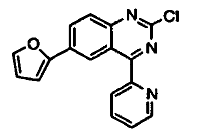

A mixture of 2,4-dichloro-6-furan-2-yl-quinazoline (1.00 g, 3.80 mmol), 0.5M 2-pyridylzinc bromide (9.0 mL in tetrahydrofuran (THF)) and Pd(PPh₃)₄ (0.25 g, 0.21 mmol) in THF (5 mL) was warmed (90 °C bath) under N₂ with stirring. After 16 h, the mixture was added to 50% NH₄Cl (100 mL) and NaCl (sat.) (10 mL), extracted with CH₂Cl₂ (100 mL and 50 mL) and dried (Na₂SO₄). The crude material was purified by flash chromatography eluting with CH₂Cl₂/EtOAc (32:1, v/v) to provide 2-chloro-6-furan-2-yl-4-pyridin-2-yl-quinazoline as a green-brown solid (0.28 g, 23%): R_{f} 0.29 (n-heptane/EtOAc, 17:3, v/v) ¹H NMR (CDCl₃, 300 MHz) δ 9.20 (d, *J* = 2.0 Hz, 1 H), 8.87 (br d, *J* = 4.7 Hz, 1 H), 8.27 (d, *J* = 7.8 Hz, 1 H), 8.21 (dd, *J* = 8.9, 2.0 Hz, 1 H), 8.01 (d, *J* = 8.9 Hz, 1 H), 7.96 (dt, *J* = 7.8, 1.3 Hz, 1 H), 7.54-7.49 (comp, 2 H), 6.82 (d, *J* = 3.4 Hz, 1 H), 6.52 (dd, *J* = 3.4, 1.9 Hz, 1 H). ESI-LCMS m/z calcd for C₁₇H₁₀ClN₃O: 307.05; found 308.2 (M + 1)⁺.

### Step 6: 4-[5-Bromo-1-(6-furan-2-yl-4-pyridin-2-yl-quinazolin-2-yl)-1H-indole-3-yl]-4-oxo-butyric acid methyl ester.

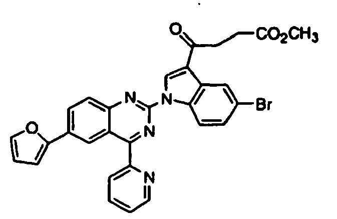

A mixture of 2-chloro-6-furan-2-yl-4-pyridin-2-yl-quinazoline (0.200 g, 0.650 mmol), 4-(5-bromo-1*H*-indol-3-yl)-4-oxo-butyric acid methyl ester (0.222 g, 0.710 mmol), K₂CO₃ (0.180 g, 1.30 mmol) and DMAP (cat.) in DMSO (5 mL) under N₂ was warmed (95 °C) with stirring. After 16 h, added H₂O (10 mL) and filtered the solids. The material was pure enough for the next step or the crude material was purified by flash chromatography eluting with CH₂Cl₂/EtOAc (99:1 and 98.5:1.5, v/v) to provide 4-[5-bromo-1-(6-furan-2-yl-4-pyridin-2-yl-quinazolin-2-yl)-1*H*-indole-3-yl]-4-oxo-butyric acid methyl ester as a yellow-green solid (0.36 g, 95%): R_{f} 0.45 (CH₂Cl₂/EtOAc, 49:1, v/v)
¹H NMR (CDCl₃, 300 MHz) δ 9.17 (s, 1 H), 9.13 (d, *J* = 2.1 Hz, 1 H), 8.95-8.91 (m, 1 H), 8.90 (d, *J* = 8.8 Hz, 1 H), 8.62 (d, *J* = 2.1 Hz, 1 H), 8.29 (d, *J* = 7.7 Hz, 1 H), 8.22 (dd, *J* = 8.6, 2.1 Hz, 1 H), 8.09-8.03 (m, 1 H), 8.08 (d, *J* = 8.6 Hz, 1 H), 7.59 (ddd, *J* = 7.7, 4.7, 1.2 Hz, 1 H), 7.54-7.50 (comp, 2 H), 6.82 (d, *J* = 3.2 Hz, 1 H), 6.53 (dd, *J* = 3.2, 1.8 Hz, 1 H), 3.73 (s, 3 H), 3.37 (t, *J* = 6.7 Hz, 2 H), 2.84 (t, *J* = 6.7 Hz, 2 H). ESI-LCMS m/z calcd for C₃₀H₂₁BrN₄O₄: 580.07; found 581.0 (M + 1)⁺.

### Step 7: 4-[5-Bromo-1-(6-furan-2-yl-4-pyridin-2-yl-quinazolin-2-yl)-1H-indole-3-yl]-4-oxo-butyric acid.

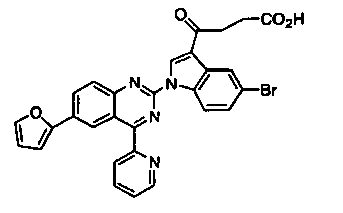

A mixture of 4-[5-bromo-1-(6-furan-2-yl-4-pyridin-2-yl-quinazolin-2-yl)-1*H*-indole-3-yl]-4-oxo-butyric acid methyl ester (0.300 g, 0.510 mmol) and NaOH (0.062 g, 1.5 mmol) in DMSO/dioxane/H₂O (14 mL, 4:4:1, v/v) was stirred under N₂ at rt, 3-16 h or 60 °C, 4 h. The mixture was acidified (2 N HCl), water was added (20 mL) and the solids were filtered, washed (H₂O) and dried. The material may be recrystallized by dissolving in THF (0.20 mL/mg) and adding hot H₂O or heptane (0.22 mL/mg) to provide 4-[5-bromo-1-(6-furan-2-yl-4-pyridin-2-yl-quinazolin-2-yl)-1*H*-indole-3-yl]-4-oxo-butyric acid as a yellow solid (0.15 g, 52%) : R_{f} 0.28 (CH₂Cl₂/MeOH, 19:1, v/v) ¹H NMR (DMSO-d₆, 300 MHz) δ 9.29 (s, 1 H), 9.15 (d, *J* = 2.1 Hz, 1 H), 8.95-891 (m, 1 H), 8.91 (d, *J* = 8.9 Hz, 1 H), 8.50 (d, *J* = 8.4 Hz, 1 H), 8.41-8.37 (comp, 2 H), 8.18 (dt, *J* = 7.6, 1.8 Hz, 1 H), 8.14 (d, *J* = 8.4 Hz, 4 H), 7.84 (d, *J* = 1.7 Hz, 1 H), 7.73 (ddd, *J* = 7. 6, 4.7, 1.2 Hz, 1 H), 7.58 (dd, *J* = 8.9, 2.1 Hz, 1 H), 7.17 (d, *J* = 3.2 Hz, 1 H), 6.66 (dd, *J* = 3.2, 1.7 Hz, 1 H), 3.36-3.27 (comp, 2 H), 2.63 (t, J = 6.3 Hz, 2 H). ESI-LCMS m/z calcd for C₂₉H₁₉BrN₄O₄: 566.1; found 567.3 (M + 1)⁺.

### Reference Example 9 (Reference compound 68)

### Preparation of 4-[5-Chloro-1-(6-furan-2-yl-4-pyridin-2-yl-quinazolin-2-yl)-1H-indole-3-yl]-4-oxo-butyric acid

### Step 1: 4-[5-Chloro-1-(6-furan-2-yl-4-pyridin-2-yl-quinazolin-2-yl)-1H-indol-3-yl]-4-oxo-butyric acid 2-trimethylsilanyl-ethyl ester

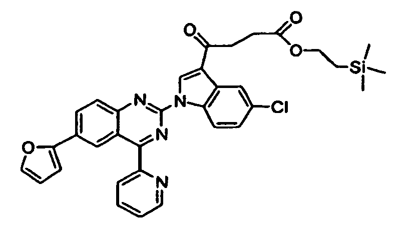

This compound was prepared in a manner analogous to that set forth in example A, except 4-(5-chloro-1*H*-indol-3-yl)-4-oxo-butyric acid 2-trimethylsilanyl-ethyl ester (0.062 mg, 0.176 mmol, 1.1 mole equivalent) was used in step 6 instead of 4-(5-bromo-1H-indol-3-yl)-4-oxo-butyric acid methyl ester to provide 4-[5-chloro-1-(6-furan-2-yl-4-pyridin-2-yl-quinazolin-2-yl)-1H-indol-3-yl]-4-oxo-butyric acid 2-trimethylsilanyl-ethyl ester as a brown solid (0-068g, 68%): R_{f} 0.39 (heptane/EtOAc, 7:3, v/v) ¹H NMR (CDCl₃, 300 MHz) δ 9.19 (s, 1 H), 9.13 (d, *J* = 2.1 Hz, 1 H), 8.96 (d, *J* = 9.0 Hz, 1 H), 8.95-8.91 (m, 1 H), 8.46 (d, *J* = 1.9 Hz, 1 H), 8.30 (d, *J* = 7.4 Hz, 1 H), 8.22 (dd, *J* = 8.8, 1.9 Hz, 1 H), 8.08 (d, *J* = 8.8 Hz, 1 H), 8.06 (dt, *J* = 7.4, 1.8 Hz, 1 H), 7.58 (ddd, *J* = 7.4, 4.8, 1.2 Hz, 1 H), 7.53 (d, *J* = 1.7 Hz, 1 H), 7.38 (dd, *J* = 9.0, 2.1 Hz, 1 H), 6.82 (d, *J* = 3.2 Hz, 1 H), 6.53 (dd, *J* = 3.2, 1.7 Hz, 1 H), 4.24-4.18 (m, 2 H), 3.60 (t, *J* = 6.9 Hz, 2 H), 2.82 (t, *J* = 6.9 Hz, 2 H), 1.05-0.99 (m, 2 H), 0.05 (s, 9 H). ESI-LCMS *m*/*z* calcd for C₃₄H₃₁ClN₄O₄Si : 622.18; found 623.4 (M + 1)⁺.

### Step 2: 4-[5-Chloro-1-(6-furan-2-yl-4-pyridin-2-yl-quinazolin-2-yl)-1H-indole-3-yl]-4-oxo-butyric acid

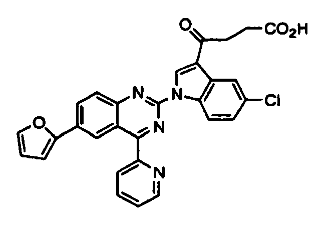

To a solution of 4-[5-chloro-1-(6-furan-2-yl-4-pyridin-2-yl-quinazolin-2-yl)-1*H*-indole-3-yl]-4-oxo-butyric acid 2-trimethylsilanyl-ethyl ester (0.060 g, 0.096 mmol) in THF (1 mL) was added tetra-n-butylammoniumflouride (TBAF) (0.380 mL, 0.38 mmol) at rt with stirring. After 3 h, water (1 mL) and 1 N HCl was added until acidic and the mixture was stirred 1h. The solids were filtered, washed with water and dried to provide 4-[5-chloro-1-(6-furan-2-yl-4-pyridin-2-yl-quinazolin-2-yl)-1*H*-indole-3-yl]-4-oxo-butyric acid as a yellow solid (0.027 g, 50%): R_{f} 0.38 (CH₂Cl₂/MeOH, 19:1, v/v) ¹H NMR (DMSO-d₆, 300 MHz) δ 9.35 (s, 1 H), 9.16 (d, *J* = 2.2 Hz, 1 H), 9.00 (d, *J* = 8.9 Hz, 1 H), 8.94 (br d with further small coupling, *J* = 4.8 Hz, 1 H), 8.52 (d, *J* = 8.4 Hz, 1 H), 8.42 (dd, *J* = 8.1, 1.9 Hz, 1 H), 8.27 (d, *J* = 2.1 Hz, 1 H), 8.21 (dt, *J* = 8.1, 1.9 Hz, 1 H), 8.18 (d, *J* = 8.4 Hz, 1 H), 7.85 (d, *J* = 1.6 Hz, 1 H), 7.74 (ddd, *J* = 8.1, 4.8, 1.9 Hz, 1 H), 7.49 (dd, *J* = 8.9, 2.2 Hz, 1 H), 7.19 (d, *J* = 3.6 Hz, 1 H), 6.67 (dd, *J* = 3.6, 1.6 Hz, 1 H), 3.36-3.27 (comp, 2 H), 2.64 (t, *J* = 6.4 Hz, 2 H). ESI-LCMS *m*/*z* calcd for C₂₉H₁₉ClN₄O₄: 522.1; found 523.0 (M + 1)⁺.

### Example 10 (compound no. 49)

Preparation of 4-{1-[4-(4-Butyl-phenyl)-6-furan-2-yl-quinazolin-2-yl]-1*H*-indol-3-yl-4-oxo-butyric acid

This compound was prepared in a manner analogous to that set forth in example A, except 4-n-butylphenylmagnesium bromide and ZnCl₂ (1 mole equivalent) was used instead of 2-pyridylzinc bromide in step 5 and 4-(1*H*-indol-3-yl)-4-oxo-butyric acid methyl ester was used instead of 4-(5-bromo-1*H*-indol-3-yl)-4-oxo-butyric acid methyl ester in step 6 to provide 4-{1-[4-(4-Butyl-phenyl)-6-furan-2-yl-quinazolin-2-yl]-1*H*-indol-3-yl-4-oxo-butyric acid as a yellow solid: R_{f} .20 (Heptane/EtOAC, 3:2, v/v). ¹H NMR (DMSO-d₆, 300 MHz) δ 12.07 (br s, 1H), 9.25 (s, 1H), 9.01 (d, *J* = 8 Hz, 1H), 8.42 (dd, *J* = 9, 2 Hz, 1H), 8.30-8.28 (m, 2H), 8.20 (d, *J* = 9 Hz, 1H), 7.92 (d, *J* = 8 Hz, 2H), 7.83 (d, *J* = 2 Hz, 1H), 7.55 (d, *J* = 8 Hz, 2H), 7.48-7.34 (m, 2H), 7.19 (d, *J* = 3 Hz, 1H), 6.65 (dd, *J* = 3, 2 Hz, 1H), 3.30 (t, *J* = 6 Hz, 2H), 2.77 (t, *J* = 7.5 Hz, 2H), 2.62 (t, *J* = 6 Hz, 2H), 1.75-1.65 (m, 2H), 1.48-1.35 (m, 2H), .967 (t, *J* = 7.5 Hz, 3H). ESI-LCMS *m*/*z* calcd for C₃₄H₂₉N₃O₄ : 543.6 ; found 544.0 (M + 1)⁺.

### Example 11 (compound no. 47)

### Preparation of 4-[1-(6-Furan-2-yl-4-phenyl-quinazolin-2-yl)-1H-indol-3-yl]-4-oxo-butyric acid

This compound was prepared in a manner analogous to that set forth in example A, except phenylmagnesium bromide and ZnCl₂ (1 mole equivalent) was used instead of 2-pyridylzinc bromide in step 5 and 4-(1*H*-indol-3-yl)-4-oxo-butyric acid methyl ester was used instead of 4-(5-bromo-1*H*-indol-3-yl)-4-oxo-butyric acid methyl ester in step 6 to provide 4-[1-(6-furan-2-yl-4-phenyl-quinazolin-2-yl)-1*H*-indol-3-yl]-4-oxo-butyric acid as a yellow solid: R_{f} .30 (CH₂Cl₂/MeOH, 19:1, v/v). ¹H NMR (DMSO-d₆, 300 MHz) δ 12.10 (br s, 1H), 9.26 (s, 1H), 9.01 (d, J = 8 Hz, 1H), 8.44 (dd, *J* = 9, 2 Hz, 1H), 8.30 (d, *J* = 8 Hz, 1H), 8.26 (d, *J* = 2 Hz, 1H), 8.22 (d, *J* = 9 Hz, 1H), 8.02-7.98 (m, 2H), 7.82 (d, *J* = 2 Hz, 1H), 7.75-7.72 (m, 3H), 7.48-7.34 (cm, 2H), 7.20 (d, *J* = 3.5 Hz, 1H), 6.65 (dd, *J* = 3.5, 2 Hz, 1H), 3.31 (t, *J* = 6 Hz, 2H), 2.62 (t, *J* = 6 Hz, 2H). ESI-LCMS *m*/*z* calcd for C₃₀H₂₁N₃O₄ : 487.5 ; found 488.0 (M + 1)⁺.

### Example 12 (compound no. 48)

### Preparation of 4-[5-Bromo-1-(6-furan-2-yl-4-phenyl-quinazolin-2-yl)-1H-indol-3-yl]-4-oxo-butyric acid

This compound was prepared in a manner analogous to that set forth in example A, except phenylmagnesium bromide and ZnCl₂ (1 mole equivalent) was used instead of 2-pyridylzinc bromide in step 5 to provide 4-[5-Bromo-1-(6-furan-2-yl-4-phenyl-quinazolin-2-yl)-1*H*-indol-3-y1]-4-oxo-butyric acid as a yellow solid: R_{f} .31 (CH₂Cl₂/MeOH, 19:1, v/v). ¹H NMR (DMSO-d₆, 300 MHz) δ 12.12 (br s, 1H), 9.23 (s, 1H), 8.89 (d, *J* = 9 Hz, 1H), 8.42-8.37 (m, 2H), 8.21 (d, *J* = 2 Hz, 1H), 8.16 (d, *J* = 9 Hz, 1H), 7.99-7.96 (m, 2H), 7.80 (d, *J* = 2 Hz, 1H), 7.73-7.71 (m, 3H), 7.56 (dd, *J* = 9, 2 Hz, 1H), 7.17 (d, *J* = 3.5 Hz, 1H). 6.64 (dd, J = 3.5, 2 Hz, 1H), 3.28 (t, *J* = 6 Hz, 2H), 2.61 (t, *J* = 6 Hz, 2H). ESI-LCMS *m*/*z* calcd for C₃₀H₂₀BrN₃O₄ : 566.4 ; found 568.0 (M + 1)⁺.

### Example 13 (compound no. 43)

### Preparation of 4-{5-Bromo-1-[6-furan-2-yl-4-(4-methoxyphenyl)-quinazolin-2-yl]-1H-indole-3-yl}-4-oxo-butyric acid.

This compound was prepared in a manner analogous to that set forth in example A, except 4-methoxyphenylmagnesium bromide and ZnCl₂ (1 mole equivalent) was used instead of 2-pyridylzinc bromide in step 5 to provide 4-{5-bromo-1-[6-furan-2-yl-4-(4-methoxy-phenyl)-quinazolin-2-yl]-1*H*-indole-3-yl}-4-oxo-butyric acid as a yellow solid: R_{f} 0.45 (CH₂Cl₂/MeOH, 19:1, v/v) ¹H NMR (DMSO-d₆, 300 MHz) δ 12.2 (s, 1 H), 9.25 (s, 1 H), 8.91 (d, *J* = 8.9 Hz, 1 H), 8.41-8.37 (comp, 2 H), 8.30 (s, 1 H), 8.14 (d, *J* = 8.8 Hz, 1 H), 7.99 (d, *J* = 8.8 Hz, 2 H), 7.82 (d, *J* = 1.7 Hz, 1 H), 7.57 (dd, *J* = 8.9, 1.9 Hz, 1 H), 7.27 (d, *J* = 8.8 Hz, 2 H), 7.18 (d, *J* = 3.5 Hz, 1 H), 6.65 (dd, *J* = 3.5, 1.7 Hz, 1 H), 3.93 (s, 3 H), 3.29 (t, *J* = 6.3 Hz, 2 H), 2.62 (t, *J* = 6.3 Hz, 2 H). ESI-LCMS *m*/*z* calcd for C₃₁H₂₂BrN₃O₅ : 595.1; found 596.0 (M + 1)⁺.

### Example 14 (compound no. 44)

### Preparation of 4-{5-Bromo-1-[4-(4-cyano-phenyl)-6-furan-2-yl-quinazolin-2-yl]-1H-indole-3-yl}-4-oxo-butyric acid.

This compound was prepared in a manner analogous to that set forth in example A, except 4-cyanophenylzinc bromide was used instead of 2-pyridylzinc bromide in step 5 to provide 4-{5-Bromo-1-[4-(4-cyano-phenyl)-6-furan-2-yl-quinazolin-2-yl]-1*H-*indole-3-yl}-4-oxo-butyric acid as a yellow solid: R_{f} 0.30 (CH₂Cl₂/MeOH, 19:1, v/v) ¹H NMR (CDCl₃, 300 MHz) δ 9.09 (s, 1 H), 8.82 (d, *J* = 9.1 Hz, 1 H), 8.57 (d, *J* = 2.0 Hz, 1 H), 8.24-8.19 (comp, 2 H), 8.10 (d, *J* = 8.8 Hz, 1 H), 7.99 (d, *J* = 2.3 Hz, 4 H), 7.53 (d, *J* = 1.8 Hz, 1 H), 7.48 (dd, *J* = 9.1, 2.0 Hz, 1 H), 6.81 (d, *J* = 3.2 Hz, 2 H), 6.54 (dd, *J* = 3.2, 1.8 Hz, 1 H), 3.41 (t, *J* = 6.6 Hz, 2 H), 2.87 (t, *J* = 6.6 Hz, 2 H). ESI-LCMS *m*/*z* calcd for C₃₁H₁₉BrN₄O₄: 590.1; found 591.0 (M + 1)⁺.

### Example 15 (compound no. 45)

### Preparation of 4-{5-Bromo-1-[4-(4-fluoro-phenyl)-6-furan-2-yl-quinazolin-2-yl]-1H-indole-3-yl}-4-oxo-butyric acid

This compound was prepared in a manner analogous to that set forth in example A, except 4-fluorophenylzinc bromide was used instead of 2-pyridylzinc bromide in step 5 to provide 4-{5-bromo-1-[4-(4-fluoro-phenyl)-6-furan-2-yl-quinazolin-2-yl]-1*H-*indole-3-yl}-4-oxo-butyric acid as a yellow solid: R_{f} 0.32 (CH₂Cl₂/MeOH, 19:1, v/v). ESI-LCMS *m*/*z* calcd for C₃₀H₁₉BrFN₃O₄ : 583.0; found 584.0 (M + 1)⁺.

### Reference Example 16 (Reference compound no. 64)

### Preparation of 4-{5-Bromo-1-[6-furan-2-yl-4-(4-methoxy-phenylamino)-quinazolin-2-yl]-1H-indole-3-yl}-4-oxo-butyric acid

### Step 1: 2-Amino-5-bromo-benzonitrile

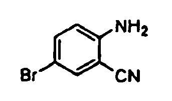

Prepared according to the literature procedure by Roche, D. Prasad, K.; Repic, O.; Blacklock, T. J. Tetrahedron Lett. 2000, 41, 2083.
R_{f} 0.46 (n-heptane/EtOAc, 3:2, v/v) ¹H NMR (CDCl₃, 300 MHz) δ 7.47 (d, *J* = 2.3 Hz, 1 H), 7.39 (dd, *J* = 8.9, 2.2 Hz, 1 H), 6.62 (d, *J* = 8.9 Hz, 1 H). ESI-LCMS *m*/*z* calcd for C₇H₅BrN₂: 196.0 ; found 197.0 (M + 1)⁺.

### Step 2: 6-Bromo-1H-quinazoline-2,4-dione

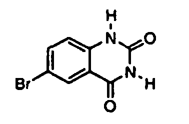

Prepared according to the literature procedure by Mizuno, T.; Okamoto, N, Ito, T.; Miyata, T. Tetrahedron Lett. 2000, 41, 1051 with some modifications.
A mixture of 2-amino-5-bromo-benzonitrile (10 g, 52 mmol) and 1,8-diazobicyclo[5.4.0]-undec-7-ene (DBU) (30 mL, 200 mmol) in dimethylformamide (DMF) (50 mL) was warmed (100 °C) with stirring under an atmosphere of carbon dioxide from an attached latex balloon for 48 hours. The solution was removed from the heat, added to cooled (ice bath) 1N HCl (500 mL) and the solids were collected, washed with H₂O and dried to provide 6-bromo-1*H*-quinazoline-2,4-dione as a yellow solid (12 g, quantitative): R_{f} 0.27 (CH₂Cl₂/MeOH, 19:1, v/v) ¹H NMR (DMSO-d₆, 300 MHz) δ 11.41 (s, 1 H), 11.27 (s, 1 H), 7.91 (d, *J* = 2.3 Hz, 1 H), 7.77 (dd, *J* = 8.8, 2.3 Hz, 1 H), 7.11 (d, *J* = 8.8 Hz, 1 H). ESI-LCMS *m*/*z* calcd for C₈H₅BrN₂O₂: 240.0 ; found 241.0 (M + 1)⁺.

### Step 3: 6-Furan-2-yl-1H-quinazoline-2,4-dione

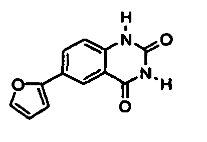

A mixture of 6-bromo-1*H*-quinazoline-2,4-dione (8.0 g, 33 mmol), 2-furanboronic acid (4.5 g, 40 mmol), potassium phosphate (K₃PO₄) (17.5 g, 82 mmol) and tetrakistriphenyphosphine palladium (Pd(PPh₃)₄) (2.0 g, 1.7 mmol) in DMF (80 mL, N₂ degassed) in a Teflon screw cap glass pressure vessel was warmed (100 °C bath) with stirring. After 16 h, the contents were added to H₂O (250 mL), the solids were filtered and washed with H₂O (50 mL). After drying, the solids were stirred in methylene chloride/heptane (4:1, 100 mL), filtered and washed with CH₂Cl₂/heptane ((4:1, 50 mL) to provide 6-furan-2-yl-1*H*-quinazoline-2,4-dione as a tan solid (7 g, 90%): R_{f} 0.27 (CH₂Cl₂/MeOH, 19:1, v/v) ¹H NMR (DMSO-d₆, 300 MHz) δ 8.10 (d, *J* = 2.0 Hz, 1 H), 7.94 (dd, *J* = 8.5, 2.0 Hz, 1 H), 7.73 (d, *J* = 1.6 Hz, 1 H), 7.18 (d, *J* = 8.5 Hz, 1 H), 6.95 (d, *J* = 3.1 Hz, 1 H), 6.58 (dd, *J* = 3.1, 1.6 Hz, 1 H). ESI-LCMS *m*/*z* calcd for C₁₂H₈N₂O₃: 228.0 ; found 229.0(M + 1)⁺.

### Step 4: 2,4-Dichloro-6-furan-2-yl-quinazoline

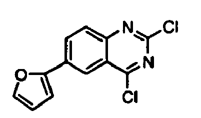

Prepared according to the literature procedure by Fujino, K.; Takami, H.; Atsumi, T.; Ogasa, T.; Mohri, S-I; Kasai, M. Org. Process Res. Dev. 2001, 5, 426 with some modifications. A mixture of 6-furan-2-yl-1*H*-quinazoline-2,4-dione (1.0 g, 4.4 mmol), phosphorous oxychloride (POCl₃) (2 mL, 21 mmol) and diisopropylethylamine (1.7 mL, 9.7 mmol) in toluene (5 mL) was warmed (85 °C bath) with stirring (3 h). The liquids were removed via distillation, the solids dissolved in toluene/ethyl acetate (EtOAc) (3:1, 20 mL) and the solution was added slowly to cooled (ice bath) 2M K₂HPO₄ (30 mL) and EtOAc (10 mL). The mixture was filtered, the organic layer separated, dried (Na₂SO₄) and the solvent removed. The solid was dissolved in CH₂Cl₂ and pulled through a 1.5" plug of silica eluting with EtOAc/heptane (1:1) to provide 2,4-dichloro-6-furan-2-yl-quinazoline as a yellow solid (0.8g, 66%). R_{f} 0.63 (n-heptane/EtOAc, 4:1, v/v) ¹H NMR (CDCl₃, 300 MHz) δ 8.44 (d, *J* = 1.9 Hz, 1 H), 8.24 (dd, *J* = 8.9, 1.9 Hz, 1 H), 7.98 (d, *J* = 8.9 Hz, 1 H), 7.59 (d, *J* = 1.6 Hz, 1 H), 6.91 (d, *J* = 3.1 Hz, 1 H), 6.57 (dd, *J* = 3.1, 1.6 Hz, 1 H). ESI-LCMS *m*/*z* calcd for C₁₂H₆Cl₂N₂O: 264.0 ; found 265.0 (M + 1)⁺.

### Step 5: 2-Chloro-6-furan-2-yl-quinazolin-4-yl-(4-methoxyphenyl)-amine

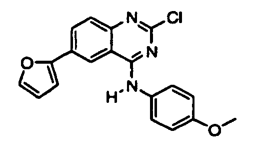

To a solution of 2,4-dichloro-6-furan-2-yl-quinazoline (0.100 g, 0.38 mmol) and p-anisidine (0.043g, 0.035 mmol) in THF (1 mL) was added with stirring under an atmosphere of N₂ at rt triethyl amine (52 uL, 0.38 mmol). After 24 h, EtOAc (5 mL) was added and the mixture was washed with NH₄Cl (50%), sat. NaHCO₃ and the organic dried (Na₂SO₄). The crude material was purified by flash chromatography eluting with heptane/EtOAc (7:3 and 3:2, v/v) to provide 2-chloro-6-furan-2-yl-quinazolin-4-yl-(4-methoxy-phenyl)-amine as a yellow solid (0.12 g, 98%). R_{f} 0.07 (n-heptane/EtOAc, 3:17, v/v) ¹H NMR (CDCl₃, 300 MHz) δ 8.06 (d, *J* = 1.8 Hz, 1 H), 8.01 (dd, *J* = 8.8, 1.8 Hz, 1 H), 7.80 (d, *J* = 8.8 Hz, 1 H), 7.64 (d, *J* = 9.1 Hz, 2 H), 7.57 (brs, 1 H), 7.52 (dd, *J* = 1.8, 0.7 Hz, 1 H), 6.96 (d, *J* = 9.1 Hz, 2 H), 6.79 (dd, *J* = 3.5, 0.7 Hz, 1 H), 6.54 (dd, *J* = 3.5, 1.8 Hz, 1 H), 3.84 (s, 3 H). ESI-LCMS *m*/*z* calcd for C₁₉H₁₄ClN₃O₂ : 351.1 ; found 352.0 (M + 1)⁺.

### Step 6: 4-(5-Bromo-1-[6-furan-2-yl-4-(4-methoxy-phenylamino)-quinazolin-2-yl]-1H-indole-3-yl)-4-oxo-butyric acid methyl ester

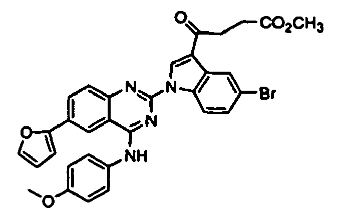

A mixture of 2-chloro-6-furan-2-yl-quinazolin-4-yl-(4-methoxyphenyl)-amine (0.300 g, 0.850 mmol), 4-(5-bromo-1*H*-indol-3-yl)-4-oxo-butyric acid methyl ester (0.290 g, 0.940 mmol), K₂CO₃ (0.300 g, 2.20 mmol) and DMAP (cat.) in DMSO (2 mL) under N₂ was warmed (95 °C) with stirring. After 16 h, added H₂O (10 mL) and filtered the solids. The material was pure enough for the next step or the crude material was extracted into EtOAc and purified by flash chromatography eluting with heptane/EtOAc (19:1-3:2, v/v) or crude material was dissolved in a minimum of THF, MeOH or CH₂Cl₂ and precipitated with heptane to provide 4-{5-bromo-1-[6-furan-2-yl-4-(4-methoxy-phenylamino)-quinazolin-2-yl]-1*H-*indole-3-yl}-4-oxo-butyric acid methyl ester as a tan solid (0.200 g, 37%): R_{f} 0.31 (n-heptane/EtOAc, 3:2, v/v) ¹H NMR (DMSO-*d*₆, 300 MHz) δ 10.39 (s, 1 H), 9.01 (s, 1 H), 8.83 (d, *J* = 2.9 Hz, 1 H), 8.62 (dd, *J* = 9.0, 2.9 Hz, 1 H), 8.33 (d, *J* = 2.0 Hz, 1 H), 8.19 (dd, *J* = 8.6, 2.0 Hz, 1 H), 7.87-7.83 (comp, 2 H), 7.69-7.64 (comp, 2 H), 7.33 (dt, *J* = 9.0, 2.9 Hz, 1 H), 7.12-7.08 (comp, 3 H), 6.69 (dd, *J* = 3.4, 1.8 Hz, 1 H), 3.84 (s, 3 H), 3.61 (s, 3 H), 3.26 (t, *J* = 6.5 Hz, 2 H), 2.69 (t, *J* = 6.5 Hz, 2 H).
ESI-LCMS *m*/*z* calcd for C₃₂H₂₅BrN₄O₅: 624.10; found 625.0 (M + 1)⁺.

### Step 7: 4-{5-Bromo-1-[6-furan-2-yl-4-(4-methoxy-phenylamino)-quinazolin-2-yl]-1H-indole-3-yl}-4-oxo-butyric acid

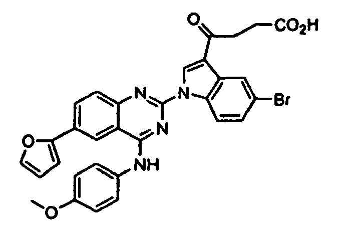

A mixture of 4-{5-bromo-1-[6-furan-2-yl-4-(4-methoxy-phenylamino)-quinazolin-2-yl]-1*H*-indole-3-yl}-4-oxo-butyric acid methyl ester (0.100 g, 0.160 mmol) and NaOH (0.020 g, 0.50 mmol) in DMSO/dioxane/H₂O (5 mL, 4:4:1, v/v) was stirred under N₂ at rt, 3-16 h or 60 °C, 4 h. The mixture was acidified (2 N HCl), water was added (20 mL) and the solids were filtered, washed (H₂O) and dried. The material may be recrystallized by dissolving in THF (0.20 mL/mg) and adding hot H₂O or heptane (0.22 mL/mg) to provide 4-{5-bromo-1-[6-furan-2-yl-4-(4-methoxy-phenylamino)-quinazolin-2-yl]-1*H*-indole-3-yl}-4-oxo-butyric acid as a yellow solid (0.100g, quant.): R_{f} 0.26 (CH₂Cl₂/MeOH, 19:1, v/v) ¹H NMR (DMSO-*d*₆, 300 MHz) δ 10.4 (s, 1 H), 9.01 (s, 1 H), 8.85 (s, 1 H), 8.62 (d, *J* = 8.9 Hz, 1 H), 8.34 (d, *J* = 2.1 Hz, 1 H), 8.19 (d, *J* = 9.1 Hz, 1 H), 7.87-7.84 (comp, 2 H), 7.67 (d, *J* = 8.8 Hz, 2 H), 7.33 (d, *J* = 8.9 Hz, 1 H), 7.12-7.08 (comp, 3 H), 6.69 (dd, *J* = 3.4, 1.9 Hz, 1 H), 3.83 (s, 3 H), 3.20 (t, *J* = 6.4 Hz, 2 H), 2.62 (t, *J* = 6.4 Hz, 2 H). ESI-LCMS *m*/*z* calcd for C₃₁H₂₃BrN₄O₅: 610.09; found 611.0 (M + 1)⁺.

### Reference Example 17 (Reference compound no. 65)

### Preparation of 4-{5-Bromo-1-[6-furan-2-yl-4-(4-methoxy-benzylamino)-quinazolin-2-yl]-1H-indole-3-yl}-4-oxo-butyric acid

This compound was prepared in a manner analogous to that set forth in example C, except used 4-methoxybenzyl amine instead of p-anisidine in step 5 to provide 4-{5-bromo-1-[6-furan-2-yl-4-(4-methoxy-benzylamino)-quinazolin-2-yl]-1*H*-indole-3-yl}-4-oxo-butyric acid as a yellow solid: R_{f} 0.26 (CH₂Cl₂/MeOH, 19:1, v/v) ¹H NMR (DMSO-*d*₆, 300 MHz) δ 9.60 (br, 1 H), 9.07 (s, 1 H), 8.79 (d, *J* = 9.1 Hz, 1 H), 8.70 (s, 1 H), 8.36 (d, *J* = 2.1 Hz, 1 H), 8.14 (dd, *J* = 8.7, 2.0 Hz, 1 H), 7.84 (s, 1 H), 7.81 (d, *J* = 8.7 Hz, 1 H), 7.49-7.42 (comp, 3 H), 7.07 (d, *J* = 3.2 Hz, 1 H), 6.92 (d, *J* = 8.6 Hz, 2 H), 6.67 (dd, *J* = 3.2, 1.7 Hz, 1 H), 4.82 (br, 2 H), 3.69 (s, 3 H), 3.28 (t, *J* = 6.4 Hz, 2 H), 2.64 (t, *J* = 6.4 Hz, 2 H). ESI-LCMS *m*/*z* calcd for C₃₂H₂₅BrN₄O₅: 624.1; found 625.0 (M + 1)⁺.

### Reference Example 18 (Reference compound no. 110)

### Preparation of 4-[5-Bromo-1-(6-furan-2-yl-4-hydroxy-quinazolin-2-yl]-1H-indole-3-yl]-4-oxo-butyric acid

This compound was prepared in a manner analogous to that set forth in example C, except used ethanol instead of p-anisidine in step 5 to provide 4-[5-bromo-1-(6-furan-2-yl-4-hydroxy-quinazolin-2-yl]-1*H*-indole-3-yl]-4-oxo-butyric acid as a yellow solid: R_{f} 0.30 (CH₂Cl₂/MeOH, 9:1, v/v) ¹H NMR (DMSO-*d*₆, 300 MHz) δ 12.19 (br, 1 H), 9.16 (s, 1 H), 8.65 (d, *J* = 8.7 Hz, 1 H), 8.37-8.36 (comp, 2 H), 8.18 (dd, *J* = 8.5, 2.4 Hz, 1 H), 7.82-7.79 (comp, 2 H), 7.58 (dd, *J* = 8.7, 2.1 Hz, 1 H), 7.16 (d, *J* = 3.4 Hz, 1 H), 6.65 (dd, *J* = 3.4, 1.9 Hz, 1 H), 3.22 (t, *J* = 6.4 Hz, 2 H), 2.64 (t, *J* = 6.4 Hz, 2 H). ESI-LCMS *m*/*z* calcd for C₂₄H₁₆BrN₃O₅: 505.0; found 506.0 (M + 1)⁺.

### Reference Example 19 (Reference compound no. 111)

### Preparation of 4-(5-Bromo-1-{6-furan-2-yl-4-[(furan-2-ylmethyl)-amino]-quinazolin-2-yl}-1H-indole-3-yl)-4-oxo-butyric acid

This compound was prepared in a manner analogous to that set forth in example C, except used furfurylamine instead of p-anisidine in step 5 to provide 4-(5-bromo-1-{6-furan-2-yl-4-[(furan-2-ylmethyl)-amino]-quinazolin-2-yl}-1*H*-indole-3-yl)-4-oxo-butyric acid as a brown amorphous solid: R_{f} 0.64 (CH₂Cl₂/MeOH, 12:1, v/v) ¹H NMR (DMSO-*d*₆, 300 MHz) δ 9.45 (br, 1 H), 9.19 (s, 1 H), 8.89 (d, *J* = 9.1 Hz, 1 H), 8.69 (br s, 1 H), 8.41(d, *J* = 1.9 Hz, 1 H), 8.17 (dd, *J* = 8.8, 1.9 Hz, 1 H), 7.86-7.83 (comp, 2 H), 7.66 (s, 1 H), 7.54 (dd, *J* = 9.1, 2.1 Hz, 1 H), 7.08 (d, *J* = 3.2 Hz, 1 H), 6.69 (dd, *J* = 3.2, 1.8 Hz, 1 H), 6.51-6.40 (comp, 2 H), 4.94 (d, *J* = 5.3 Hz, 1 H), 3.32 (t, *J* = 6.2 Hz, 2 H), 2.66 (t, *J* = 6.2 Hz, 2 H). ESI-LCMS *m*/*z* calcd for C₂₉H₂₁BrN₄O₅: 584.07; found 585.0 (M + 1)⁺.

### Reference Example 20 (Reference compound no. 86)

### Preparation of 2-{2-[5-Bromo-3-(3-carboxy-propionyl)-indol-1-yl]-6-furan-2-yl-quinazolin-4-ylamino}-butyric acid

This compound was prepared in a manner analogous to that set forth in example C, except used 2-amino-*n*-butyric acid methyl ester instead of p-anisidine in step 5 to provide 2-(2-[5-bromo-3-(3-carboxy-propionyl)-indol-1-yl]-6-furan-2-yl-quinazolin-4-ylamino)-butyric acid as a brown solid: ; R_{f} 0.38 (CH₂Cl₂/MeOH, 17:3, v/v) ¹H NMR (DMSO-*d*₆, 300 MHz) δ 9.15 (s, 1 H), 9.02 (d, *J* = 6.6 Hz, 1 H), 8.96 (d, *J* = 9.0 Hz, 1 H), 8.80 (s, 1 H), 8.39 (d, *J* = 1.9 Hz, 1 H), 8.18 (dd, *J* = 8.8, 1.9 Hz, 1 H), 7.86 (d, *J* = 8.8 Hz, 1H), 7.86 (d, *J* = 1.7 Hz, 1 H), 7.54 (dd, *J* = 9.0, 2.2 Hz, 1 H), 7.10 (d, *J* = 3.3 Hz, 1 H), 6.68 (dd, *J* = 3.3, 1.7 Hz, 1 H), 4.53 (q, *J* = 6.6 Hz, 1 H), 3.26 (t, *J* = 6.1 Hz, 2 H), 2.63 (t, *J* = 6.1 Hz, 2 H), 2.12-2.02 (m, 2 H), 1.13 (t, *J* = 7.3 Hz, 3 H).
ESI-LCMS *m*/*z* calcd for C₂₈H₂₃BrN₄O₆: 590.08; found 591.0 (M + 1)⁺.

### Reference Example 21 (Reference compound no. 87)

### Preparation of 2-{2-[5-Chloro-3-(3-carboxy-propionyl)-indol-1-yl]-6-furan-2-yl-quinazolin-4-ylamino}-butyric acid

This compound was prepared in a manner analogous to that set forth in example C, except 2-amino-*n*-butyric acid methyl ester was used instead of p-anisidine in step 5 and 4-(5-chloro-1*H*-indol-3-yl)-4-oxo-butyric acid allyl ester was used instead of 4-(5-bromo-1H-indol-3-yl)-4-oxo-butyric acid methyl ester in step 6 to provide 2-(2-[5-chloro-3-(3-carboxypropionyl)-indol-1-yl]-6-furan-2-yl-quinazolin-4-ylamino)-butyric acid as a tan solid: R_{f} 0.38 (CH₂Cl₂/MeOH, 17:3, v/v) ¹H NMR (DMSO-*d*₆*,* 300 MHz) δ 9.18 (s, 1 H), 9.02 (d, *J* = 8.9 Hz, 1 H), 9.04-9.02 (m, 1 H), 8.81 (s, 1 H), 8.24 (d, *J* = 2.0 Hz, 1 H), 8.18 (dd, *J* = 8.6, 2.0 Hz, 1 H), 7.88-7.85 (comp, 2 H), 7.42 (dd, *J* = 8.9, 2.2 Hz, 1 H), 7.10 (d, *J* = 2.2 Hz, 1 H), 6.69 (dd, *J* = 3.4, 2.2 Hz, 1 H), 4.53 (q, *J* = 7.2 Hz, 1 H), 3.26 (t, *J* = 6.3 Hz, 2 H), 2.63 (t, *J* = 6.3 Hz, 2 H), 2.07 (p, *J* = 7.2 Hz, 2 H), 1.13 (t, *J* = 7.2 Hz, 3 H). ESI-LCMS *m*/*z* calcd for C₂₈H₂₃ClN₄O₆ : 546.13 ; found 547.0 (M + 1)⁺.

### REFERENCE EXAMPLE 22 (REFERENCE COMPOUND NO. 74)

### Preparation of 1-{2-[5-Bromo-3-(3-carboxy-propionyl)-indol-1-yl]-6-furan-2-yl-quinazolin-4-yl}-piperidine-4-carboxylic acid

This compound Was prepared in a manner analogous to that set forth in example C, except piperidine-4-carboxylic acid ethyl ester was used instead of p-anisidine in step 5 to provide 1-{2-[5-bromo-3-(3-carboxy-propionyl)-indol-1-yl]-6-furan-2-yl-quinazolin-4-yl}-piperidine-4-carboxylic acid as a tan solid R_{f} 0.25 (CH₂Cl₂/MeOH, 19:1, v/v). ¹H NMR (DMSO-*d*₆, 300 MHz) δ 12.26 (br s, 2H), 9.12 (s, 1H), 8.85 (d *J* = 9 Hz, 1H), 8.37 (d, *J* = 2 Hz, 1H), 8.16 (dd, *J* = 9, 2 Hz, 1H), 8.13 (d, *J* = 2 Hz, 1H), 7.88 (d, *J* = 9 Hz, 1H), 7.84 (d, *J* = 2 Hz, 1H), 7.54 (dd, *J* = 9, 2 Hz, 1H), 7.12 (d, *J* = 3 Hz, 1H), 6.65 (dd, *J* = 3, 2 Hz, 1H), 4.43-4.38 (m, 2H), 3.53-3.45 (m, 2H), 3.28 (t, *J* = 6 Hz, 2H), 2.73-2.69 (m, 1H), 2.62 (t, *J* = 6 Hz, 2H), 2.13-2.09 (m, 2H), 1.94-1.83 (m, 2H). ESI-LCMS *m*/*z* calcd for C₃₀H₂₅BrN₄O₆ : 617.5 ; found 619.0 (M + 1)⁺.

### Reference Example 23 (Reference compound no. 76)

### Preparation of 1-{2-[3-(3-Carboxy-propionyl)-5-chloro-indol-1-yl]-6-furan-2-yl-quinazolin-4-yl}-piperidine-4-carboxylic acid

This compound was prepared in a manner analogous to that set forth in example C, except piperidine-4-carboxylic acid ethyl ester was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 to provide 1-{2-[3-(3-Carboxy-propionyl)-5-chloro-indol-1-yl]-6-furan-2-yl-quinazolin-4-yl}-piperidine-4-carboxylic acid as a white solid: ; R_{f} 0.33 (CH₂Cl₂/MeOH, 33:1, v/v). ¹H NMR (DMSO-*d*₆, 300 MHz) δ 12.25 (br s, 2H), 9.17 (s, 1H), 8.94 (d, *J* = 9 Hz, 1H), 8.24 (d, *J* = 2 Hz, 1H), 8.18 (dd, *J* = 9, 2 Hz, 1H), 8.15 (d, *J* = 2 Hz, 1H), 7.91 (d, *J* = 9 Hz, 1H), 7.84 (d, *J* = 2 Hz, 1H), 7.95 (dd, *J* = 9, 2 Hz, 1H), 7.14 (d, *J* = 3 Hz, 1H), 6.66 (dd, *J* = 3, 2 Hz, 1H), 4.44-4.39 (m, 2H), 3.54-3.47 (m, 2H), 3.29 (t, *J* = 6 Hz, 2H), 2.74-2.70 (m, 1H), 2.62 (t, *J* = 6 Hz, 2H), 2.13-2.09 (m, 2H), 1.95-1.87 (m, 2H). ESI-LCMS *m*/*z* calcd for C₃₀H₂₅ClN₄O₆: 573.0 ; found 573.3 (M + 1)⁺.

### REFERENCE EXAMPLE 29 (REFERENCE COMPOUND NO. 75)

### Preparation of 1-{2-[3-(3-Carboxy-propionyl)-5-chloro-indol-1-yl]-6-furan-2-yl-quinazolin-4-yl}-piperidine-4-carboxylic acid ethyl ester

This compound Was prepared in a manner analogous to that set forth in example C, except piperidine-4-carboxylic acid ethyl ester was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of the trimethylsilanyl-ethyl ester with tetra-n-butylammoniumflouride (TBAF) and 1 N HCl to provide 1-{2-[3-(3-carboxy-propionyl)-5-chloro-indol-1-yl]-6-furan-2-yl-quinazolin-4-yl}-piperidine-4-carboxylic acid ethyl ester: ; R_{f} 0.30 (CH₂Cl₂/MeOH, 33:1, v/v). ¹H NMR (CDCl₃, 300 MHz) δ 9.00 (s, 1H), 8.80 (d, *J* = 9 Hz, 1H), 8.38 (d, *J* = 2 Hz, 1H), 8.11 (d, *J* = 2Hz, 1H), 7.98 (dd, *J* = 9, 2 Hz, 1H), 7.84 (d, *J* = 9 Hz, 1H), 7.52 (d, *J* = 2 Hz, 1H), 7.31 (dd, *J* = 9, 2 Hz, 1H), 6.74 (d, *J* = 3 Hz, 1H), 6.53 (dd, *J* = 3, 2 Hz, 1H), 9.41-4.36 (m, 2H), 4.21 (q, *J* = 7 Hz, 2H), 3.46-3.39 (m, 2H), 3.34 (t, *J* = 6 Hz, 2H), 2.84 (t, *J* = 6 Hz, 2H), 2.78-2.71 (m, 1H), 2.23-2.04 (m, 4H), 1.31 (t, *J* = 7 Hz, 3H). ESI-LCMS *m*/*z* calcd for C₃₂H₂₉ClN₄O₆ : 601.0 ; found 601.3 (M + 1)⁺.

### Reference Example 25 (Reference compound no. 77)

### Preparation of 1-{2-[5-Bromo-3-(3-carboxy-propionyl)-indol-1-yl]-6-furan-2-yl-quinazolin-4-yl}-piperidine-4-carboxylic acid ethyl ester

This compound was prepared in a manner analogous to that set forth in example C, except piperidine-4-carboxylic acid ethyl ester was used instead of p-anisidine in step 5 and 4-(5-bromo-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 1-{2-[5-bromo-3-(3-carboxy-propionyl)-indol-1-yl]-6-furan-2-yl-quinazolin-4-yl}-piperidine-4-carboxylic acid ethyl ester as a white solid: R_{f} 0.34 (CH₂Cl_{2/}MeOH, 33:1, v/v). ¹H NMR (CDCl₃, 300 MHz) δ 9.02 (s, 1H), 8.78 (d, *J* = 9 Hz, 1H), 8.57 (d, *J* = 2Hz, 1H), 8.13 (d, *J* = 2 Hz, 1H) 7.99 (dd, *J* = 9, 2 Hz, 1H), 7.86 (d, *J* = 9 Hz, 1H), 7.52 (d , *J* = 2 Hz, 1H), 7.46 (dd, *J* = 9, 2 Hz, 1H), 6.74 (d, *J* = 3 Hz, 1H), 6.53 (dd, *J* = 3, 2 Hz, 1H), 4.42-4.38 (m, 2H), 4.21 (q, *J* = 7 Hz, 2H), 3.48-3.40 (m, 2H), 3.35 (t, *J* = 6 HZ, 2H), 2.87 (t, *J* = 6 Hz, 2H), 2.78-2.71 (m, 1H), 2.24-2.04 (m, 4H), 1.31 (t, *J* = 7 Hz, 3H).

### REFERENCE EXAMPLE 26 (REFERENCE COMPOUND NO. 79)

### Preparation of 4-[5-Chloro-1-(6-furan-2-yl-4-morpholin-4-yl-quinazolin-2-yl)-1H-indol-3-yl]-4-oxo-butyric acid

This compound was prepared in a manner analogous to that set forth in example C, except morpholine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-[5-chloro-l-(6-furan-2-yl-4-morpholin-4-yl-quinazolin-2-yl)-1*H*-indol-3-yl]-4-oxo-butyric acid as a white solid: ; R_{f} 0.23 (CH₂Cl₂/MeOH, 33:1, v/v). ¹H NMR (DMSO-d₆, 300 MHz) δ 12. 12 (br s, 1H), 9.12 (s, 1H), 8.88 (d, *J* = 9 Hz, 1H), 8.21 (d, *J* = 2 Hz, 1H), 8.19-8.16 (m, 2H), 7.91 (d, *J* = 9Hz, 1H), 7.83 (d, *J* = 2 Hz, 1H), 7.41 (dd, *J* = 9, 2 Hz, 1H), 7.14 (d, *J* = 3 Hz, 1H), 6.65 (dd, *J* = 3, 2 Hz, 1H), 3.97-3.96 (m, 9H), 3.88-3.87 (m, 4H), 3.27 (t, *J* = 6 Hz, 2H), 2.62 (t, *J* = 6 Hz, 2H). ESI-LCMS *m*/*z* calcd for C₂₉H₂₃ClN₄O₅ : 530.9 ; found 531.3 (M + 1)⁺.

### Reference Example 27 (Reference compound no. 81)

### Preparation of 4-{5-Chloro-1-[6-furan-2-yl-4-(4-hydroxy-piperidin-1-yl)-quinazolin-2-yl]-1H-indol-3-yl}-4-oxo-butyric acid

This compound was prepared in a manner analogous to that set forth in example C, except 4-hydroxypiperidine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-(5-chloro-1-[6-furan-2-yl-4-(4-hydroxy-piperidin-1-yl)-quinazolin-2-yl]-1*H*-indol-3-yl)-4-oxo-butyric acid as a yellow solid: R_{f} 0.18 (CH₂Cl₂/MeOH, 19:1, v/v). ¹H NMR (DMSO-*d*₆, 300 MHz) δ 12.10 (br s, 1H), 9.15 (s, 1H), 8.92 (d, *J* = 9 Hz, 1H), 8.23 (d, *J* = 2 Hz, 1H), 8.18-8.15 (m, 2H), 7.89 (d, *J* = 9 Hz, 1H), 7.82 (d, *J* = 2 Hz, 1H), 7.43 (dd, *J* = 9, 2 Hz, 1H), 7.12 (d, *J* = 3 Hz, 1H), 6.65 (dd, *J* = 3, 2 Hz, 1H), 4.89 (br s, 1H) 4.26-4.22 (m, 2H), 3.90 (m, 1H), 3.68-3.58 (m, 2H), 3.28 (t, *J* = 6 Hz, 2H), 2.62 (t, *J* = 6 Hz, 2H), 2.02 (m, 2H), 1.75-1.68 (m, 2H).

### REFERENCE EXAMPLE 28 (REFERENCE COMPOUND NO. 112)

### Preparation of 4-{5-Chloro-1-[6-furan-2-yl-4-(4-hydroxymethyl-piperidin-1-yl)-quinazolin-2-yl]-1H-indol-3-yl}-4-oxo-butyric acid

This compound was prepared in a manner analogous to that set forth in example C, except 4-hydroxymethylpiperidine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-{5-chloro-1-[6-furan-2-yl-4-(4-hydroxymethyl-piperidin-1-yl)-quinazolin-2-yl]-1*H*-indol-3-yl}-4-oxo-butyric acid as a yellow solid: ; R_{f} 0.20 (CH₂Cl₂/MeOH, 19:1, v/v). ¹H NMR (DMSO-*d*₆, 300 MHz) δ 12.10 (br s, 1H), 9.15 (s, 1H), 8.92 (d, *J* = 9 Hz, 1H), 8.22 (d, *J* = 2 Hz, 1H), 8.18-8.15 (m, 2H), 7.89 (d, *J* = 9 Hz, 1H), 7.83 (d, *J* = 2 Hz, 1H), 7.43 (dd, *J* = 9, 2 Hz, 1H), 7.12 (d, *J* = 3 Hz, 1H), 6.65 (dd, *J* = 3, 2 Hz, 1H), 4.61 (br s, 1H), 4.54-4.50 (m, 2H), 3.36-3.33 (m, 4H), 3.28 (t, *J* = 6 Hz, 2H), 2.62 (t, *J* = 6 Hz, 2H), 1.95-1.83 (m, 3H), 1.55-1.50 (m, 2H).

### REFERENCE EXAMPLE 29 (REFERENCE COMPOUND NO. 67)

### Preparation of 4-{5-Chloro-1-[4-(4-fluoro-benzylamino)-6-furan-2-yl-quinazolin-2-yl]-1H-indol-3-yl}-4-oxo-butyric acid

This compound was prepared in a manner analogous to that set forth in example C, except 4-fluoro-benzylamine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-{5-chloro-1-[4-(4-fluoro-benzylamino)-6-furan-2-yl-quinazolin-2-yl]-1*H*-indol-3-yl}-4-oxo-butyric acid as a light yellow solid: R_{f} 0.40 (CH₂Cl₂/MeOH, 33:1, v/v). ¹H NMR (DMSO-*d*₆, 300 MHz) δ 12.15 (br s, 1H), 9.55 (t, *J* = 5 Hz, 1H), 9.01 (s, 1H), 8.81 (d, *J* = 9 Hz, 1H), 8.67 (d, *J* = 2 Hz, 1H), 8.20 (d, *J* = 2 Hz, 1H), 8.15 (dd, *J* = 9, 2 Hz, 1H), 7.84 (d, *J* = 2 Hz, 1H) 7.82 (d, *J* = 9 Hz, 1H), 7.58-7.53 (m, 2H), 7.34 (dd, *J* = 9, 2 Hz, 1H), 7.22-7.16 (m, 2H), 7.06 (d, *J* = 3 Hz, 1H), 6.67 (dd, *J* = 3, 2 Hz, 1H), 4.89 (d, *J* = 5 Hz, 2H), 3.26 (t, *J* = 6 Hz, 2H), 2.64 (t, *J* = 6 Hz, 2H). ESI-LCMS *m*/*z* calcd for C₃₁H₂₂ClFN₄O₄ : 568.9 ; found 569.3 (M + 1)⁺.

### REFERENCE EXAMPLE 30 (REFERENCE COMPOUND NO. 69)

### Preparation of 4-(5-Chloro-1-{6-furan-2-yl-4-[(pyridin-4-ylmethyl)-amino]-quinazolin-2-yl}-1H-indol-3-yl)-4-oxo-butyric acid

This compound was prepared in a manner analogous to that set forth in example C, except 4-(aminomethyl)pyridine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-(5-chloro-1-{6-furan-2-yl-4-[(pyridin-4-ylmethyl)-amino]-quinazolin-2-yl}-1*H*-indol-3-yl)-4-oxo-butyric acid as a tan solid: R_{f} 0.10 (CH₂Cl₂/MeOH, 19:1, v/v). ¹H NMR (DMSO-*d*₆, 300 MHz) δ 9.89 (t, *J* = 5 Hz, 1H), 8.89 (s, 1H), 8.80-8.78 (m, 3H), 8.72 (d, *J* = 9 Hz, 1H), 8.22-8.17 (m, 2H), 8.09 (d, *J* = 6 Hz, 2H), 7.88-7.85 (m, 2H), 7.34 (dd, *J* = 9, 2 Hz, 1H), 7.13 (d, *J* = 3 Hz, 1H), 6.69 (dd, *J* = 3, 2 Hz, 1H), 5.14 (d, *J* = 5 Hz, 2H), 3.21 (t, *J* = 6 Hz, 2H), 2.63 (t, *J* = 6 Hz, 2H).

### REFERENCE EXAMPLE 31 (REFERENCE COMPOUND NO. 89)

### Preparation of 4-{1-[4-(Carbamoylmethyl-amino)-6-furan-2-yl-quinazolin-2-yl]-5-chloro-1H-indol-3-yl}-4-oxo-butyric acid

This compound was prepared in a manner analogous to that set forth in example C, except glycinamide was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-{1-[4-(carbamoylmethyl-amino)-6-furan-2-yl-quinazolin-2-yl]-5-chloro-1*H*-indol-3-yl}-4-oxo-butyric acid as a tan solid R_{f} 0.27 (CH₂Cl₂/MeOH, 19:1, v/v). ¹H NMR (DMSO-*d*₆, 300 MHz) δ 9.32 (br s, 1H), 9.21 (s, 1H), 8.97 (d, *J* = 9 Hz, 1H), 8.64 (d, *J* = 2 Hz, 1H), 8.22 (d, *J* = 2 Hz, 1H), 8.15 (dd, *J* = 9, 2 Hz, 1H), 7.85-7.82 (m, 2H), 7.41 (dd, *J* = 9, 2 Hz, 1H), 7.25 (m, 2H), 7.05 (d, *J* = 3 Hz, 1H), 6.66 (dd, *J* = 3, 2 Hz, 1H), 4.18 (d, *J* = 5 Hz, 2H), 3.25 (t, *J* = 6 Hz, 2H), 2.60 (t, *J* = 6 Hz, 2H). ESI-LCMS *m*/*z* calcd for C₂₆H₂₀ClN₅O₅ : 517.9. ; found 516.5 (M - 1)⁻.

### REFERENCE EXAMPLE 32 (REFERENCE COMPOUND NO. 88)

### Preparation of 4-{5-Chloro-1-[6-furan-2-yl-4-(2-methanesulfonyl-ethylamino)-quinazolin-2-yl]-1H-indol-3-yl}-4-oxo-butyric acid

This compound was prepared in a manner analogous to that set forth in example C, except 2-aminoethylmethyl sulfone was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-{5-chloro-1-[6-furan-2-yl-4-(2-methanesulfonyl-ethylamino)-quinazolin-2-yl]-1*H*-indol-3-yl}-4-oxo-butyric acid as a yellow solid: R_{f} 0.22 (CH₂Cl₂/MeOH, 19:1, v/v). ¹H NMR (DMSO-*d*₆, 300 MHz) δ 9.25 (m, 2H), 8.98 (d, *J* = 9 Hz, 1H), 8.53 (s, 1H), 8.21 (d, *J* = 2 Hz, 1H), 8.11 (d, *J* = 9 Hz, 1H), 7.82-7.79 (m, 2H), 7.39 (dd, *J* = 9, 2 Hz, 1H), 7.04 (d, *J* = 3, 2 Hz, 1H), 6.66 (dd, *J* = 3, 2 Hz, 1H), 4.11 (m, 2H), 3.63 (m, 2H), 3.24 (t, *J* = 6 Hz, 2H), 3.12 (s, 3H), 2.56 (t, *J* = 6 Hz, 2H). ESI-LCMS *m*/*z* calcd for C₂₇H₂₃ClN₄O₆S: 567.0 ; found 567.3 (M + 1)⁺.

### REFERENCE EXAMPLE 33 (REFERENCE COMPOUND NO. 113)

### Preparation of 4-(5-Chloro-1-{4-[(furan-2-ylmethyl)-amino]-6,7-dimethoxy-quinazolin-2-yl}-1H-indol-3-yl)-4-oxo-butyric acid.

This compound was prepared in a manner analogous to that set forth in example C, except furfurylamine and 2,4-dichloro-6,7-dimethoxyquinazoline was used instead of p-anisidine and 2,4-dichloro-6-furan-2-yl-quinazoline in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-(5-chloro-1-{4-[(furan-2-ylmethyl)-amino]-6,7-dimethoxy-quinazolin-2-yl}-1H-indol-3-yl)-4-oxo-butyric acid as a yellow solid: R_{f} 0.18 (CH₂Cl₂/MeOH, 19:1, v/v) ¹H NMR (DMSO-*d*₆, 300 MHz) δ 9.18 (s, 1 H), 8.94-8.91 (m, 1 H), 8.93 (d, *J* = 8.9 Hz, 1 H), 8.23 (d, *J* = 2.2 Hz, 1 H), 7.74 (s, 1 H), 7.63 (s with further small coupling, 1 H), 7.39 (dd, *J* = 8.9, 2.2 Hz, 1 H), 7.26 (s, 1 H), 6.45-6.41 (comp, 2 H), 4.88 (d, *J* = 3.8 Hz, 1 H), 3.95 (s, 3H), 3.90 (s, 3 H), 3.28 (t, *J* = 6.4 Hz, 2 H), 2.64 (t, *J* = 6.4 Hz, 2 H). ESI-LCMS *m*/*z* calcd for C₂₇H₂₃ClN₄O₆ : 539.13; found 533.0 (M - 1)⁻.

### REFERENCE EXAMPLE 34 (REFERENCE COMPOUND NO. 70)

### Preparation of 4-{5-Chloro-1-[6,7-dimethoxy-4-(4-methoxy-benzylamino)-quinazolin-2-yl]-1H-indol-3-yl}-4-oxo-butyric acid

This compound was prepared in a manner analogous to that set forth in example C, except 4-methoxybenzyl amine and 2,4-dichloro-6,7-dimethoxyquinazoline was used instead of p-anisidine and 2,4-dichloro-6-furan-2-yl-quinazoline in step 5 and 4-(5-chloro-1*H*-indol-3-yl)-4-oxo-butyric acid 2-trimethylsilanyl-ethyl ester was used instead of 4-(5-bromo-1*H-*indol-3-yl)-4-oxo-butyric acid methyl ester in step 6 and step 7 was replaced by treatment of trimethylsilanyl-ethyl ester with TBAF and 1 N HCl to provide 4-{5-chloro-1-[6,7-dimethoxy-4-(4-methoxy-benzylamino)-quinazolin-2-yl]-1*H*-indol-3-yl}-4-oxo-butyric acid as a yellow solid: R_{f} 0.18 (CH₂Cl₂/MeOH, 19:1, v/v) ¹H NMR (DMSO-*d*₆, 300 MHz) δ 9.08 (s, 1 H), 9.0 (br s, 1 H), 8.84 (d, *J* = 8.8 Hz, 1 H), 8.21 (d, *J* = 2.3 Hz, 1 H), 7.75 (s, 1 H), 7.41 (d, *J* = 8.8 Hz, 2 H), 7.33 (dd, *J* = 8.8, 2.3 Hz, 1 H), 7.25 (s, 1 H), 6.92 (d, *J* = 8.8 Hz, 2 H), 4.80 (br d, *J* = 4.7 Hz, 1 H), 3.95 (s, 3 H), 3.91 (s, 3 H), 3.69 (s, 3 H), 3.26 (t, *J* = 6.5 Hz, 2 H), 2.64 (t, *J* = 6.5 Hz, 2 H).
ESI-LCMS m/z calcd for C₃₀H₂₇ClN₄O₆: 574.16; found 573.0 (M -1)-.

### REFERENCE EXAMPLE 35 (REFERENCE COMPOUND NO. 80)

### Preparation of 1-{2-[3-(3-Carboxy-propionyl)-5-chloro-indol-1-yl]-6-nitro-quinazolin-4-yl}-piperidine-4-carboxylic acid ethyl ester

This compound was prepared in a manner analogous to that set forth in example C, except 2-amino-5-nitro-benzonitrile was used instead of 2-amino-5-bromo-benzonitrile in step 1, piperidine-4-carboxylic acid ethyl ester was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 1-{2-[3-(3-carboxypropionyl)-5-chloro-indol-1-yl]-6-nitro-quinazolin-4-yl}-piperidine-4-carboxylic acid ethyl ester as a yellow solid R_{f} 0.28 (CH₂Cl₂/MeOH, 19:1, v/v) ¹H NMR (DMSO-*d*₆, 300 MHz) δ 9.15 (s, 1 H), 8.88 (d, *J* = 8.8 Hz, 1 H), 8.76 (d, *J* = 2.3 Hz, 1 H), 8.50 (dd, *J* = 9.3, 2.3 Hz, 1 H), 8.21 (d, *J* = 2. 3 Hz, 1 H), 7.96 (d, *J* = 9.3 Hz, 1 H), 7.44 (dd, *J* = 8.8, 2.3 Hz, 1 H), 9.51 (br d, *J* = 12.9 Hz, 2 H), 4.11 (q, *J* = 7.1 Hz, 2 H), 3.68-3.56 (comp, 2 H), 3.34-3.26 (comp, 2 H), 2.92-2.82 (m, 1 H), 2.62 (t, *J* = 5.7 Hz, 2 H), 2.18-2.08 (comp, 2H), 1.98-1.82 (comp, 2 H), 1.22 (t, *J* = 7.1 Hz, 2 H).
ESI-LCMS *m*/*z* calcd for C₂₉H₂₆ClN₅O₇: 579.15; found 578.0 (M - 1)⁻.

### REFERENCE EXAMPLE 36 (REFERENCE COMPOUND NO. 114)

### Preparation of 1-{6-Amino-2-[3-(3-carboxy-propionyl)-5-chloro-indol-1-yl]-quinazolin-4-yl}-piperidine-4-carboxylic acid ethyl ester

This compound was prepared in a manner analogous to that set forth in example C, except 2-amino-5-nitro-benzonitrile was used instead of 2-amino-5-bromo-benzonitrile in step 1, piperidine-4-carboxylic acid ethyl ester was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6. Step 6 product was reacted with hydrogen over palladium and step 7 was replaced by treatment of trimethylsilanyl-ethyl ester with TBAF and 1 N HCl to provide 1-{6-amino-2-[3-(3-carboxy-propionyl)-5-chloro-indol-1-yl]-quinazolin-4-yl}-piperidine-4-carboxylic acid ethyl ester as a yellow solid: R_{f} 0.24 (CH₂Cl₂/MeOH, 19:1, v/v) ¹H NMR (DMSO-*d*₆, 300 MHz) δ 9.14 (s, 1 H), 8.90 (d, *J* = 9.1 Hz, 1 H), 8.24 (d, *J* = 2.3 Hz, 1 H), 7.79 (d, *J* = 8.8 Hz, 1 H), 7.47-7.40 (comp, 3 H), 4.31 (br d, *J* = 13.8 Hz, 2 H), 4.11 (q, *J* = 7.0 Hz, 2 H), 3.38-3.25 (comp, 4 H), 2.85-2.70 (m, 1 H), 2.62 (t, *J* = 6.3 Hz, 2 H), 2.15-2.05 (comp, 2H), 1.95-1.80 (comp, 2 H), 1.22 (t, *J* = 7.0 Hz, 3 H).
ESI-LCMS *m*/*z* calcd for C₂₈H₂₈ClN₅O₅: 549.18; found 550.0 (M + 1)⁺.

### Reference Example 37 (Reference compound no. 115)

### Preparation of 4-[1-(6-Amino-4-morpholin-4-yl-quinazolin-2-yl)-5-chloro-1H-indol-3-yl]-4-oxo-butyric acid.

This compound was prepared in a manner analogous to that set forth in example C, except 2-amino-5-nitro-benzonitrile was used instead of 2-amino-5-bromo-benzonitrile in step 1, morpholine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6. Step 6 product was reacted with hydrogen over palladium and step 7 was replaced by treatment of trimethylsilanyl-ethyl ester with TBAF and 1 N HCl to provide 4-[1-(6-amino-4-morpholin-4-yl-quinazolin-2-yl)-5-chloro-1H-indol-3-yl]-4-oxo-butyric acid as a brown solid: R_{f} 0.19 (CH₂Cl₂/MeOH, 19:1, v/v) ¹H NMR (DMSO-*d*₆, 300 MHz) δ 9.14 (s, 1 H), 8.84 (d, *J* = 9.1 Hz, 1 H), 8.24 (d, *J* = 2.2 Hz, 1 H), 7.82 (br d, *J* = 8.8 Hz, 1 H), 7.60-7.46 (comp, 2 H), 7.43 (dd, *J* = 9.1, 2.2 Hz, 2 H), 3.92-3.80 (comp, 8 H), 3.28 (t, *J* = 6.4 Hz, 2 H), 2.62 (t, *J* = 6.4 Hz, 2 H). ESI-LCMS *m*/*z* calcd for C₂₄H₂₂ClN₅O₄: 479.14; found 480.3 (M + 1)⁺.

### Reference Example 38 (Reference compound no. 82)

### Preparation of 1-[2-[3-(3-Carboxy-propionyl)-5-chloro-indol-1-yl]-6-(2-oxo-pyrrolidin-1-yl)-quinazolin-4-yl]-piperidine-4-carboxylic acid ethyl ester

This compound was prepared in a manner analogous to that set forth in example C, except piperidine-4-carboxylic acid ethyl ester and 6-bromo-2,4-dichloroquinazoline were used instead of p-anisidine and 2,4-dichloro-6-furan-2-yl-quinazoline in step 5 and 4-(5-chloro-1*H*-indol-3-yl)-4-oxo-butyric acid 2-trimethylsilanyl-ethyl ester was used instead of 4-(5-bromo-1*H-*indol-3-yl)-4-oxo-butyric acid methyl ester in step 6. Step 6 product was reacted with pyrrolidine and 4,5-bis(diphenylphosphino-9,9-dimethylxanthine (Xantphos) over Pd₂(dba)₃ in toluene at 100 °C and step 7 was replaced by treatment of trimethylsilanyl-ethyl ester with TBAF and 1 N HCl to provide 1-[2-[3-(3-carboxy-propionyl)-5-chloro-indol-1-yl]-6-(2-oxo-pyrrolidin-1-yl)-quinazolin-4-yl]-piperidine-4-carboxylic acid ethyl ester as a tan solid (0.011 g, 42%): R_{f} 0.29 (CH₂Cl₂/MeOH, 19:1, v/v) ¹H NMR (DMSO-*d*₆, 300 MHz) δ 9.17 (s, 1 H), 8.80 (d, *J* = 8.8 Hz, 1 H), 8.40 (d, *J* = 2.2 Hz, 1 H), 8.25 (d, *J* = 2.3 Hz, 1 H), 8.08 (dd, *J* = 9.1, 2.2 Hz, 1 H), 7.91 (d, *J* = 9.1 Hz, 1 H), 7.45 (dd, *J* = 8.8, 2.3 Hz, 1 H), 4.42 (br d, *J* = 14.1 Hz, 2 H), 4.11 (q, *J* = 7.1 Hz, 2 H), 3.98 (t, *J* = 6.9 Hz, 2 H), 3.49-3.25 (comp, 4 H), 2.85-2.75 (m, 1 H), 2.65-2.54 (comp, 4 H), 2.15-2.05 (comp, 4 H), 1.95-1.80 (comp, 2 H), 1.22 (t, *J* = 7.1 Hz, 3 H).
ESI-LCMS *m*/*z* calcd for C₃₂H₃₂ClN₅O₆: 617.2; found 618.0 (M + 1)⁺.

### Reference Example 39 (Reference compound no. 116)

### Preparation of 4-{5-Chloro-1-[4-(4-methoxy-benzylamino)-6-trifluoromethyl-quinazolin-2-yl]-1H-indol-3-yl}-4-oxo-butyric acid

This compound was prepared in a manner analogous to that set forth in example C, except 2-amino-5-trifluoromethylbenzonitrile was used instead of 2-amino-5-bromobenzonitrile in step 1, 4-methoxybenzyl amine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-{5-chloro-1-[4-(4-methoxy-benzylamino)-6-trifluoromethyl-quinazolin-2-yl]-1H-indol-3-yl}-4-oxo-butyric acid as a tan solid: R_{f} 0.67 (CH₂Cl₂/MeOH, 12:1, v/v) ¹H NMR (DMSO-*d*₆, 300 MHz) δ 9.66 (br t, *J* = 5.3 Hz, 1 H), 9.07 (s, 1 H), 8.85-8.80 (comp, 2 H), 8.18 (d, *J* = 2.6 Hz, 1 H), 8.04 (dd, *J* = 8.8, 2.6 Hz, 1 H), 7.91 (d, *J* = 8.8 Hz, 1 H), 7.93 (d, *J* = 8.6 Hz, 2 H), 7.34 (dd, *J* = 9.1, 2.3 Hz, 1 H), 6. 93 (d, *J* = 8.6 Hz, 2 H), 6.67 (dd, *J* = 3.2, 1.7 Hz, 1 H), 4.82 (br d, *J* = 5.3 Hz, 2 H), 3.69 (s, 3 H), 3.27 (t, *J* = 6.4 Hz, 2 H), 2.64 (t, *J* = 6. 9 Hz, 2 H). ESI-LCMS *m*/*z* calcd for C₂₉H₂₂ClF₃N₄O₄: 582.13; found 583.3 (M + 1)⁺.

### Reference Example 40 (Reference compound no. 83)

### Preparation of 4-{5-Chloro-1-[4-(4-hydroxy-piperidin-1-yl)-6-trifluoromethyl-quinazolin-2-yl]-1H-indol-3-yl}-4-oxo-butyric acid

This compound was prepared in a manner analogous to that set forth in example C, except 2-amino-5-trifluoromethylbenzonitrile was used instead of 2-amino-5-bromobenzonitrile in step 1, 4-hydroxypiperidine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6 and step 7 was replaced by treatment of trimethylsilanyl-ethyl ester with TBAF and 1 N HCl to provide 4-{5-chloro-1-[4-(4-hydroxy-piperidin-1-yl)-6-trifluoromethyl-quinazolin-2-yl]-1H-indol-3-yl}-4-oxo-butyric acid as an orange solid: R_{f} 0.23 (CH₂Cl₂/MeOH, 12:1, v/v) ¹H NMR (DMSO-*d*₆, 300 MHz) δ 9.14 (s, 1 H), 8.89 (d, *J* = 8.9 Hz, 1 H), 8.21 (s, 2 H), 8.04 (1/2AB, *J* = 9.1 Hz, 1 H), 8.03 (1/2AB, *J* = 9.1 Hz, 1 H), 7.42 (d, *J* = 8.9 Hz, 1 H), 4.91 (d, *J* = 8.9 Hz, 1 H), 4.28-4.19 (comp, 2 H), 3.96-3.86 (m, 1 H), 3.76-3.64 (comp, 2 H), 3.28 (t, *J* = 6.9 Hz, 2 H), 2.62 (t, *J* = 6.9 Hz, 2 H), 2.08-1.98 (comp, 2 H), 1.74-1.60 (comp, 2 H). ESI-LCMS *m*/*z* calcd for C₂₆H₂₂ClF₃N₄O₄: 546.13; found 569.3 (M + Na + 1)⁺.

### Reference Example 41 (Reference compound no. 84)

### Preparation of 4-{5-Chloro-1-[4-(4-hydroxymethyl-piperidin-1-yl)-6-trifluoromethyl-quinazolin-2-yl]-1H-indol-3-yl}-4-oxo-butyric acid

This compound was prepared in a manner analogous to that set forth in example C, except 2-amino-5-trifluoromethylbenzonitrile was used instead of 2-amino-5-bromobenzonitrile in step 1, 4-hydroxymethylpiperidine was used instead of p-anisidine in step 5 and 4-(5-chloro-1*H*-indol-3-yl)-4-oxo-butyric acid 2-trimethylsilanyl-ethyl ester was used instead of 4-(5-bromo-1*H*-indol-3-yl)-4-oxo-butyric acid methyl ester in step 6. Step 7 was replaced by treatment of trimethylsilanyl-ethyl ester with TBAF and 1 N HCl to provide 4-{5-chloro-1-[4-(4-hydroxymethyl-piperidin-1-yl)-6-trifluoromethyl-quinazolin-2-yl]-1H-indol-3-yl}-4-oxo-butyric acid as a yellow solid: R_{f} 0.36 (CH₂Cl₂/MeOH, 12:1, v/v) ¹H NMR (DMSO-*d*₆, 300 MHz) δ 9.10 (s, 1 H), 8.86 (d, *J* = 9.0 Hz, 1 H), 8.20-8.15 (comp, 2 H), 8.02 (1/2AB, *J* = 8.8 Hz, 1 H), 8.01 (1/2AB, *J* = 8.8 Hz, 1 H), 7.40 (dd, *J* = 9.0, 2.3 Hz, 1 H), 4.60-4.46 (comp, 3 H), 3.47-3.24 (comp, 6 H), 2.62 (t, *J* = 6.3 Hz, 2 H), 2.00-1.80 (comp, 3 H), 1.56-1.38 (comp, 2 H).
ESI-LCMS *m*/*z* calcd for C₂₇H₂₄ClF₃N₄O₄: 560.14; found 583.3 (M + Na + 1)⁺.

### Reference Example 42 (Reference compound no. 117)

### Preparation of 4-{5-Chloro-1-[4-(4-methoxy-phenylamino)-6-trifluoromethyl-quinazolin-2-yl]-1H-indol-3-yl}-4-oxo-butyric acid

This compound was prepared in a manner analogous to that set forth in example X, except 4-{5-chloro-1-[4-(4-methoxy-phenylamino)-6-trifluoromethyl-quinazolin-2-yl]-1*H*-indol-3-yl}-4-oxo-butyric acid 2-trimethylsilanyl-ethyl ester (0.100 g, 0.150 mmol) was used instead of xxxxx to provide 4-{5-chloro-1-[4-(4-methoxy-phenylamino)-6-trifluoromethyl-quinazolin-2-yl]-1H-indol-3-yl}-4-oxo-butyric acid as a yellow solid (0.065, 76%): R_{f} 0.20 (CH₂Cl₂/MeOH, 19:1, v/v) ¹H NMR (DMSO-*d*₆, 300 MHz) δ 10.52 (s, 1 H), 8.95 (s, 1 H), 8.93 (s, 1 H), 8.58 (d, *J* = 9.0 Hz, 1 H), 8.12 (d, *J* = 2.0 Hz, 1 H), 8.06 (dd, *J* = 8.7, 2.0 Hz, 1 H), 7.91 (d, *J* = 8.7 Hz, 1 H), 7.62 (d, *J* = 8.9 Hz, 2 H), 7.15 (dd, *J* = 9.0, 2.2 Hz, 1 H), 7.09 (d, *J* = 8.9 Hz, 2 H), 3.84 (s, 3 H), 3.15 (t, *J* = 6.4 Hz, 2 H), 2.60 (t, *J* = 6.4 Hz, 2 H). ESI-LCMS *m*/*z* calcd for C₂₈H₂₀ClF₃N₄O₄: 568.11; found 569.3 (M + 1)⁺.

### Reference Example 43 (Reference compound no. 118)

### 2-Chloro-4,6-diphenylpyrimidine.

A mixture of 2,4,6-Trichloropyrimidine, 2.76g (15.0 mmol), phenylboronic acid, 3.66g (30.0 mmol), Pd(OAc)₂, 86mg (0.38 mmol), triphenylphosphine, 200mg (0.76 mmol) in 150mL of ethylene glycol dimethyl ether was heated to obtain a clear solution. To the solution was added 25mL of 4.0M aq. Na₂CO₃. The reaction mixture was refluxed for 24h at 70 □C. The mixture was cooled to room temperature and diluted with 100mL ethyl acetate. The organic layer was washed with water (2x50mL), sat. aq. NaCl (1×50mL), and dried (MgSO₄). After the solution was concentrated, the residue was recrystallized with Et₂O-Heptane (1:3) to afford the desired product in 1.64g (41%) as a pale yellow solid. ¹H NMR (CDCl₃): δ 8.15-8.12 (m, 4H), 8.02 (s, 1H), 7.57-7.51 (m, 6H).

### Reference Example 44 (Reference compound no. 119)

### 4-(5-Chloro-1H-indol-3-yl)-4-oxo-butyric acid 2-trimethylsilanyl-ethyl ester.

To a two-necked, round bottom flask equipped with an addition funnel containing 5Å molecular sieves topped with a jacketed water condenser was added the known compound 4-(5-Chloro-1H-indol-3-yl)-4-oxo-butyric acid methyl ester, 32.0g (0.12 mol), 2-trimethylsilanyl-ethanol, 51.8mL (0.36 mol), and LiBr, 52.3g (0.60 mol); and 500mL of THF/CH₂Cl₂ (3:1). The mixture was stirred at reflux fro 24h. The mixture was cooled to room temperature, concentrated to 200mL, and diluted with 200mL of EtOAc. The mixture was washed with water (3x300mL), sat. aq. NaCl (2x300mL), and dried (MgSO₄). After the solution was concentrated, the residue was recrystallized with CH₂Cl₂-Heptane (1:2) to afford the desired product in 27.6g (65%) as an off-white solid. ¹H NMR (CDCl₃) : δ 8.43 (s, 1H), 8.11 (d, 1H, J = 2.3Hz), 7.48 (d, 1H, J = 9.3Hz), 7.21 (dd, 1H, J = 9.3, 2.3Hz), 4.09 (dd, 2H, J = 9.0, 9.0Hz), 3.16 (t, 2H, J = 7.2Hz), 2.61 (t, 2H, J = 7.2Hz), 0.93 (dd, 2H, J = 9.0, 9.0Hz), 0.02 (s, 9H).

### Reference Example 45 (Reference compound no. 92)

### 4-[5-Chloro-1-(4,6-diphenyl-pyrimidin-2-yl)-1H-indol-3-yl]-4-oxo-butyric acid.

To a solution of 4-[5-chloro-1-(9,6-diphenyl-pyrimidin-2-yl)-1H-indol-3-yl]-4-oxo-butyric acid 2-trimethylsilanylethyl ester, 160mg (0.27 mmol), in 25mL of CH₂Cl₂ under a nitrogen atmosphere was added trifluoroacetic acid, 1.04mL (13.5mmol). The solution was stirred at room temperature overnight. The solution was concentrated to dryness, diluted with toluene, and concentrated again. This concentration/dilution process was repeated three more times. The remaining residue was crystallized with acetone-heptane (1:2) to afford the desired product in 104mg (80%) as a pale yellow solid. R_{f} 0.15 (50% EtOAc-heptane) : ¹H NMR (DMSO-d₆) : δ 9.43 (s, 1H), 8.91 (d, 1H, J = 10.0Hz), 8.61 (s, 1H), 8.55-8.52 (m, 3H), 8.31 (d, 1H, J = 2.3Hz), 7.68-7.66 (m, 5H), 7.56 (dd, 1H, J = 10.0, 2.3Hz), 3.39 (t, 2H, J = 6.3Hz), 2.67 (t, 2H, J = 6.3Hz). LCMS *m*/*z* calcd for C₂₈H₂₀ClN₃O₃: 481.1 found 481.4 (M+1).

### Reference Example 46 (Reference compound no. 97)

### 4-[5-Chloro-1-(4-phenyl-6-phenylamino-pyrimidin-2-yl)-1H-indol-3-yl]-4-oxo-butyric acid.

This compound was synthesized as reported previously, starting with aniline instead of phenylboronic acid to afford 4-[5-chloro-1-(4-phenyl-6-phenylamino-pyrimidin-2-yl)-1H-indol-3-yl]-4-oxo-butyric acid. R_{f} 0.29 (50% EtOAc-heptane); ¹H NMR (DMSO-d₆): δ 10.1 (s, 1H), 9. 16 (s, 1H), 8.76 (d, 1H, J = 9.7Hz), 8.24 (d, 1H, J = 2.3Hz), 8.16-8.12 (m, 2H), 7.92 (s, 1H), 7.66-7.57 (m, 4H), 7.44 (t, 2H, J = 8.7), 7.36 (dd, 1H, J = 9.7, 2.3Hz), 3.39 (t, 2H, J = 6.3Hz), 2.60 (t, 2H, J = 6.3Hz). LCMS *m*/*z* calcd for C₂₈H₂₁ClN₄O₃: 496.1 found 997.3 (M+1).

### Reference Example 47 (Reference compound no. 121)

### 4-[5-Chloro-1-(2,6-diphenyl-pyrimidin-4-yl)-1H-indol-3-yl]-4-oxo-butyric acid.

This compound was synthesized as reported previously, starting with 4,6-dichloro-2-phenylpyrimidine, phenylboronic acid, and 4-(5-chloro-1H-indol-3-yl)-4-oxo-butyric acid methyl ester to afford 4-[5-chloro-1-(2,6-diphenyl-pyrimidin-4-yl)-1H-indol-3-yl]-4-oxo-butyric acid. R_{f} 0.10 (50% EtOAc-heptane); ¹H NMR (DMSO-d₆): δ 9.45 (s, 1H), 8.84 (d, 1H, J = 10.0Hz), 8.59-8.56 (m, 2H), 8.51-8.48 (m, 3H), 8.29 (d, 1H, J = 2.3Hz), 7.65-7. 62 (m, 6H), 7.55 (dd, 1H, J = 10. 0, 2.3Hz), 3.39 (t, 2H, J = 6.3Hz), 2. 60 (t, 2H, J = 6.3Hz). LCMS *m*/*z* calcd for C₂₈H₂₀ClN₃O₃: 481.1 found 482.5 (M+1).

### Reference Example 48 (Reference compound no. 99)

### 4-{5-Chloro-1-[5-(4-chloro-phenyl)-pyrimidin-2-yl]-1H-indol-3-yl}-4-oxo-butyric acid.

This compound was synthesized as reported previously, starting with 2-chloro-5-(4-chloro-phenyl)-pyrimidine and 4-(5-chloro-1H-indol-3-yl)-4-oxo-butyric acid methyl ester to afford 4-{5-chloro-1-[5-(4-chloro-phenyl)-pyrimidin-2-yl]-1H-indol-3-yl}-4-oxo-butyric acid. R_{f} 0.15 (50% EtOAc-heptane); ¹H NMR (DMSO-d₆): δ 9.28 (s, 2H), 9.19 (s, 1H), 8.78 (d, 1H, J = 9.7Hz), 8.25 (d, 1H, J = 2.3Hz), 7.91 (d, 2H, J = 9.3Hz), 7.62 (d, 2H, J = 9.3Hz), 7.49 (dd, 1H, J = 9.7, 2.3Hz), 3.29 (t, 2H, J = 6.3Hz), 2.60 (t, 2H, J = 6.3Hz). LCMS *m*/*z* calcd for C₂₂H₁₅Cl₂N₃O₃ : 439.1 found 440.0 (M+1).

### Reference Example 49 (Reference compound no. 122)

### 4-[5-Chloro-1-(3-cyano-,6-diphenyl-pyridin-2-yl)-1H-indol-3-yl]-4-oxo-butyric acid.

This compound was synthesized as reported previously, starting with the commercially available 2-chloro-3-cyano-4,6-diphenyl-pyridine and 4-(5-chloro-1H-indol-3-yl)-4-oxo-butyric acid methyl ester to afford 4-[5-chloro-1-(3-cyano-4,6-diphenyl-pyridin-2-yl)-1H-indol-3-yl]-4-oxo-butyric acid. R_{f} 0.79 (100% EtOAc); ¹H NMR (DMSO-d₆) 9.19 (s, 1H), 8.40 (s, 1H), 8.32-8.27 (m, 3H), 7.96-7.88 (m, 3H), 7.66-7.63 (m, 3H), 7.58-7.54 (m, 3H), 7.44 (dd, 1H, J = 9.7, 2.7Hz), 3.23 (t, 2H, J = 6.3Hz), 2.69 (t, 2H, J = 6.3Hz). LCMS *m*/*z* calcd for C₃₀H₂₀ClN₃O₃: 505.1 found 505.4 (M+1).

### Methods for measuring PTPLB activity

The test compounds were evaluated for their in vitro inhibitory activity against recombinant human PTP1B with phosphotyrosyl dodecapeptide TRDI(P)YETD(P)Y(P)YRK. This corresponds to the 1142-1153 insulin receptor kinase regulatory domain, phosphorylated on the 1146, 1150 and 1151 tyrosine residues; IR-triphosphopeptide)as a source of substrate. Enzyme reaction progression was monitored via the release of inorganic phosphate as detected by the malachite green - ammonium molybdate method for the phosphopeptide.

The invention and the manner and process of making and using it, are now described in such full, clear, concise and exact terms as to enable any person skilled in the art to which it pertains, to make and use the same. It is to be understood that the foregoing describes preferred embodiments of the invention and that modifications may be made therein without departing from the scope of the invention.

## Claims

1. A compound of the formula (II): 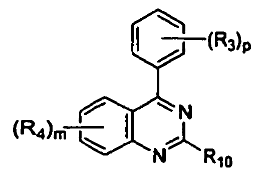 or a pharmaceutically acceptable salt thereof, wherein
m is 0, 1, 2, 3, or 4;
p is 0, 1, 2, 3, 4, or 5;
R₁₀ is indolyl, substituted with 1, 2, 3, or 4 groups that are independently selected from the group consisting of oxo, -C(O)-(C₁-C₆alkyl)-C(O)-OR₁, -C(O)-NH-(C₁-C₆ alkyl)-C(O)-OR₁, -C(O)-(C₁-C₆haloalkyl)-C(O)-OR₁,C₂-C₆ haloalkenyl-C(O)-OR₁, -C(O)-OR₁,-SO₂-(C₁-C₆-alkyl),-SO₂-phenyl, halogen, C₁-C₄ alkyl, C₁-C₄ alkoxy, C₁-C₄ alkoxycarbonyl, C₂-C₆ alkanoyl optionally substituted with CO₂H or CO₂-(C₁-C₄alkyl), CO₂H, and phenyl substituted with 1, 2, 3, 4, or 5 groups that are independently halogen, C₁-C₄ alkyl, C₁-C₄ alkoxy, NO₂, CF₃, or OCF₃;
R₁ is H, C₁-C₆ alkyl, -(C₁-C₅ alkyl)-CO₂H, or -(C₁-C₅ alkyl)-CO₂, C₁-C₆ alkyl;
each R₃ is independently C₁-C₆ alkyl, halogen, C₁-C₄ alkoxy, -CN, -OH, -C(O)-OR₁, -C(O)-(C₁-C₆ alkyl)-C(O)-OR₁ or-(C₁-C₄ alkoxy)-phenyl, wherein the alkyl portions are optionally substituted with one or two groups that are independently oxo, -NH₂, -OH, -SO₂-C₁-C₆ alkyl, C₁-C₆ alkyl, C₁-C₆ alkoxy, -C(O)-OR₁, or C₁-C₄-aryl; and
each R₄ is independently halogen, heterocycloalkyl, heteroaryl, C₁-C₆ haloalkyl, C₁-C₆ alkoxy, or -NO₂, wherein the heterocycloalkyl is optionally substituted with one or two groups that are independently C₁-C₆ alkyl, C₁-C₆ alkoxy, -OH, -NH₂, -NO₂, oxo, or -CN.

2. The compounds according to claim 1, wherein
R₁₀ is indolyl, substituted with 1, 2, or 3 groups that are independently selected from the group consisting of oxo, -C(O)-(C₁-C₆ alkyl)-C(O)-OR₁, -C(O)-NH-(C₁-C₆ alkyl)-C(O)-OR₁, -C(O)-(C₁-C₆ haloalkyl)-C(O)-OR₁, C₂-C₆ haloalkenyl-C(O)-OR₁, -SO₂-(C₁-C₆ alkyl), -SO₂-phenyl, halogen, C₁-C₄ alkyl, C₁-C₄ alkoxy, C₁-C₄ alkoxycarbonyl, C₂-C₆ alkanoyl optionally substituted with CO₂H or CO₂-(C₁-C₄ alkyl), CO₂H, and phenyl substituted with 1, 2, 3, 4, or 5 groups that are independently halogen, C₁-C₄ alkyl, C₁-C₄ alkoxy, NO₂, CF₃, or OCF₃.

3. The compounds according to claim 2 wherein R₁₀ is indolyl substituted with one or two groups that are independently halogen, -C(O)-(C₁-C₆ alkyl)-C(O)-OR₁, -C(O)-NH-(C₁-C₆ alkyl)-C(O)-OR₁, -C(O)-(C₁-C₆ haloalkyl)-C(O)-OR₁, or C₂-C₆ haloalkenyl-C(O)-OR₁.

4. The compounds according to claim 3 wherein R₁₀ is indolyl substituted with one or two groups that are independently bromo, chloro, -C(O)-(C₁-C₆ alkyl)-C(O)-OR₁,-C(O)-NH-(C₁-C₆ alkyl)-C(O)-OR₁, -C(O)-(C₁-C₆ haloalkyl)-C(O)-OR₁, or C₂-C₆ haloalkenyl-C(O)-OR₁

5. The compounds according to claim 3 wherein
R₁ is -H; and
R₁₀ is indolyl substituted with one or two groups that are independently halogen, -C(O)-CH₂-C(O)-OR₁, -C(O)-C₂H₄-C(O)-OR₁, -C(O)-NH-CH₂-C(O)-OR₁, -C(O)-NH-C₂H₄-C(O)-OR₁, -C(O)-(C₁-C₆ haloalkyl)-C(O)-OR₁, or C₂-C₆ haloalkenyl-C(O)-OR₁.

6. The compounds according to claim 3 wherein R₁₀ is indolyl substituted with one or two groups that are independently halogen, -C(O)-(C₁-C₆ alkyl)-C(O)-OR₁, -C(O)-NH-(C₁-C₆ alkyl)-C(O)-OR₁, -C(O)-(CF₂)-C(O)-OR₁, or C₂-C₆ haloalkenyl-C(O)-OR₁.

7. The compounds according to claim 3 wherein R₁₀ is indolyl substituted with one or two groups that are independently halogen, -C(O)-(C₁-C₆ alkyl)-C(O)-OR₁, -C(O)-NH-(C₁-C₆ alkyl)-C(O)-OR₁, -C(O)-(C₁-C₆ haloalkyl)-C(O)-OR₁, or -CH=CF-C(O)-OR₁.

8. The compounds according to claim 1, wherein each R₃ is independently C₁-C₆ alkyl, halogen, -CN, C₁-C₄ - alkoxy, or -(C₁-C₄ alkoxy)-phenyl; and each R₄ is independently halogen, or furanyl.

9. The compounds according to claim 1, wherein
R₁₀ is indol-1-yl, substituted with 1, 2, or 3 groups that are independently selected from the group consisting of -C(O)-C₂H₄-C(O)-OH, -C(O)-NH-C₂H₄-C(O)-OH, -SO₂-(C₁-C₆ alkyl), -SO₂-phenyl, halogen, C₁-C₄ alkyl, C₁-C₄ alkoxy, C₄ alkoxycarbonyl, C₂-C₆ alkanoyl optionally substituted with CO₂H or CO₂-(C₁-C₄ alkyl), CO₂H, and phenyl substituted with 1, 2, 3, 4, or 5 groups that are independently halogen, C₁-C₄ alkyl, C₁-C₄ alkoxy, NO₂, CF₃, or OCF₃;
each R₃ is independently C₁-C₆ alkyl, halogen, or benzyloxy; and
each R₄ is independently halogen.

10. The compounds according to claim 2, wherein
R₁₀ is indol-1-yl or indol-2-yl, each of which is substituted with 1 or 2 groups that are independently -SO₂-(C₁-C₆ alkyl), -SO₂-phenyl, halogen, C₁-C₄ alkyl, C₁-C₄ alkoxy, C₄ alkoxycarbonyl, C₂-C₆ alkanoyl optionally substituted with CO₂H or CO₂-(C₁-C₄ alkyl), CO₂H, or phenyl substituted with 1, 2, 3, 4, or 5 groups that are independently halogen, C₁-C₄ alkyl, C₁-C₄ alkoxy, NO₂, CF₃, OCF₃;
each R₃ is independently C₁-C₆ alkyl, halogen, or benzyloxy; and
each R₄ is independently halogen.

11. The compounds according to claim 10, wherein
R₁₀ is indol-1-yl substituted with 1 or 2 groups that are independently halogen, C₁-C₄ alkyl, C₁-C₄ alkoxy, C₄ alkoxycarbonyl, or C₂-C₆ alkanoyl optionally substituted with CO₂H or CO₂-(C₁-C₄ alkyl);
each R₃ is independently C₁-C₆ alkyl, halogen, or benzyloxy; and
each R₄ is independently halogen.

12. The compounds according to claim 11, wherein R₁₀ is indol-1-yl substituted with 1 or 2 groups that are independently halogen, C₁-C₄ alkyl, C₁-C₄ alkoxy, or C₂-C₄ alkanoyl optionally substituted with CO₂H or CO₂-(C₁-C₄ alkyl).

13. The compounds according to claim 11, wherein R₁₀ is indol-1-yl substituted with 1 or 2 groups that are independently halogen, C₁-C₄ alkyl, C₁-C₄ alkoxy, or C₂-C₄ alkanoyl optionally substituted with CO₂H or CO₂-(C₁-C₄ alkyl), where at least one group is C₂-C₄ alkanoyl optionally substituted with CO₂H or CO₂-(C₁-C₄ alkyl) in the 3-position of the indole ring.

14. A compound according to claim 1 which is
4-[1-(6-Chloro-4-phenyl-quinazolin-2-yl)-1H-indol-3-yl]-4-oxo-butyric acid;
4-{5-Bromo-1-[4-(4-butyl-phenyl)-6-furan-2-yl-quinazolin-2-yl]-1H-indol-3-yl}-4-oxo-butyric acid;
3-[5-Chloro-1-(6-chloro-4-phenyl-quinazolin-2-yl)-1H-indol-3-yl]-2,2-difluoro-3-oxo-propionic acid;
3-[5-Chloro-1-(6-chloro-4-phenyl-quinazolin-2-yl)-1H-indol-3-yl]-2-fluoro-acrylic acid;
1-(6-Chloro-4-phenyl-quinazolin-2-yl)-1H-indole-5-carboxylic acid methyl ester;
4-[5-Bromo-1-(6-chloro-4-phenyl-quinazolin-2-yl)-1H-indol-3-yl]-4-oxo-butyric acid;
4-[5-Bromo-1-(6-fluoro-4-phenyl-quinazolin-2-yl)-1H-indol-3-yl]-4-oxo-butyric acid;
4-{1-[6-Bromo-4-(2-fluoro-phenyl)-quinazolin-2-yl]-1H-indol-3-yl}-4-oxo-butyric acid;
4-{5-Bromo-1-[6-furan-2-yl-4-(4-methoxy-phenyl)-quinazolin-2-yl]-1H-indol-3-yl}-4-oxo-butyric acid;
4-{5-Bromo-1-[4-(4-cyano-phenyl)-6-furan-2-yl-quinazolin-2-yl]-1H-indol-3-yl}-4-oxo-butyric acid;
4-{5-Bromo-1-[4-(4-fluoro-phenyl)-6-furan-2-yl-quinazolin-2-yl]-1H-indol-3-yl}-4-oxo-butyric acid;
4-[1-(6-Chloro-4-phenyl-quinazolin-2-yl)-6-fluoro-1H-indol-3-yl]-4-oxo-butyric acid;
4-[1-(6-Furan-2-yl-4-phenyl-quinazolin-2-yl)-1H-indol-3-yl]-4-oxo-butyric acid;
4-[5-Bromo-1-(6-furan-2-yl-4-phenyl-quinazolin-2-yl)-1H-indol-3-yl]-4-oxo-butyric acid;
4-{1-[4-(4-Butyl-phenyl)-6-furan-2-yl-quinazolin-2-yl]-1H-indol-3-yl}-4-oxo-butyric acid;
4-[5-Chloro-1-(6-chloro-4-phenyl-quinazolin-2-yl)-1H-indol-3-yl]-4-oxo-butyric acid;
{[5-Chloro-1-(6-chloro-4-phenyl-quinazolin-2-yl)-1H-indole-3-carbonyl]-amino}-acetic acid;
3-(3-Carboxy-propionyl)-1-(6-chloro-4-phenyl-quinazolin-2-yl)-1H-indole-5-carboxylic acid methyl ester;
3-{[5-Chloro-1-(6-chloro-4-phenyl-quinazolin-2-yl)-1H-indole-3-carbonyl]-amino}-propionic acid;
3-[5-Chlord-1-(6-chloro-4-phenyl-quinazolin-2-yl)-1H-indol-3-yl]-3-oxo-propionic acid;
4-[6-Chloro-1-(6-chloro-4-phenyl-quinazolin-2-yl)-1H-indol-3-yl]-4-oxo-butyric acid;
or a pharmaceutically acceptable salt thereof.

15. Use of a compound or salt according to claim 1 for the manufacture of a medicament for treating diabetes.

16. A pharmaceutical composition of a compound or salt of claim 1 and at least one pharmaceutically acceptable solvent, carrier, adjuvant or excipient.

## Patentansprüche

1. Verbindung der Formel (II): 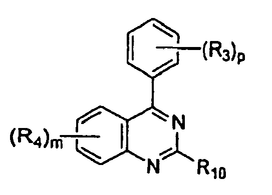 oder ein pharmazeutisch verträgliches Salz davon, worin
m 0, 1, 2, 3 oder 4 ist;
p 0, 1, 2, 3, 4 oder 5 ist;
R₁₀ Indolyl ist, substituiert mit 1, 2, 3 oder 4 Gruppen, unabhängig ausgewählt aus der Gruppe, bestehend aus Oxo,-C(O)-(C₁-C₆-Alkyl)-C(O)-OR₁, -C(O)-NH-(C₁-C₆-Alkyl)-C(O)-OR₁,-C(O)-(C₁-C₆-Halogenalkyl)-C(O)-OR₁, C₂-C₆-Halogenalkenyl-C(O)-OR₁, -C(O)-OR₁, -SO₂-(C₁-C₆-Alkyl), -SO₂-Phenyl, Halogen, C₁-C₄-Alkyl, C₁-C₄-Alkoxy, C₁-C₄-Alkoxycarbonyl, C₂-C₆-Alkanoyl, gegebenenfalls substituiert mit CO₂H oder CO₂-(C₁-C₄-Alkyl), CO₂H und Phenyl, substituiert mit 1, 2, 3, 4 oder 5 Gruppen, unabhängig ausgewählt aus Halogen, C₁-C₄-Alkyl, C₁-C₄-Alkoxy, NO₂, CF₃ oder OCF₃;
R₁ H, C₁-C₆-Alkyl, -(C₁-C₅-Alkyl)-CO₂H oder -(C₁-C₅-Alkyl)-CO₂, C₁-C₆-Alkyl ist;
jeder Rest R₃ unabhängig C₁-C₆-Alkyl, Halogen, C₁-C₄-Alkoxy, -CN, -OH, -C(O)-OR₁, -C(O)-(C₁-C₆-Alkyl)-C(O)-OR₁ oder (C₁-C₄-Alkoxy)-phenyl ist, wobei die Alkylabschnitte gegebenenfalls substituiert sind mit einer oder zwei Gruppen, unabhängig ausgewählt aus Oxo, -NH₂, -OH, -SO₂-C₁-C₆-Alkyl, C₁-C₆-Alkyl, C₁-C₆-Alkoxy, -C(O)-OR₁ oder C₁-C₄-Aryl; und jeder Rest R₄ unabhängig Halogen, Heterocycloalkyl, Heteroaryl, C₁-C₆-Halogenalkyl, C₁-C₆-Alkoxy oder -NO₂ ist, wobei der Heterocycloalkylrest gegebenenfalls substituiert ist mit einer oder zwei Gruppen, unabhängig ausgewählt aus C₁-C₆-Alkyl, C₁-C₆-Alkoxy, -OH, -NH₂, -NO₂, Oxo oder -CN.

2. Verbindungen nach Anspruch 1, worin
R₁₀ Indolyl ist, substituiert mit 1, 2 oder 3 Gruppen, unabhängig ausgewählt aus der Gruppe, bestehend aus Oxo, -C(O)-(C₁-C₆-Alkyl)-C(O)-OR₁, -C(O)-NH-(C₁-C₆-Alkyl)-C(O)-OR₁, -C(O)-(C₁-C₆-Halogenalkyl)-C(O)-OR₁, C₂-C₆-Halogenalkenyl-C(O)-OR₁, -SO₂-(C₁-C₆-Alkyl), -SO₂-Phenyl, Halogen, C₁-C₄-Alkyl, C₁-C₄-Alkoxy, C₁-C₄-Alkoxycarbonyl, C₂-C₆-Alkanoyl, gegebenenfalls substituiert mit CO₂H oder CO₂-(C₁-C₄-Alkyl), CO₂H und Phenyl, substituiert mit 1, 2, 3, 4 oder 5 Gruppen, unabhängig ausgewählt aus Halogen, C₁-C₄-Alkyl, C₁-C₄-Alkoxy, NO₂, CF₃, oder OCF₃.

3. Verbindungen nach Anspruch 2, worin
R₁₀ Indolyl ist, substituiert mit einer oder zwei Gruppen, unabhängig ausgewählt aus Halogen, -C(O)-(C₁-C₆-Alkyl)-C(O)-OR₁, -C(O)-NH-(C₁-C₆-Alkyl)-C(O)-OR₁, -C(O)-(C₁-C₆-Halogenalkyl) -C(O)-OR₁ oder C₂-C₆-Halogenalkenyl-C(O)-OR₁.

4. Verbindungen nach Anspruch 3, worin
R₁₀ Indolyl ist, substituiert mit einer oder zwei Gruppen, unabhängig ausgewählt aus Brom, Chlor, -C(O)-(C₁-C₆-Alkyl)-C(O)-OR₁, -C(O)-NH-(C₁-C₆-Alkyl)-C(O)-OR₁, -C(O)-(C₁-C₆-Halogenalkyl) -C(O)-OR₁ oder C₂-C₆-Halogenalkenyl-C(O)-OR₁.

5. Verbindungen nach Anspruch 3, worin
R₁ -H ist; und
R₁₀ Indolyl ist, substituiert mit einer oder zwei Gruppen, unabhängig ausgewählt aus Halogen, -C(O)-CH₂-C(O)-OR₁,-C(O)-C₂H₄-C(O)-OR₁, -C(O)-NH-CH₂-C(O)-OR₁, -C(O)-NH-C₂H₄-C(O)-OR₁, -C(O)-(C₁-C₆-Halogenalkyl)-C(O)-OR₁ oder C₂-C₆-Halogenalkenyl-C(O)-OR₁.

6. Verbindungen nach Anspruch 3, worin
R₁₀ Indolyl ist, substituiert mit einer oder zwei Gruppen, unabhängig ausgewählt aus Halogen, -C(O)-(C₁-C₆-Alkyl)-C(O)-OR₁, -C(O)-NH-(C₁-C₆-Alkyl)-C(O)-OR₁, -C(O)-(CF₂)-C(O)-OR₁ oder C₂-C₆-Halogenalkenyl-C(O)-OR₁.

7. Verbindungen nach Anspruch 3, worin
R₁₀ Indolyl ist, substituiert mit einer oder zwei Gruppen, unabhängig ausgewählt aus Halogen, -C(O)-(C₁-C₆-Alkyl)-C(O)-OR₁, -C(O)-NH-(C₁-C₆-Alkyl)-C(O)-OR₁, -C(O)-(C₁-C₆-Halogenalkyl) -C(O)-OR₁ oder -CH=CF-C(O)-OR₁.

8. Verbindungen nach Anspruch 1, worin
jeder Rest R₃ unabhängig C₁-C₆-Alkyl, Halogen, -CN, C₁-C₄-Alkoxy oder -(C₁-C₄-Alkoxy)-phenyl ist; und
jeder Rest R₄ unabhängig Halogen oder Furanyl ist.

9. Verbindungen nach Anspruch 1, worin
R₁₀ Indol-1-yl ist, substituiert mit 1, 2 oder 3 Gruppen, unabhängig ausgewählt aus der Gruppe, bestehend aus -C(O)-C₂H₄-C(O)-OH, -C(O)-NH-C₂H₄-C(O)-OH, -SO₂-(C₁-C₆-Alkyl), -SO₂-Phenyl, Halogen, C₁-C₄-Alkyl, C₁-C₄-Alkoxy, C₄-Alkoxycarbonyl, C₂-C₆-Alkanoyl, gegebenenfalls substituiert mit CO₂H oder CO₂-(C₁-C₄-Alkyl), CO₂H und Phenyl, substituiert mit 1, 2, 3, 4 oder 5 Gruppen, unabhängig ausgewählt aus Halogen, C₁-C₄-Alkyl, C₁-C₄-Alkoxy, NO₂, CF₃ oder OCF₃;
jeder Rest R₃ unabhängig C₁-C₆-Alkyl, Halogen oder Benzyloxy ist; und
jeder Rest R₄ unabhängig Halogen ist.

10. Verbindungen nach Anspruch 2, worin
R₁₀ Indol-1-yl oder Indol-2-yl ist, jeweils substituiert mit 1 oder 2 Gruppen, unabhängig ausgewählt aus -SO₂-(C₁-C₆-Alkyl), -SO₂-Phenyl, Halogen, C₁-C₄-Alkyl, C₁-C₄-Alkoxy, C₄-Alkoxycarbonyl, C₂-C₆-Alkanoyl, gegebenenfalls substituiert mit CO₂H oder CO₂-(C₁-C₄-Alkyl), CO₂H oder Phenyl, substituiert mit 1, 2, 3, 4 oder 5 Gruppen, unabhängig ausgewählt aus Halogen, C₁-C₄-Alkyl, C₁-C₄-Alkoxy, NO₂, CF₃, OCF₃;
jeder Rest R₃ unabhängig C₁-C₆-Alkyl, Halogen oder Benzyloxy ist; und
jeder Rest R₄ unabhängig Halogen ist.

11. Verbindungen nach Anspruch 10, worin
R₁₀ Indol-1-yl ist, substituiert mit 1 oder 2 Gruppen, unabhängig ausgewählt aus Halogen, C₁-C₄-Alkyl, C₁-C₄-Alkoxy, C₄-Alkoxycarbonyl oder C₂-C₆-Alkanoyl, gegebenenfalls substituiert mit CO₂H oder CO₂-(C₁-C₄-Alkyl);
jeder Rest R₃ unabhängig C₁-C₆-Alkyl, Halogen oder Benzyloxy ist; und
jeder Rest R₄ unabhängig Halogen ist.

12. Verbindungen nach Anspruch 11, worin
R₁₀ Indol-1-yl ist, substituiert mit 1 oder 2 Gruppen, unabhängig ausgewählt aus Halogen, C₁-C₄-Alkyl, C₁-C₄-Alkoxy oder C₂-C₄-Alkanoyl, gegebenenfalls substituiert mit CO₂H oder CO₂-(C₁-C₄-Alkyl).

13. Verbindungen nach Anspruch 11, worin
R₁₀ Indol-1-yl ist, substituiert mit 1 oder 2 Gruppen, unabhängig ausgewählt aus Halogen, C₁-C₄-Alkyl, C₁-C₄-Alkoxy, oder C₂-C₄-Alkanoyl, gegebenenfalls substituiert mit CO₂H oder CO₂-(C₁-C₄-Alkyl), wobei mindestens eine Gruppe C₂-C₄-Alkanoyl ist, gegebenenfalls substituiert mit CO₂H oder CO₂-(C₁-C₄-Alkyl) in der 3-Position des Indolrings.

14. Verbindung nach Anspruch 1, ausgewählt aus
4-[1-(6-Chlor-4-phenylchinazolin-2-yl)-1H-indol-3-yl]-4-oxo-buttersäure;
4-{5-Brom-1-[4-(4-butylphenyl)-6-furan-2-yl-chinazolin-2-yl]-1H-indol-3-yl}-4-oxo-buttersäure;
3-[5-Chlor-1-(6-chlor-4-phenylchinazolin-2-yl)-1H-indol-3-yl]-2,2-difluor-3-oxo-propionsäure;
3-[5-Chlor-1-(6-chlor-4-phenylchinazolin-2-yl)-1H-indol-3-yl]-2-fluor-acrylsäure;
1-(6-Chlor-4-phenylchinazolin-2-yl)-1H-indol-5-carbonsäuremethylester;
4-[5-Brom-1-(6-chlor-4-phenylchinazolin-2-yl)-1H-indol-3-yl]-4-oxo-buttersäure;
4-[5-Brom-1-(6-fluor-4-phenylchinazolin-2-yl)-1H-indol-3-yl]-4-oxo-buttersäure;
4-{1-[6-Brom-4-(2-fluorphenyl)-chinazolin-2-yl]-1H-indol-3-yl}-4-oxo-buttersäure;
4-{5-Brom-1-[6-furan-2-yl-4-(4-methoxyphenyl)-chinazolin-2-yl]-1H-indol-3-yl}-4-oxo-buttersäure;
4-{5-Brom-1-[4-(4-cyanophenyl)-6-furan-2-yl-chinazolin-2-yl]-1H-indol-3-yl}-4-oxo-buttersäure;
4-{5-Brom-1-[4-(4-fluorphenyl)-6-furan-2-yl-chinazolin-2-yl]-1H-indol-3-yl}-4-oxo-buttersäure;
4-[1-(6-Chlor-4-phenylchinazolin-2-yl)-6-fluor-1H-indol-3-yl]-4-oxo-buttersäure;
4-[1-(6-Furan-2-yl-4-phenylchinazolin-2-yl)-1H-indol-3-yl]-4-oxo-buttersäure;
4-[5-Brom-1-(6-furan-2-yl-4-phenylchinazolin-2-yl)-1H-indol-3-yl]-4-oxo-buttersäure;
4-{1-[4-(4-Butylphenyl)-6-furan-2-yl-chinazolin-2-yl]-1H-indol-3-yl}-4-oxo-buttersäure;
4-[5-Chlor-1-(6-chlor-4-phenylchinazolin-2-yl)-1H-indol-3-yl]-4-oxo-buttersäure;
{[5-Chlor-1-(6-chlor-4-phenylchinazolin-2-yl)-1H-indol-3-carbonyl]-amino}-essigsäure;
3-(3-Carboxypropionyl)-1-(6-chlor-4-phenylchinazolin-2-yl)-1H-indol-5-carbonsäuremethylester;
3-{[5-Chlor-1-(6-chlor-4-phenylchinazolin-2-yl)-1H-indol-3-carbonyl]-amino}-propionsäure =;
3-[5-Chlor-1-(6-chlor-4-phenylchinazolin-2-yl)-1H-indol-3-yl]-3-oxo-propionsäure;
4-[6-Chlor-1-(6-chlor-4-phenylchinazolin-2-yl)-1H-indol-3-yl]-4-oxo-buttersäure;
oder ein pharmazeutisch verträgliches Salz davon.

15. Verwendung einer Verbindung oder eines Salzes gemäß Anspruch 1 zur Herstellung eines Medikaments zur Behandlung von Diabetes.

16. Pharmazeutische Zusammensetzung einer Verbindung oder eines Salzes gemäß Anspruch 1 und mindestens eines pharmazeutisch verträglichen Lösungsmittels, Trägerstoffs, Zusatzstoffs oder Exzipiens.

## Revendications

1. Composé de formule (II) : 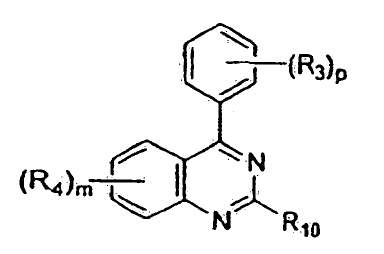 ou sel de celui-ci acceptable du point de vue pharmaceutique,
dans laquelle :
m est 0, 1, 2, 3 ou 4 ;
p est 0, 1, 2, 3, 4 ou 5 ;
R₁₀ est un groupe indolyle substitué par 1, 2, 3 ou 4 groupes qui sont choisis indépendamment dans le groupe consistant en oxo, -C(O)-alkyl(en C₁₋₆)-C(O)-OR₁, -C(O)-NH-alkyl(en C₁₋₆)-C(O)-OR₁, -C(O)-haloalkyl(en C₁₋₆)-C(O)-OR₁, haloalcényl(en C₂₋₆)-C(O)-OR₁, -C(O)-OR₁, -SO₂-alkyle en C₁₋₆, -SO₂-phényle, halogène, alkyle en C₁₋₄, alcoxy en C₁₋₄, alcoxycarbonyle en C₁₋₄, alcanoyle en C₂₋₆ éventuellement substitué par CO₂H ou -CO₂-alkyle en C₁₋₄, CO₂H, et phényle substitué par 1, 2, 3, 4 ou 5 groupes qui sont indépendamment un atome d'halogène, un groupe alkyle en C₁₋₄, alcoxy en C₁₋₄, NO₂, CF₃ ou OCF₃ ;
R₁ est un atome d'hydrogène, un groupe alkyle en C₁₋₆, -alkyl(en C₁₋₅)-CO₂H ou -alkyl(en C₁₋₅)-CO₂, alkyle en C₁₋₆ ;
chaque R₃ est indépendamment un groupe alkyle en C₁₋₆, halogène, alcoxy en C₁₋₄, -CN, -OH, -C(O)-OR₁, -C(O)-alkyl(en C₁₋₆)-C(O)-OR₁ ou -alcoxy(en C₁₋₄)-phényle, où les parties alkyle sont éventuellement substituées par un ou deux groupes qui sont indépendamment un groupe oxo, -NH₂, -OH, -SO₂-alkyle en C₁₋₆, alkyle en C₁₋₆, alcoxy en C₁₋₆, -C(O)-OR₁ ou aryle en C₁₋₄ ; et
chaque R₄ est indépendamment un atome d'halogène, un groupe hétérocycloalkyle, hétéroaryle, haloalkyle en C₁₋₆, alcoxy en C₁₋₆ ou -NO₂, où le groupe hétérocycloalkyle est éventuellement substitué par un ou deux groupes qui sont indépendamment un groupe alkyle en C₁₋₆, alcoxy en C₁₋₆, -OH, -NH₂, -NO₂, oxo ou -CN.

2. Composés selon la revendication 1, dans lesquels
R₁₀ est un groupe indolyle substitué par 1, 2 ou 3 groupes qui sont choisis indépendamment dans le groupe consistant en oxo, -C(O)-alkyl(en C₁₋₆)-C(O)-OR₁, -C(O)-NH-alkyl(en C₁₋₆)-C(O)-OR₁, -C(O)-haloalkyl(en C₁₋₆)-C(O)-OR₁, haloalcényl(en C₂₋₆)-C(O)-OR₁, -SO₂-alkyle en C₁₋₆, -SO₂-phényle, halogène, alkyle en C₁₋₄, alcoxy en C₁₋₄, alcoxycarbonyle en C₁₋₄, alcanoyle en C₂₋₆ éventuellement substitué par CO₂H ou -CO₂-alkyle en C₁₋₄, CO₂H, et phényle substitué par 1, 2, 3, 4 ou 5 groupes qui sont indépendamment un atome d'halogène, un groupe alkyle en C₁₋₄, alcoxy en C₁₋₄, NO₂, CF₃ ou OCF₃.

3. Composés selon la revendication 2, dans lesquels R₁₀ est un groupe indolyle substitué par un ou deux groupes qui sont indépendamment un atome d'halogène, un groupe -C(O)-alkyl(en C₁₋₆)-C(O)-OR₁, -C(O)-NH-alkyl(en C₁₋₆)-C(O)-OR₁, -C(O)-haloalkyl(en C₁₋₆)-C(O)-OR₁ ou haloalcényl(en C₂₋₆)-C(O)-OR₁.

4. Composés selon la revendication 3, dans lesquels R₁₀ est un groupe indolyle substitué par un ou deux groupes qui sont indépendamment un atome de brome, de chlore, un groupe -C(O)-alkyl(en C₁₋₆)-C(O)-OR₁, -C(O)-NH-alkyl(en C₁₋₆)-C(O)-OR₁, -C(O)-haloalkyl(en C₁₋₆)-C(O)-OR₁ ou haloalcényl(en C₂₋₆)-C(O)-OR₁.

5. Composés selon la revendication 3, dans lesquels :
R₁ est -H ; et
R₁₀ est un groupe indolyle substitué par un ou deux groupes qui sont indépendamment un atome d'halogène, un groupe -C(O)-CH₂-C(O)-OR₁, -C(O)-C₂H₄-C(O)-OR₁, -C(O)-NH-CH₂-C(O)-OR₁, -C(O)-NH-C₂H₄-C(O)-OR₁, -C(O)-haloalkyl(en C₁₋₆)-C(O)-OR₁ ou haloalcényl(en C₂₋₆)-C(O)-OR₁.

6. Composés selon la revendication 3, dans lesquels R₁₀ est un groupe indolyle substitué par un ou deux groupes qui sont indépendamment un atome d'halogène, un groupe -C(O)-alkyl(en C₁₋₆)-C(O)-OR₁, -C(O)-NH-alkyl(en C₁₋₆)-C(O)-OR₁, -C(O)-(CF₂)-C(O)-OR₁ ou haloalcényl(en C₂₋₆)-C(O)-OR₁.

7. Composés selon la revendication 3, dans lesquels R₁₀ est un groupe indolyle substitué par un ou deux groupes qui sont indépendamment un atome d'halogène, un groupe -C(O)-alkyl(en C₁₋₆)-C(O)-OR₁, -C(O)-NH-alkyl(en C₁₋₆)-C(O)-OR₁, -C(O)-haloalkyl(en C₁₋₆)-C(O)-OR₁ ou -CH=CF-C(O)-OR₁.

8. Composés selon la revendication 1, dans lesquels chaque R₃ est indépendamment un groupe alkyle en C₁₋₆, un atome d'halogène, un groupe -CN, alcoxy en C₁₋₄ ou -alcoxy(en C₁₋₄)-phényle ; et chaque R₄ est indépendamment un atome d'halogène ou un groupe furanyle.

9. Composés selon la revendication 1, dans lesquels
R₁₀ est un groupe indol-1-yle substitué par 1, 2 ou 3 groupes qui sont choisis indépendamment dans le groupe consistant en -C(O)-C₂H₄-C(O)-OH, -C(O)-NH-C₂H₄-C(O)-OH, -SO₂-alkyle en C₁₋₆, -SO₂-phényle, halogène, alkyle en C₁₋₄, alcoxy en C₁₋₄, alcoxycarbonyle en C₄, alcanoyle en C₂₋₆ éventuellement substitué par CO₂H ou CO₂-alkyle en C₁₋₄, CO₂H et phényle substitué par 1, 2, 3, 4 ou 5 groupes qui sont indépendamment un atome d'halogène, un groupe alkyle en C₁₋₄, alcoxy en C₁₋₄, NO₂, CF₃, ou OCF₃ ;
chaque R₃ est indépendamment un groupe alkyle en C₁₋₆, halogène ou benzyloxy ; et
chaque R₄ est indépendamment un atome d'halogène.

10. Composés selon la revendication 2, dans lesquels
R₁₀ est un groupe indol-1-yle ou indol-2-yle, qui sont chacun substitués par 1 ou 2 groupes qui sont indépendamment un groupe -SO₂-alkyle en C₁₋₆, -SO₂-phényle, halogène, alkyle en C₁₋₄, alcoxy en C₁₋₄, alcoxycarbonyle en C₄, alcanoyle en C₂₋₆ éventuellement substitué par CO₂H ou CO₂-alkyle en C₁₋₄, CO₂H ou phényle substitué par 1, 2, 3, 4 ou 5 groupes qui sont indépendamment un atome d'halogène, un groupe alkyle en C₁₋₄, alcoxy en C₁₋₄, NO₂, CF₃, OCF₃ ;
chaque R₃ est indépendamment un groupe alkyle en C₁₋₆, halogène ou benzyloxy ; et
chaque R₄ est indépendamment un atome d'halogène.

11. Composés selon la revendication 10, dans lesquels :
R₁₀ est un groupe indol-1-yle substitué par 1 ou 2 groupes qui sont indépendamment un atome d'halogène, un groupe alkyle en C₁₋₄, alcoxy en C₁₋₄, alcoxycarbonyle en C₄ ou alcanoyle en C₂₋₆ éventuellement substitué par CO₂H ou CO₂-alkyle en C₁₋₄ ;
chaque R₃ est indépendamment un groupe alkyle en C₁₋₆, halogène ou benzyloxy ; et
chaque R₄ est indépendamment un atome d'halogène.

12. Composés selon la revendication 11, dans lesquels R₁₀ est un groupe indol-1-yle substitué par 1 ou 2 groupes qui sont indépendamment un atome d'halogène, un groupe alkyle en C₁₋₄, alcoxy en C₁₋₄ ou alcanoyle en C₁₋₄ éventuellement substitué par CO₂H ou CO₂-alkyle en C₁₋₄.

13. Composés selon la revendication 11, dans lesquels R₁₀ est un groupe indol-1-yle substitué par 1 ou 2 groupes qui sont indépendamment un atome d'halogène, un groupe alkyle en C₁₋₄, alcoxy en C₁₋₄ ou alcanoyle en C₂₋₄ éventuellement substitué par CO₂H ou CO₂-alkyle en C₁₋₄, où au moins un groupe est un groupe alcanoyle en C₂₋₄ éventuellement substitué par CO₂H ou CO₂-alkyle en C₁₋₄ en position 3 du noyau indole.

14. Composé selon la revendication 1, qui est :
l'acide 4-[1-(6-chloro-4-phényl-quinazolin-2-yl)-1H-indol-3-yl]-4-oxo-butyrique ;
l'acide 4-{5-bromo-1-[4-(4-butyl-phényl)-6-furan-2-yl-quinazolin-2-yl]-1H-indol-3-yl}-4-oxo-butyrique ;
l'acide 3-[5-chloro-1-(6-chloro-4-phényl-quinazolin-2-yl)-1H-indol-3-yl]-2,2-difluoro-3-oxo-propionique ;
l'acide 3-[5-chloro-1-(6-chloro-4-phényl-quinazolin-2-yl)-1H-indol-3-yl]-2-fluoro-acrylique ;
l'ester méthylique de l'acide 1-(6-chloro-4-phényl-quinazolin-2-yl)-1H-indole-5-carboxylique ;
l'acide 4-[5-bromo-1-(6-chloro-4-phényl-quinazolin-2-yl)-1H-indol-3-yl]-4-oxo-butyrique ;
l'acide 4-[5-bromo-1-(6-fluoro-4-phényl-quinazolin-2-yl)-1H-indol-3-yl]-4-oxo-butyrique ;
l'acide 4-{1-[6-bromo-4-(2-fluoro-phényl)-quinazolin-2-yl]-1H-indol-3-yl}-4-oxo-butyrique ;
l'acide 4-{5-bromo-1-[6-furan-2-yl-4-(4-méthoxy-phényl)-quinazolin-2-yl]-1H-indol-3-yl}-4-oxo-butyrique ;
l'acide 4-{5-bromo-1-[4-(4-cyano-phényl)-6-furan-2-yl-quinazolin-2-yl]-1H-indol-3-yl}-4-oxo-butyrique ;
l'acide 4-{5-bromo-1-[4-(4-fluoro-phényl)-6-furan-2-yl-quinazolin-2-yl]-1H-indol-3-yl}-4-oxo-butyrique ;
l'acide 4-[1-(6-chloro-4-phényl-quinazolin-2-yl)-6-fluoro-1H-indol-3-yl]-4-oxo-butyrique ;
l'acide 4-[1-(6-furan-2-yl-4-phényl-quinazolin-2-yl)-1H-indol-3-yl]-4-oxo-butyrique ;
l'acide 4-[5-bromo-1-(6-furan-2-yl-4-phényl-quinazolin-2-yl)-1H-indol-3-yl]-4-oxo-butyrique ;
l'acide 4-{1-[4-(4-butyl-phényl)-6-furan-2-yl-quinazolin-2-yl]-1H-indol-3-yl}-4-oxo-butyrique ;
l'acide 4-[5-chloro-1-(6-chloro-4-phényl-quinazolin-2-yl)-1H-indol-3-yl]-4-oxo-butyrique ;
l'acide {[5-chloro-1-(6-chloro-4-phényl-quinazolin-2-yl)-1H-indole-3-carbonyl]-amino}-acetique ;
l'ester méthylique de l'acide 3-(3-carboxy-propionyl)-1-(6-chloro-4-phényl-quinazolin-2-yl)-1H-indole-5-carboxylique ;
l'acide 3-{[5-chloro-1-(6-chloro-4-phényl-quinazolin-2-yl)-1H-indole-3-carbonyl]-amino}-propionique ;
l'acide 3-[5-chloro-1-(6-chloro-4-phényl-quinazolin-2-yl)-1H-indol-3-yl]-3-oxo-propionique ;
l'acide 4-[6-chloro-1-(6-chloro-4-phényl-quinazolin-2-yl)-1H-indol-3-yl]-4-oxo-butyrique ;
ou un sel de ceux-ci acceptable du point de vue pharmaceutique.

15. Utilisation d'un composé ou d'un sel selon la revendication 1 pour la fabrication d'un médicament destiné à traiter le diabète.

16. Composition pharmaceutique d'un composé ou d'un sel selon la revendication 1 et d'au moins un solvant, support, adjuvant ou excipient acceptable du point de vue pharmaceutique.
